# EUROPÄISCHE PATENTSCHRIFT

(11) **EP 1 095 045 B1**
(45) Veröffentlichungstag und Bekanntmachung des Hinweises auf die Patenterteilung: **15.01.2003**
(21) Anmeldenummer: 99932747.1
(22) Anmeldetag: 29.06.1999
(51) Int. Cl.: C07D 498/04, A01N 43/90, C07D 513/04, C07C 281/08, C07C 337/08, C07D 487/04

(54) **VERFAHREN ZUR HERSTELLUNG VON ANELLIERTEN TRIAZOLEN UND NEUE ANELLIERTE TRIAZOLE UND DEREN VERWENDUNG**
METHOD FOR PRODUCING ANELLATED TRIAZOLES AND NEW ANELLATED TRIAZOLES AND THEIR USE
PROCEDE DE PREPARATION DE TRIAZOLS ANNELES ET NOUVEAUX TRIAZOLS ANNELES ET LEUR UTILISATION

(30) Priorität: 03.07.1998 DE 19829745
(43) Veröffentlichungstag der Anmeldung: 02.05.2001
(73) Patentinhaber: BASF AKTIENGESELLSCHAFT, 67056 Ludwigshafen (DE)
(72) Erfinder: HAMPRECHT, Gerhard, D-69469 Weinheim (DE); MENKE, Olaf, D-67317 Altleiningen (DE); REINHARD, Robert, D-67061 Ludwigshafen (DE); SCHÄFER, Peter, D-67308 Ottersheim (DE); ZAGAR, Cyrill, D-67061 Ludwigshafen (DE); OTTEN, Martina, D-67069 Ludwigshafen (DE); WESTPHALEN, Karl-Otto, D-67346 Speyer (DE); WALTER, Helmut, D-67283 Obrigheim (DE)
(74) Vertreter: Kinzebach, Werner, Dr.
(86) Internationale Anmeldenummer: EP9904482
(87) Internationale Veröffentlichungsnummer: WO00001700

(56) Entgegenhaltungen:
- WO-A-94/10173

## Beschreibung

Die vorliegende Erfindung betrifft ein Verfahren zur Herstellung von anellierten Triazolen der Formel I 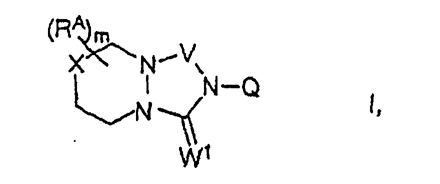 wobei die Substituenten folgende Bedeutungen haben:
- X: O, S, SO, SO₂ oder N(R¹);
- V: C=W²;
- W¹: Sauerstoff oder Schwefel;
- W²: Sauerstoff;
- R^{A}: Wasserstoff, Hydroxy, COOH, COOR², Halogen, Cyano, C(O)NR¹¹R¹², OR³, C₁-C₆-Alkyl, C₁-C₆-Haloalkyl, C₃-C₆-Alkenyl, C₃-C₆-Alkinyl, COR³, S(O)ₙR³ oder C(O)SR²;
- R¹: Wasserstoff, Hydroxy, Halogen, CN, C₁-C₆-Alkyl, C₁-C₆-Haloalkyl, C₂-C₆-Alkenyl, C₂-C₆-Alkinyl, COR³, CHO, OR³, COOR², C(O)SR², C(O)NR¹¹R¹²;
- R²: Wasserstoff, C₁-C₆-Alkyl, C₁-C₆-Haloalkyl, C₂-C₆-Alkenyl oder C₂-C₆-Alkinyl;
- R³: C₁-C₆-Alkyl, C₃-C₆-Cycloalkyl, C₂-C₆-Alkenyl, C₂-C₆-Alkinyl, C₁-C₆-Haloalkyl, C₁-C₆-Alkoxy-C₁-C₆-alkyl, Carboxyl-C₁-C₆-alkyl, C₁-C₆-Alkoxycarbonyl- C₁-C₆-alkyl, C₁-C₆-Alkylthio-C₁-C₆-alkyl, C₁-C₆-Alkylsulfinyl-C₁-C₆-alkyl, C₁-C₆-Alkylsulfonyl-C₁-C₆-alkyl, C₃-C₆-Cycloalkyl-C₁-C₆-alkyl, C₃-C₆-Alkenyloxycarbonyl-C₁-C₆-alkyl, C₃-C₆-Alkinyloxycarbonyl-C₁-C₆-alkyl, C₃-C₆-Cycloalkoxy-C₁-C₆-alkyl, C₃-C₆-Alkenyloxy-C₁-C₆-alkyl, C₃-C₆-Alkinyloxy-C₁-C₆-alkyl, C₁-C₆-Haloalkoxy-C₁-C₆-alkyl, C₃-C₆-Haloalkenyloxy-C₁-C₆-alkyl, C₃-C₆-Haloalkinyloxy-C₁-C₆-alkyl, C₃-C₆-Cycloalkyl-C₁-C₆-thioalkyl, C₃-C₆-Alkenylthio-C₁-C₆-alkyl, C₃-C₆-Alkinylthio-C₁-C₆-alkyl, Cyano-C₁-C₆-alkyl, C₃-C₆-Halocyclo-C₁-C₆-alkyl, Halogen-C₃-C₆-alkenyl, C₁-C₆-Alkoxy-C₃-C₆-alkenyl, C₁-C₆-Haloalkoxy-C₃-C₆-alkenyl, C₁-C₆-Alkylthio-C₃-C₆-alkenyl, C₃-C₆-Haloalkinyl, C₁-C₆-Alkoxy-C₃-C₆-alkinyl, C₃-C₆-Haloalkoxyalkinyl, C₁-C₆-Alkylthioalkinyl, C₁-C₆-Alkylcarbonyl, CHR¹⁶COR¹⁷, CHR¹⁶P(O)(OR¹⁷)₂, P(O)(OR¹⁷)₂, CHR¹⁶P(S)(OR¹⁷)₂, CHR¹⁶C(O)NR¹¹R¹², CHR¹⁶C(O)NH₂, C₁-C₆-Alkyl, welches mit Phenoxy oder Benzyloxy substituiert ist, wobei die Ringe ihrerseits mit Halogen, C₁-C₄-Alkyl oder C₁-C₄-Haloalkyl substituiert sein können, Benzyl welches mit Halogen, C₁-C₄-Alkyl oder C₁-C₄-Haloalkyl substituiert sein kann oder Phenyl und Pyridyl, welche mit Halogen, C₁-C₄-Alkyl, C₁-C₄-Haloalkyl oder C₁-C₄-Alkoxy substituiert sein können;
- m: den Wert 0, 1, 2 oder 3;
- n: den Wert 0, 1 oder 2;
- Q: einen der Reste Q-1 bis Q-7 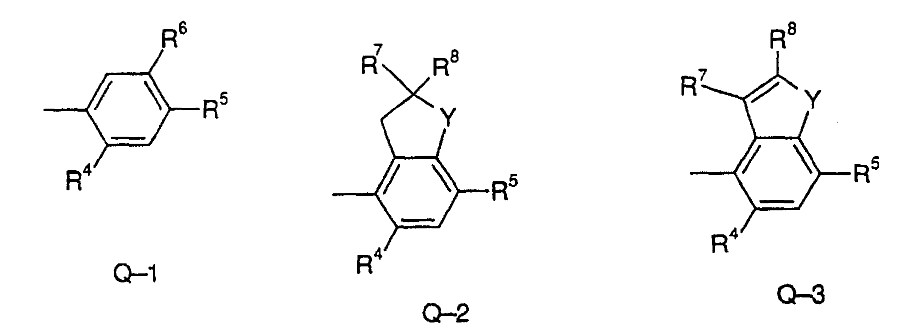 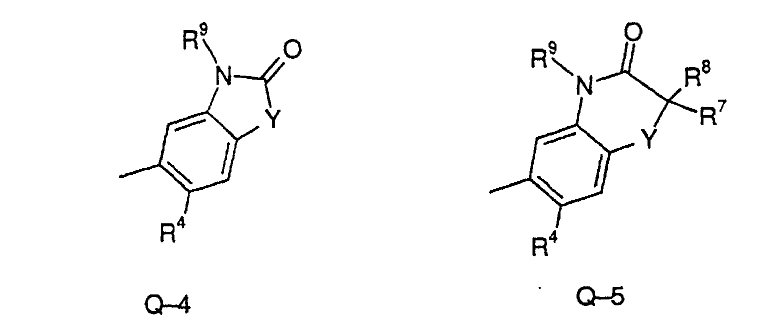 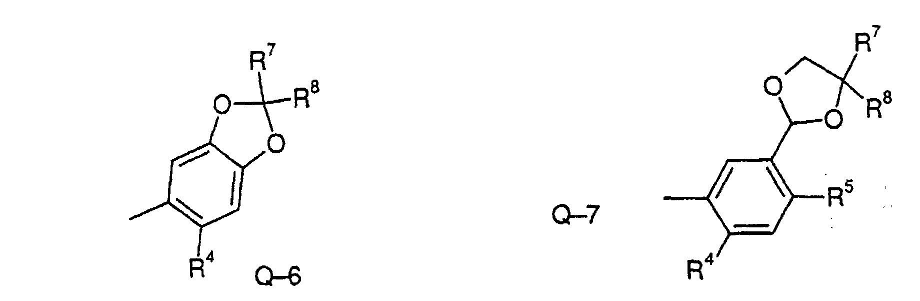
mit
- Y: Sauerstoff oder Schwefel;
- R⁴: Wasserstoff oder Halogen;
- R⁵: C₁-C₆-Alkyl, C₁-C₆-Haloalkyl, OCH₃, SCH₃, OCHF₂, Halogen, CN oder NO₂;
- R⁶: Wasserstoff, C₁-C₆-Alkyl, C₁-C₆-Haloalkyl, Halogen, OR¹⁰, S(O)ₙR¹⁰, COR¹⁰, COOR¹⁰, C(O)SR¹⁰, SCH₂C≡CH, C(O)NR¹¹R¹², CHO, CH=CHCO-OR¹⁰, CO-ON=CR¹³R¹⁴, NO₂, CN, NHSO₂R¹⁵, NHSO₂NHR¹⁵, -C(R¹⁸)=C(R¹⁹)-CO-R²⁰, -CH(R¹⁸)-CH(R¹⁹)-CO-R²⁰, -C(R¹⁸)=C(R¹⁹)-CO-N(R²⁰,R²¹), -CH(R¹⁸)-CH(R¹⁹)-CO-N(R²⁰,R²¹), -C(R²¹)=N-OR²², -COOC(R²³)(R²⁴)-COOR²⁵, -CO-N(R²⁶)-OR²² oder -C(OR²⁷)=N-OR²²;
- R⁷, R⁸: unabhängig voneinander Wasserstoff, C₁-C₆-Alkyl, C₁-C₆-Haloalkyl oder Halogen; wenn Q Q-2, Q-5 oder Q-6 ist, können R⁷ und R⁸ zusammen mit dem C-Atom, an das sie gebunden sind, eine Gruppe C=O bilden;
- R⁹: C₁-C₆-Alkyl, C₁-C₆-Haloalkyl, C₁-C₆-Alkoxy, C₁-C₆-Alkoxy-C₁-C₆-alkyl, C₂-C₆-Alkenyl oder C₂-C₆-Alkinyl;
- R¹⁰: einen der unter R³ angegebenen Reste;
- R¹¹, R¹³: unabhängig voneinander Wasserstoff oder C₁-C₆-Alkyl;
- R¹², R¹⁴: unabhängig voneinander C₁-C₆-Alkyl oder Phenyl, welches mit Halogen, C₁-C₄-Alkyl, C₁-C₄-Haloalkyl oder C₁-C₄-Alkoxy substituiert sein kann;
- R¹¹ and R¹²: können zusammen eine Gruppierung -(CH₂)₅-, -(CH₂)₄- oder -CH₂CH₂OCH₂CH₂-sein, wobei jeder Ring mit C₁-C₃-Alkyl substituiert sein kann, oder Phenyl oder Benzyl;
- R¹³ und R¹⁴: können auch zusammen mit dem C-Atom, an das sie gebunden sind, eine C₃-C₈-Cyclyoalkylgruppe bilden;
- R¹⁵: C₁-C₆-Alkyl oder C₁-C₆-Haloalkyl;
- R¹⁶: Wasserstoff oder C₁-C₆-Alkyl;
- R¹⁷: C₁-C₆-Alkyl, C₂-C₆-Alkenyl oder C₂-C₆-Alkinyl;
- R¹⁸, R²³, R²⁴: jeweils Wasserstoff oder C₁-C₃-Alkyl;
- R¹⁹: Halogen, Cyano oder Methyl;
- R²⁰: Hydroxy, C₁-C₆-Alkoxy, C₃-C₆-Cycloalkoxy, C₁-C₄-Alkoxy-C₁-C₄-alkoxy, C₁-C₄-Alkylthio-C₁-C₄-alkoxy, C₁-C₄-Alkylsulfinyl-C₁-C₄-alkoxy, C₁-C₄-Alkylsulfonyl-C₁-C₄-alkoxy, Cyano-C₁-C₆-alkoxy, C₁-C₄-Alkoxycarbonyl-C₁-C₄-alkoxy, C₃-C₆-Alkenyloxy, C₃-C₆-Alkinyloxy, partiell oder vollständig halogeniertes C₁-C₆-Alkoxy, partiell oder vollständig halogeniertes C₃-C₆-Alkenyloxy, partiell oder vollständig halogeniertes C₃-C₆-Alkinyloxy, C₁-C₆-Alkylthio, ferner C₁-C₆-Alkoxy, das noch zwei C₁-C₆-Alkoxysubstituenten tragen kann;
- R²¹: Wasserstoff, C₁-C₆-Alkyl oder C₁-C₆-Halogenalkyl;
- R²²: Wasserstoff, C₁-C₆-Alkyl, C₁-C₄-Alkoxy-C₁-C₄-alkyl, C₁-C₆-Halogenalkyl, C₁-C₆-Cyanoalkyl, C₃-C₆-Cycloalkyl, C₃-C₆-Alkenyl, C₃-C₆-Alkinyl, C₃-C₆-Halogenalkenyl, C₃-C₆-Halogenalkinyl, Carboxy-C₁-C₆-alkyl, C₁-C₆-Alkoxycarbonyl-C₁-C₄-alkyl, C₁-C₆-Alkylcarbonyl-C₁-C₆-alkyl oder C₁-C₆-Alkylcarbonyloxy-C₁-C₆-alkyl;
- R²⁵: Wasserstoff, C₁-C₆-Alkyl, C₃-C₆-Cycloalkyl, C₁-C₆-Alkoxy-C₁-C₄-alkyl, Cyano-C₁-C₆-alkyl, Halogen-C₁-C₆-alkyl, C₃-C₆-Alkenyl oder C₃-C₆-Alkinyl;
- R²⁶,R²⁷: unabhängig voneinander C₁-C₆-Alkyl, C₁-C₄-Alkoxy-C₁-C₄-alkyl, C₃-C₆-Cycloalkyl, C₃-C₆-Alkenyl, C₃-C₆-Alkinyl, C₁-C₆-Halogenalkyl, C₁-C₆-Cyanoalkyl, C₃-C₆-Halogenalkenyl, C₃-C₆-Halogenalkinyl oder C₁-C₆-Alkoxycabonyl-C₁-C₄-alkyl;
- R²⁶: darüberhinaus Wasserstoff.

Darüber hinaus betrifft die Erfindung neue anellierte Triazole der Formel I' 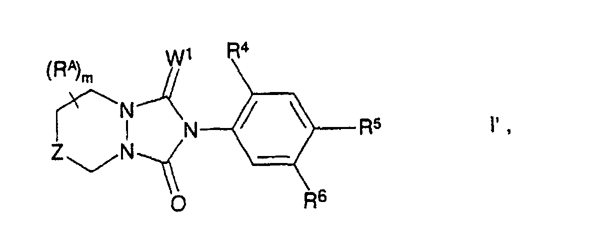 wobei Z für Sauerstoff, Schwefel, SO oder SO₂ steht, deren Verwendung im Pflanzenschutz, sowie anellierte Triazole der Formel I''. 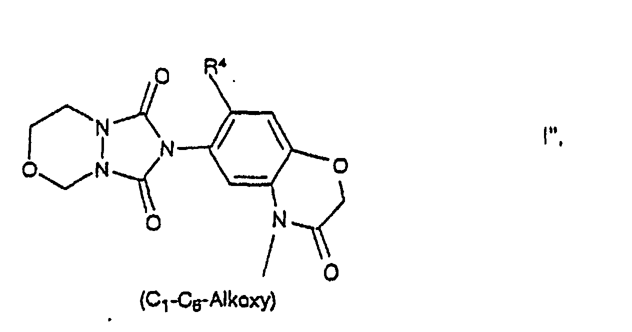 wobei R⁴ für Wasserstoff oder Halogen steht.

Weiterhin betrifft die Erfindung substituierte N-Methylenimino-N'-phenylharnstoffe der Formel III' 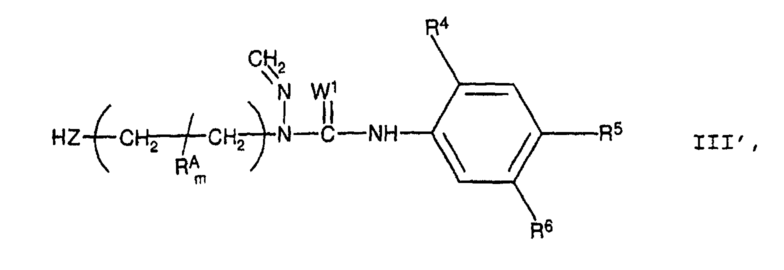 in der die Substituenten folgende Bedeutungen haben:
- Z: O, S, S=O oder SO₂;
- R^{A}: Halogen oder C₁-C₃-Alkyl;
- W¹: Sauerstoff oder Schwefel;
- R⁴: Wasserstoff oder Halogen;
- R⁵: Halogen, Cyano oder Trifluormethyl;
- R⁶: eine Gruppe -C(R¹⁸)=C(R¹⁹)-CO-R²⁰, -CH(R¹⁸)-CH(R¹⁹)-CO-R²⁰, -C(R¹⁸)=C(R¹⁹)-CO-N(R²⁰,R²¹), -CH(R¹⁸)-CH(R¹⁹)-CO-N(R²⁰,R²¹), -C(R²¹)=N-OR²², -CO-OC(R²³)(R²⁴)-CO-OR²⁵, CO-N(R²⁶)-OR²² oder -C(OR²⁷)=N-OR²²;
- R¹⁸, R²³, R²⁴: jeweils Wasserstoff oder C₁-C₃-Alkyl;
- R¹⁹: Halogen, Cyano oder Methyl;
- R²⁰: Hydroxy, C₁-C₆-Alkoxy, C₃-C₆-Cycloalkoxy, C₁-C₄-Alkoxy-C₁-C₄-alkoxy, C₁-C₄-Alkylthio-C₁-C₄-alkoxy, C₁-C₄-Alkylsulfinyl-C₁-C₄-alkoxy, C₁-C₄-Alkylsulfonyl-C₁-C₄-alkoxy, Cyano-C₁-C₆-alkoxy, C₁-C₄-Alkoxycarbonyl-C₁-C₄-alkoxy, C₃-C₆-Alkenyloxy, C₃-C₆-Alkinyloxy, partiell oder vollständig halogeniertes C₁-C₆-Alkoxy, partiell oder vollständig halogeniertes C₃-C₆-Alkenyloxy, partiell oder vollständig halogeniertes C₃-C₆-Alkinyloxy, C₁-C₆-Alkylthio,
ferner C₁-C₆-Alkoxy, das noch zwei C₁-C₆-Alkoxysubstituenten tragen kann;
- R²¹: Wasserstoff, C₁-C₆-Alkyl oder C₁-C₆-Halogenalkyl;
- R²²: Wasserstoff, C₁-C₆-Alkyl, C₁-C₄-Alkoxy-C₁-C₄-alkyl, C₁-C₆-Halogenalkyl, C₁-C₆-Cyanoalkyl, C₃-C₆-Cycloalkyl, C₃-C₆-Alkenyl, C₃-C₆-Alkinyl, C₃-C₆-Halogenalkenyl, C₃-C₆-Halogenalkinyl, Carboxy-C₁-C₆-alkyl, C₁-C₆-Alkoxycarbonyl-C₁-C₄-alkyl, C₁-C₆-Alkylcarbonyl-C₁-C₆-alkyl oder C₁-C₆-Alkylcarbonyloxy-C₁-C₆-alkyl;
- R²⁵: Wasserstoff, C₁-C₆-Alkyl, C₃-C₆-Cycloalkyl, C₁-C₆-Alkoxy-C₁-C₄-alkyl, Cyano-C₁-C₆-alkyl, Halogen-C₁-C₆-alkyl, C₃-C₆-Alkenyl oder C₃-C₆-Alkinyl;
- R²⁶, R²⁷: C₁-C₆-Alkyl, C₁-C₄-Alkoxy-C₁-C₄-alkyl, C₃-C₆-Cyaloalkyl, C₃-C₆-Alkenyl, C₃-C₆-Alkinyl, C₁-C₆-Halogenalkyl, C₁-C₆-Cyano-alkyl, C₃-C₆-Halogenalkenyl, C₃-C₆-Halogenalkinyl oder C₁-C₆-Alkoxycarbonyl-C₁-C₄-alkyl, und
- R²⁶: darüberhinaus Wasserstoff;
- m: 0, 1, 2 oder 3,
und deren Verwendung zur Herstellung von anellierten Triazolen der Formel I'.

In der EP-A 210 137 und der WO 94/10173 werden herbizid wirksame anellierte Triazole und Verfahren zu deren Herstellung beschrieben. Nach dem in der WO 94/10173 beschriebenen Verfahren werden N'-substituierte N-Aminoharnstoffderivate der Formel II mit Aldehyden in Anwesenheit von Säure zu substituierten Tetrahydro-4H-1,3,4-ox(bzw. thia)diazinen der Formel IV umgesetzt und letztere mit Phosgen oder einem Phosgenersatz zu den Verbindungen der Formel I cyclisiert.

Dieses Verfahren liefert jedoch anellierte Triazole nur bei einfacher Phenylsubstitution und nicht in den gewünschten Ausbeuten, weshalb ein Bedarf bestand, insbesondere für mehrfach Phenylsubstituierte Produkte, ein besseres Verfahren zur Herstellung der anellierten Triazole wie sie in der WO 94/10173 beschrieben werden, aufzuzeigen.

Überraschenderweise wurde nun gefunden, daß anellierte Triazole der Formel I mit hoher Ausbeute und verbesserter Reinheit erhalten werden können, wenn man N'-substituierte N-Aminoharnstoffderivate II 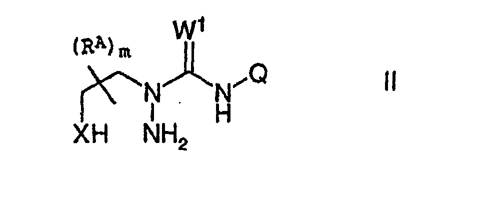 in einem ersten Schritt mit wässrigem Formaldehyd oder Paraformaldehyd in Abwesenheit von Säure in neutralem oder schwach alkalischem Medium zu neuen N-Methylenimino-N'-substituierten Harnstoffen der Formel III 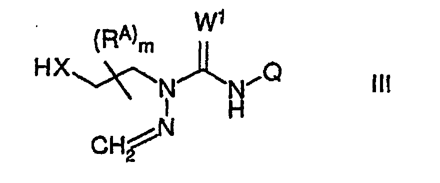 umsetzt, diese in einem zweiten Schritt unter Zugabe von katalytischen Mengen Säure oder eines neutralen oder sauren oberflächenaktiven Metalloxids zu substituierten Tetrahydro-4H-1,3,4-ox(bzw. thia)diazinen der Formel IV 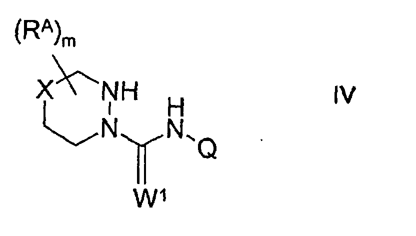 cyclisiert und letztere mit Phosgen oder einem Phosgenersatz zu den Verbindungen der Formel I cyclisiert.

Die bei der Definition der Substituenten verwendeten Bezeichnungen stellen Sammelbegriffe für individuelle Aufzählungen der einzelnen Gruppenmitglieder dar. Sämtliche Alkyl-Reste können geradkettig oder verzweigt sein. Der Halogenalkylrest trägt vorzugsweise ein bis fünf gleiche oder verschiedene Halogenatome.

Im einzelnen stehen beispielsweise:
- Halogen für: Fluor, Chlor, Brom oder Jod, vorzugsweise Fluor oder Chlor;
- C₁-C₃-Alkyl für: Methyl, Ethyl, n-Propyl oder 1-Methylethyl;
- C₁-C₄-Alkyl für: C₁-C₃-Alkyl wie vorstehend genannt, sowie n-Butyl, 1-Methylpropyl, 2-Methylpropyl oder 1,1-Dimethylethyl;
- C₁-C₆-Alkyl für: C₁-C₄-Alkyl wie vorstehend genannt, sowie n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl;
- (C₁-C₆-Alkyl)carbonyl für: Methylcarbonyl, Ethylcarbonyl, n-Propylcarbonyl, 1-Methylethylcarbonyl, n-Butylcarbonyl, 1-Methylpropylcarbonyl, 2-Methylpropylcarbonyl, 1,1-Dimethylethylcarbonyl, n-Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, n-Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl, insbesondere für Methylcarbonyl, Ethylcarbonyl oder 1-Methylethylcarbonyl;
- C₃-C₄-Alkenyl für: Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methyl-prop-1-en-1-yl, 1-Methyl-prop-2-en-1-yl oder 2-Methyl-prop-2-en-1-yl;
- C₃-C₆-Alkenyl für: C₃-C₄-Alkenyl wie vorstehend genannt, sowie n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, , 1,2-Dimethyl-prop-2-en-1-yl, 1-Ethyl-prop-2-en-2-yl, 1-Ethyl-prop-2-en-1-yl, n-Hex-1-en-2-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methylpent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl, vorzugsweise Ethenyl oder Prop-2-en-1-yl;
- C₃-C₄-Alkinyl für: Prop-1-in-1-yl, Prop-2-in-3-yl, n-But-1-in-1-yl, n-But-1-in-4-yl oder n-But-2-in-1-yl;
- C₃-C₆-Alkinyl für: C₃-C₄-Alkinyl wie vorstehend genannt, sowie z.B. n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-1-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methylpent-2-in-4-yl oder 4-Methyl-pent-2-in-5-yl, insbesondere für Prop-2-in-1-yl oder 1-Methyl-prop-2-in-1-yl;
- C₃-C₆-Cycloalkyl-C₁-C₄-alkyl für: Cyclopropylmethyl, Cyclobutylmethyl, Cyclopentylmethyl, Cyclohexylmethyl, Cycloheptylmethyl, Cyclooctylmethyl, 1-(Cyclopropyl)ethyl, 1-(Cyclobutyl)ethyl, 1-(Cyclopentyl)ethyl, 1-(Cyclohexyl)ethyl, 2-(Cyclopropyl)ethyl, 2-(Cyclobutyl)ethyl, 2-(Cyclopentyl)ethyl, 2-(Cyclohexyl)ethyl, 2-(Cyclopropyl)ethyl, 3-(Cyclopropyl)ethyl, 3-(Cyclopropyl)propyl, 3-(Cyclobutyl)propyl, 3-(Cyclopentyl)propyl, 3-(Cyclohexyl)propyl, 4-(Cyclopropyl)butyl, 4-(Cyclobutyl)butyl, 4-(Cyclopentyl)butyl, 4-(Cyclohexyl)butyl, insbesondere für Cyclopentylmethyl oder Cyclohexylmethyl;
- C₃-C₆-Cycloalkyl-C₁-C₆-alkyl für: Cyclopropylmethyl, Cyclobutylmethyl, Cyclopentylmethyl, Cyclohexylmethyl, Cycloheptylmethyl, Cyclooctylmethyl, 1-(Cyclopropyl)ethyl, 1-(Cyclobutyl)ethyl, 1-(Cyclopentyl)ethyl, 1-(Cyclohexyl)ethyl, 2-(Cyclopropyl)ethyl, 2-(Cyclobutyl)ethyl, 2-(Cyclopentyl)ethyl, 2-(Cyclohexyl)ethyl, 2-(Cyclopropyl)propyl, 3-(Cyclopropyl)propyl, 3-(Cyclobutyl)propyl, 3-(Cyclopentyl)propyl, 3-(Cyclohexyl)propyl, 2-(Cyclopropyl)butyl, 3-(Cyclopropyl)butyl, 4-(Cyclopropyl)butyl, 4-(Cyclobutyl)butyl, 4-(Cyclopentyl)butyl, 4-(Cyclohexyl)butyl, 2-(Cyclopropyl)pentyl, 3-(Cyclopropyl)pentyl, 4-(Cyclopropyl)pentyl, 5-(Cyclopropyl)pentyl, 2-(Cyclobutyl)pentyl, 3-(Cyclobutyl)pentyl, 5-(Cyclobutyl)pentyl, 2-(Cyclopropyl)hexyl, 3-(Cyclopropyl)hexyl, 6-(Cyclopropyl)hexyl, insbesondere für Cyclopentylmethyl oder Cyclohexylmethyl;
- C₃-C₆-Cycloalkoxy für: Cyclopropoxy, Cyclobutoxy, Cyclopentoxy oder Cyclohexoxy;
- C₁-C₄-Alkoxy für: OCH₃, OC₂H₅, OCH₂-C₂H₅, OCH(CH₃)₂, n-Butoxy, 1-Methylpropoxy, 2-Methylpropoxy oder OC(CH₃)₃, insbesondere für OCH₃ oder OC₂H₅;
- C₁-C₆-Alkoxy für: C₁-C₄-Alkoxy wie vorstehend genannt, sowie z. B. n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy, insbesondere für OCH₃, OC₂H₅ oder OCH(CH₃)₂;
- (C₁-C₆-Alkoxy)carbonyl für: (C₁-C₄-Alkoxy)carbonyl wie vorstehend genannt, sowie z.B. n-Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, n-Hexoxycarbonyl, 1,1-Dimethylpropoxycarbonyl, 1,2-Dimethylpropoxycarbonyl, 1-Methylpentoxycarbonyl; 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl oder 1-Ethyl-2-methyl-propoxycarbonyl, insbesondere für Methoxycarbonyl, Ethoxycarbonyl oder 1-Methylethoxycarbonyl;
- Cyano-C₁-C₆-alkyl für z. B. CH₂CN, 1-Cyanoeth-1-yl, 2-Cyanoeth-1-yl, 1-Cyanoprop-1-yl, 2-Cyanoprop-1-yl, 3-Cyanoprop-1-yl, 1-Cyanoprop-2-yl, 2-Cyanoprop-2-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 1-Cyanobut-3-yl, 2-Cyanobut-3-yl, 1-Cyano-2-methyl-prop-3-yl, 2-Cyano-2-methylprop-3-yl, 3-Cyano-2-methyl-prop-3-yl oder 2-Cyanomethylprop-2-yl, insbesondere für CH₂CN oder 2-Cyanoethyl;
- Cyano-C₁-C₆-alkoxy für: 1-Cyano-1-ethoxy, 2-Cyano-1-ethoxy, 1-Cyano-1-propoxy, 2-Cyano-1-propoxy, 3-Cyano-1-propoxy, 1-Cyano-2-propoxy, 1-Cyano-1-butoxy, 2-Cyano-1-butoxy, 3-Cyano-1-butoxy, 4-Cyano-1-butoxy, 1-Cyano-2-butoxy, 2-Cyano-2-butoxy, 1-Cyano-3-butoxy, 2-Cyano-3-butoxy, 1-Cyano-2-methyl-3-propoxy, 2-Cyano-2-methyl-3-propoxy, 3-Cyano-2-methyl-3-propoxy oder 2-Cyanomethyl-2-propoxy insbesondere 2-Cyano-1-ethoxy oder 3-Cyano-1-propoxy;
- C₁-C₆-Halogenalkyl für: C₁-C₄-Alkyl wie vorstehend genannt, das partiell oder vollständig durch Fluor, Chlor und/oder Brom substituiert ist, also z. B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Chlorethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 1-Chlor-1,2,2-trifluorethyl, 2,2-Dichlor-2-fluorethyl, 1,2,2-Trifluorethyl, 2,2,2-Trichlorethyl, Pentafluorethyl oder 3-Chlorpropyl, vorzugsweise Trifluormethyl;
- C₃-C₆-Halogenalkenyl für: C₃-C₆-Alkenyl wie vorstehend genannt, das partiell oder vollständig durch Fluor, Chlor und/oder Brom substituiert ist, also z. B. 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl, insbesondere für 2-Chlorallyl oder 3,3-Dichlorallyl;
- C₃-C₆-Halogenalkinyl für: C₃-C₆-Alkinyl wie vorstehend genannt, das partiell oder vollständig durch Fluor, Chlor und/oder Brom substituiert ist, also z. B. 3-Chlorpropargyl, 3-Brompropargyl, 3-Fluorpropargyl, 3,3,3-Trifluaxpropargyl, 4-Chlor-but-2-inyl, 4-Brom-but-2-inyl, 4,4,4-Trifluorbut-2-inyl, 1,4-Dichlor-but-2-inyl, 5-Chlor-pent-3-inyl, 5-Fluor-pent-3-inyl, 5,5,5-Trifluor-pent-3-inyl, 6-Chlorhex-2-inyl, vorzugsweise 3-Chlorpropargyl, 3,3,3-Trifluorpropargyl, 4,4,4-Trifluor-but-2-inyl;
- C₁-C₄-Halogenalkoxy für: einen C₁-C₄-Alkoxyrest wie vorstehend genannt, der partiell oder vollständig durch Fluor, Chlor, Brom und/oder Iod substituiert ist, also z. B. OCH₂F, OCHF₂, OCF₃, OCH₂Cl, OCH(Cl)₂, OC(Cl)₃, Chlorfluormethoxy, Dichlorfluormethoxy, Chlordifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, OC₂F₅, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, OCH₂-C₂F₅, OCF₂-C₂F₅, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy, insbesondere für 2-Chlorethoxy oder 2,2,2-Trifluorethoxy;
- C₁-C₆-Halogenalkoxy für: einen C₁-C₆-Alkoxyrest wie vorstehend genannt, der partiell oder vollständig durch Fluor, Chlor, Brom und/oder Iod substituiert ist, also z. B, einen unter C₁-C₄-Halogenalkoxy genannten Reste oder für 5-Fluor-1-pentoxy, 5-Chlor-1-pentoxy, 5-Brom-1-pentoxy, 5-Iod-1-pentoxy, 5,5,5-Trichlor-1-pentoxy, Undecafluorpentoxy, 6-Fluor-1-hexoxy, 6-Chlor-1-hexoxy, 6-Brom-1-hexoxy, 6-Iod-1-hexoxy, 6,6,6-Trichlor-1-hexoxy oder Dodecafluorhexoxy, insbesondere für OCH₂F, OCHF₂, OCF₃, OCH₂Cl, 2-Fluorethoxy, 2-Chlorethoxy oder 2,2,2-Trifluorethoxy;
- C₃-C₆-Alkenyloxy für: Prop-1-en-1-yloxy, Prop-2-en-1-yloxy, 1-Methylethenyloxy, n-Buten-1-yloxy, n-Buten-2-yloxy, n-Buten-3-yloxy, 1-Methyl-prop-1-en-1-yloxy, 2-Methyl-prop-1-en-1-yloxy, 1-Methyl-prop-2-en-1-yloxy, 2-Methyl-prop-2-en-1-yloxy, n-Penten-1-yloxy, n-Penten-2-yloxy, n-Penten-3-yloxy, n-Penten-4-yloxy, 1-Methyl-but-1-en-1-yloxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methyl-but-2-en-1-yloxy, 1-Methyl-but-3-en-1-yloxy, 2-Methyl-but-3-en-1-yloxy, 3-Methyl-but-3-en-1-yloxy, 1,1-Dimethyl-prop-2-en-1-yloxy, 1,2-Dimethyl-prop-1-en-1-yloxy, 1,2-Dimethyl-prop-2-en-1-yloxy, 1-Ethyl-prop-1-en-2-yloxy, 1-Ethylprop-2-en-1-yloxy, n-Hex-1-en-1-yloxy, n-Hex-2-en-1-yloxy, n-Hex-3-en-1-yloxy, n-Hex-4-en-1-yloxy, n-Hex-5-en-1-yloxy, 1-Methyl-pent-1-en-1-yloxy, 2-Methyl-pent-1-en-1-yloxy, 3-Methyl-pent-1-en-1-yloxy, 4-Methyl-pent-1-en-1-yloxy, 1-Methyl-pent-2-en-1-yloxy, 2-Methyl-pent-2-en-1-yloxy, 3-Methyl-pent-2-en-1-yloxy, 4-Methyl-pent-2-en-1-yloxy, 1-Methyl-pent-3-en-1-yloxy, 2-Methyl-pent-3-en-1-yloxy, 3-Methyl-pent-3-en-1-yloxy, 4-Methyl-pent-3-en-1-yloxy, 1-Methyl-pent-4-en-1-yloxy, 2-Methyl-pent-4-en-1-yloxy, 3-Methyl-pent-4-en-1-yloxy, 4-Methyl-pent-4-en-1-yloxy, 1,1-Dimethyl-but-2-en-1-yloxy, 1,1-Dimethyl-but-3-en-1-yloxy, 1,2-Dimethyl-but-2-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 2,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, 1-Ethyl-but-2-en-1-yloxy, 1-Ethyl-but-3-en-1-yloxy, 2-Ethyl-but-1-en-1-yloxy, 2-Ethyl-but-2-en-1-yloxy, 2-Ethyl-but-3-en-1-yloxy, 1,1,2-Trimethyl-prop-2-en-1-yloxy, 1-Ethyl-1-methyl-prop-2-en-1-yloxy, 1-Ethyl-2-methyl-prop-1-en-1-yloxy oder 1-Ethyl-2-methyl-prop-2-en-1-yloxy, vorzugsweise Ethenyloxy oder Prop-2-en-1-yloxy;
- C₃-C₆-Alkinyloxy für: Porpargyloxy, Prop-1-in-1-oxy, But-1-in-3-oxy, 3-Methyl-but-1-in-3-oxy, 3,3-Dimethyl-but-1-in-4-oxy, Pent-1-in-3-oxy, 3-Methyl-pent-1-in-3-oxy, Hex-3-in-5-oxy, insbesondere Propargyloxy, But-1-in-3-oxy oder 3-Methyl-but-1-in-3-oxy;
- C₁-C₆-Alkylthio-C₁-C₆-alkoxy für: durch C₁-C₆-Alkylthio wie vorstehend genannt substituiertes C₁-C₆-Alkoxy, also z.B. für Methylthiomethoxy, Ethylthiomethoxy, n-Propylthiomethoxy, (1-Methylethylthio)methoxy, n-Butylthiomethoxy, (1-Methylpropylthio)methoxy, (2-Methylpropylthio)methoxy, (1,1-Dimethylethylthio)methoxy, 2-(Methylthio)ethoxy, 2-(Ethylthio)ethoxy, 2-(n-Propylthio)ethoxy, 2-(1-Methylethylthio)ethoxy, 2-(n-Butylthio)ethoxy, 2-(1-Methylpropylthio)ethoxy, 2-(2-Methylpropylthio)ethoxy, 2-(1,1-Dimethylethylthio)ethoxy, 2-(Methylthio)propoxy, 2-(Ethylthio)-propoxy, 2-(n-Propylthio)propoxy, 2-(1-Methylethylthio)-propoxy, 2-(n-Butylthio)propoxy, 2-(1-Methylpropylthio)-propoxy, 2-(2-Methylpropylthio)propoxy, 2-(1,1-Dimethylethylthio)propoxy, 3-(Methylthio)propoxy, 3-(Ethylthio)propoxy, 3-(n-Propylthio)propoxy, 3-(1-Methylethylthio)propoxy, 3-(n-Butylthio)propoxy, 3-(1-Methylpropylthio)propoxy, 3-(2-Methylpropylthio)propoxy, 3-(1,1-Dimethylethylthio)-propoxy, 2-(Methylthio)butoxy, 2-(Ethylthio)butoxy, 2-(n-Propylthio)butoxy, 2-(1-Methylethylthio)butoxy, 2-(n-Butylthio)butoxy, 2-(1-Methylpropylthio)butoxy, 2-(2-Methylpropylthio)butoxy, 2-(1,1-Dimethylethylthio)-butoxy, 3-(Methylthio)butoxy, 3-(Ethylthio)butoxy, 3-(n-Propylthio)butoxy, 3-(1-Methylethylthio)butoxy, 3-(n-Butylthio)butoxy, 3-(1-Methylpropylthio)butoxy, 3-(2-Methylpropylthio)butoxy, 3-(1,1-Dimethylethylthio)-butoxy, 4-(Methylthio)butoxy, 4-(Ethylthio)butoxy, 4-(n-Propylthio)butoxy, 4-(1-Methylethylthio)butoxy, 4-(n-Butylthio)butoxy, 4-(1-Methylpropylthio)butoxy, 4-(2-Methylpropylthio)butoxy, 4-(1,1-Dimethylethylthio)butoxy, 5-(Methylthio)pentoxy, 5-(Ethylthio)pentoxy, 5-(n-Propylthio)pentoxy, 5-(1-Methylethylthio)pentoxy, 5-(n-Butylthio)pentoxy, 5-(1-Methylpropylthio)pentoxy, 5-(2-Methylpropylthio)pentoxy, 5-(1,1-Dimethylethylthio)pentoxy, 6-(Methylthio)hexoxy, 6-(Ethylthio)hexoxy, 6-(n-Propylthio)hexoxy, 6-(1-Methylethylthio)hexoxy, 6-(n-Butylthio)hexoxy, 6-(1-Methylpropylthio)hexoxy, 6-(2-Methylpropylthio)hexoxy oder 6-(1,1-Dimethylethylthio)hexoxy, insbesondere für Methylthiomethoxy oder Ethylthioethoxy;
- C₁-C₆-Alkylthio für: SCH₃, SC₂H₅, SCH₂-C₂H₅, SCH(CH₃)₂, n-Butylthio, SCH(CH₃)-C₂H₅, 2-Methylpropylthio, 1,1-Dimethylethylthio, n-Pentylthio, 1-Methylbutylthio, 2-Methylbutylthio, 3-Methylbutylthio, 2,2-Dimethylpropylthio, 1-Ethylpropylthio, n-Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio, insbesondere für SCH₃ oder SC₂H₅;
- C₁-C₆-Alkylthio-C₁-C₆-alkyl für: durch C₁-C₆-Alkylthio wie vorstehend genannt substituiertes C₁-C₆-Alkyl, also z.B. für Methylthiomethyl, Ethylthiomethyl, n-Propylthiomethyl, (1-Methylethylthio)methyl, n-Butylthiomethyl, (1-Methylpropylthio)methyl, (2-Methylpropylthio)methyl, (1,1-Dimethylethylthio)methyl, 2-Methylthioethyl, 2-Ethylthioethyl, 2-(n-Propylthio)ethyl, 2-(1-Methylethylthio)ethyl, 2-(n-Butylthio)ethyl, 2-(1-Methylpropylthio)ethyl, 2-(2-Methylpropylthio)ethyl, 2-(1,1-Dimethylethylthio)ethyl, 2-(Methylthio)propyl, 3-(Methylthio)propyl, 2-(Ethylthio)-propyl, 3-(Ethylthio)propyl, 3-(Propylthio)propyl, 3-(Butylthio)propyl, 4-(Methylthio)butyl, 4-(Ethylthio)butyl, 4-(n-Propylthio)butyl oder 4-(n-Butylthio)butyl, insbesondere für 2-(Methylthio)ethyl;
- C₁-C₄-Alkylsulfonyl für: Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl oder 1,1-Dimethylethylsulfonyl, insbesondere Methylsulfonyl oder Ethylsulfonyl;
- C₁-C₆-Alkylsulfonyl für: Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl, 1,1-Dimethylethylsulfonyl, n-Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, n-Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl oder 1-Ethyl-2-methylpropylsulfonyl, insbesondere für Methylsulfonyl;
- C₁-C₄-Alkylsulfinyl für: Methylsulfinyl, Ethylsulfinyl, n-Propylsulfinyl, 1-Methylethylsulfinyl, n-Butylsulfinyl, 1-Methylpropylsulfinyl, 2-Methylpropylsulfinyl oder 1,1-Dimethylethylsulfinyl, insbesondere Methylsulfinyl oder Ethylsulfinyl;
- C₂-C₆-Alkylsulfinyl für: Methylsulfinyl, Ethylsulfinyl, n-Propylsulfinyl, 1-Methylethylsulfinyl, n-Butylsulfinyl, 1-Methylpropylsulfinyl, 2-Methylpropylsulfinyl, 1,1-Dimethylethylsulfinyl, n-Pentylsulfinyl, 1-Methylbutylsulfinyl, 2-Methylbutylsulfinyl, 3-Methylbutylsulfinyl, 1,1-Dimethylpropylsulfinyl, 1,2-Dimethylpropylsulfinyl, 2,2-Dimethylpropylsulfinyl, 1-Ethylpropylsulfinyl, n-Hexylsulfinyl, 1-Methylpentylsulfinyl, 2-Methylpentylsulfinyl, 3-Methylpentylsuifinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl oder 1-Ethyl-2-methylpropylsulfinyl, insbesondere für Methylsulfinyl;
- C₁-C₄-Alkoxy-C₁-C₄-alkyl für: durch C₁-C₄-Alkoxy wie Methoxy, Ethoxy, n-Propoxy, 1-Methylethoxy, n-Butoxy, 1-Methylpropoxy, 2-Methylpropoxy und 1,1-Dimethylethoxy substituiertes C₁-C₄-Alkyl, also z. B. für CH₂OCH₃, CH₂OC₂H₅, n-Propoxymethyl, (1-Methylethoxy)methyl, n-Butoxymethyl, (1-Methylpropoxy)-methyl, (2-Methylpropoxy)-methyl, (1,1-Dimethylethoxy)methyl, 2-(Methoxy)ethyl, 2-(Ethoxy)ethyl, 2-(n-Propoxy)ethyl, 2-(2-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(n-Propoxy)propyl, 3-(1-Methylethoxy)propyl, 3-(n-Butoxy)propyl, 3-(1-Methylpropoxy)propyl, 3-(2-Methylpropoxy)propyl, 3-(1,1-Dimethylethoxy)propyl, 2-(Methoxy)-butyl, 2-(Ethoxy)butyl, 2-(n-Propoxy)butyl, 2-(1-Methylethoxy)butyl, 2-(n-Butoxy)butyl, 2--(2-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, bevorzugt n-Propoxymethyl, (1-Methylethoxy)methyl, 2-(n-Propoxy)ethyl oder 2-(1-Methylethoxy)ethyl sowie besonders bevorzugt CH₂OCH₃, CH₂OC₂H₅, 2-Methoxyethyl oder 2-Ethoxyethyl;
- C₁-C₆-Alkoxy-C₁-C₆-alkyl für: durch C₁-C₆-Alkoxy wie vorstehend genannt substituiertes C₁-C₆-Alkyl, also z.B. für Methoxymethyl, Ethoxymethyl, n-Propoxymethyl, (1-Methylethoxy)methyl, n-Butoxymethyl, (1-Methylpropoxy)methyl, (2-Methylpropoxy)methyl, (1,1-Dimethylethoxy)methyl, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(n-Propoxy)propyl, 3-(1-Methylethoxy)propyl, 3-(n-Butoxy)propyl, 3-(2-Methylpropoxy)propyl, 3-(2-Methylpropoxy)propyl, 3-(1,1-Dimethylethoxy)propyl, 2-(Methoxy)-butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, insbesondere für Methoxymethyl oder 2-Methoxyethyl;
- C₁-C₄-Alkoxy-C₁-C₄-alkoxy für: durch C₁-C₄-Alkoxy wie Methoxy, Ethoxy, n-Propoxy, 1-Methylethoxy, n-Butoxy, 1-Methylpropoxy, 2-Methylpropoxy und 1,1-Dimethylethoxy substituiertes C₁-C₄-Alkoxy, also z. B. für OCH₂OCH₃, OCH₂OC₂H₅, n-Propoxymethoxy, (1-Methylethoxy)methoxy, n-Butoxymethoxy, (1-Methylpropoxy)-methoxy, (2-Methylpropoxy)-methoxy, (1,1-Dimethylethoxy)methoxy, 2-(Methoxy)ethoxy, 2-(Ethoxy)ethoxy, 2-(n-Propoxy)ethoxy, 2-(1-Methylethoxy)ethoxy, 2-(n-Butoxy)-ethoxy, 2-(1-Methylpropoxy)ethoxy, 2-(2-Methylpropoxy)ethoxy, 2-(1,1-Dimethyl-ethoxy)ethoxy, 2-(Methoxy)propoxy, 2-(Ethoxy)propoxy, 2-(n-Propoxy)propoxy, 2-(1-Methylethoxy)-propoxy, 2-(n-Butoxy)propoxy, 2-(1-Methylpropoxy)propoxy, 2-(2-Methylpropoxy)propoxy, 2-(1,1-Dimethylethoxy)propoxy, 3-(Methoxy)propoxy, 3-(Ethoxy)propoxy, 3-(n-Propoxy)propoxy, 3-(1-Methylethoxy)propoxy, 3-(n-Butoxy)propoxy, 3-(1-Methylpropoxy)propoxy, 3-(2-Methylpropoxy)propoxy, 3-(1,1-Dimethylethoxy)propoxy, 2-(Methoxy)butoxy, 2-(Ethoxy)butoxy, 2-(n-Propoxy)butoxy, 2-(1-Methylethoxy)butoxy, 2-(n-Butoxy)-butoxy, 2-(1-Methylpropoxy)butoxy, 2-(2-Methylpropoxy)butoxy, 2-(1,1-Dimethylethoxy)butoxy, 3-(Methoxy)butoxy, 3-(Ethoxy)butoxy, 3-(n-Propoxy)butoxy, 3-(1-Methylethoxy)-butoxy, 3-(n-Butoxy)butoxy, 3-(1-Methylpropoxy)butoxy, 3-(2-Methylpropoxy)butoxy, 3-(1,1-Dimethylethoxy)butoxy, 4-(Methoxy)butoxy, 4-(Ethoxy)butoxy, 4-(n-Propoxy)butoxy, 4-(1-Methylethoxy)butoxy, 4-(n-Butoxy)butoxy, 4-(1-Methylpropoxy)butoxy, 4-(2-Methylpropoxy)butoxy oder 4-(1,1-Dimethylethoxy)butoxy, bevorzugt n-Propoxymethoxy, (1-Methylethoxy)methoxy, 2-(n-Propoxy)ethoxy oder 2-(1-Methylethoxy)-ethoxy sowie besonders bevorzugt OCH₂OCH₃, OCH₂OC₂H₅, 2-Methoxyethoxy oder 2-Ethoxyethoxy;
- C₁-C₆-Alkoxy-C₁-C₆-alkoxy für: durch C₁-C₆-Alkoxy wie vorstehend genannt substituiertes C₁-C₆-Alkoxy, also z.B. für Methoxymethoxy, Ethoxymethoxy, n-Propoxymethoxy, (1-Methylethoxy)methoxy, n-Butoxymethoxy, (1-Methylpropoxy)methoxy, (2-Methylpropoxy)methoxy, (1,1-Dimethylethoxy)methoxy, 2-(Methoxy)ethoxy, 2-(Ethoxy)ethoxy, 2-(n-Propoxy)ethoxy, 2-(1-Methylethoxy)ethoxy, 2-(n-Butoxy)ethoxy, 2-(1-Methylpropoxy)ethoxy, 2-(2-Methylpropoxy)ethoxy, 2-(1,1-Dimethylethoxy)ethoxy, 2-(Methoxy)propoxy, 2-(Ethoxy)propoxy, 2-(n-Propoxy)propoxy, 2-(1-Methylethoxy)propoxy, 2-(n-Butoxy)propoxy, 2-(1-Methylpropoxy)propoxy, 2-(2-Methylpropoxy)propoxy, 2-(1,1-Dimethylethoxy)propoxy, 3-(Methoxy)-propoxy, 3-(Ethoxy)propoxy, 3-(n-Propoxy)propoxy, 3-(1-Methylethoxy)propoxy, 3-(n-Butoxy)propoxy, 3-(1-Methylpropoxy)propoxy, 3-(2-Methylpropoxy)propoxy, 3-(1,1-Dimethylethoxy)propoxy, 2-(Methoxy)butoxy, 2-(Ethoxy)butoxy, 2-(n-Propoxy)butoxy, 2-(1-Methylethoxy)butoxy, 2-(n-Butoxy)-butoxy, 2-(1-Methylpropoxy)butoxy, 2-(2-Methylpropoxy)butoxy, 2-(1,1-Dimethylethoxy)butoxy, 3-(Methoxy)butoxy, 3-(Ethoxy)-butoxy, 3-(n-Propoxy)butoxy, 3-(1-Methylethoxy)butoxy, 3-(n-Butoxy)butoxy, 3-(1-Methylpropoxy)butoxy, 3-(2-Methylpropoxy)butoxy, 3-(1,1-Dimethylethoxy)butoxy, 4-(Methoxy)-butoxy, 4-(Ethoxy)butoxy, 4-(n-Propoxy)butoxy, 4-(1-Methylethoxy)butoxy, 4-(n-Butoxy)butoxy, 4-(1-Methylpropoxy)butoxy, 4-(2-Methylpropoxy)butoxy, 4-(1,1-Dimethylethoxy)butoxy, 5-(Methoxy)pentoxy, 5-(Ethoxy)pentoxy, 5-(n-Propoxy)pentoxy, 5-(1-Methylethoxy)pentoxy, 5-(n-Butoxy)pentoxy, 5-(1-Methylpropoxy)pentoxy, 5-(2-Methylpropoxy)pentoxy, 5-(1,1-Dimethylethoxy)pentoxy, 6-(Methoxy)hexoxy, 6-(Ethoxy)hexoxy, 6-(n-Propoxy)hexoxy, 6-(1-Methylethoxy)hexoxy, 6-(n-Butoxy)-hexoxy, 6-(2-Methylpropoxy)hexoxy, 6-(2-Methylpropoxy)hexoxy oder 6-(1,1-Dimethylethoxy)hexoxy, insbesondere für Methoxymethoxy oder Ethoxymethoxy;
- Carboxy-C₁-C₆-alkyl für: durch Carboxy substituiertes C₁-C₆-Alkyl wie vorstehen genannt, also z. B. Carboxy-methyl, 2-Carboxy-1-ethyl, 3-Carboxy-1-propyl, 1-Carboxy-1-ethyl, 1-Carboxy-1-propyl, 3-Carboxy-2-propyl, 2-Carboxy-2-propyl, 1-Carboxy-1-butyl, 1-Carboxy-2-butyl, 2-Carboxy-2-butyl, 4-Carboxy-1-butyl, 4-Carboxy-2-butyl, 5-Carboxy-1-pentyl, 5-Carboxy-2-pentyl, 5-Carboxy-3-pentyl, 5-Carboxy-1-hexyl, insbesondere Carboxymethyl, 2-Carboxy-1-ethyl oder 1-Carboxy-1-ethyl;
- (C₁-C₆-Alkoxy)carbonyl-C₁-C₂-alkyl für: durch (C₁-C₆-Alkoxy)-carbonyl wie COOCH₃, COOC₂H₅, n-Propoxycarbonyl, COOCH(CH₃)₂, n-Butoxycarbonyl, 1-Methylpropoxycarbonyl, 2-Methylpropoxycarbonyl, COOC(CH₃)₃ n-Pentoxycarbonyl, 1-Methyl-butoxycarbonyl und n-Hexoxycarbonyl substituiertes C₁-C₂-Alkyl, also z. B. für CH₂-COOCH₃, CH₂-COOC₂H₅, n-Propoxycarbonyl-methyl, CH₂-COOCH(CH₃)₂, n-Butoxycarbonylmethyl, (1-Methylpropoxycarbonyl)methyl, (2-Methylpropoxycarbonyl)methyl, CH₂-COOC (CH₃)₃, n-Pentoxycarbonylmethyl, (1-Methylbutoxycarbonyl)-methyl, n-Hexoxycarbonylmethyl, 1-(Methoxycarbonyl)ethyl, 1-(Ethoxycarbonyl)ethyl, 1-(n-Propoxycarbonyl)ethyl, 1-(1-Methylethoxycarbonyl)ethyl, 1-(n-Butoxycarbonyl)ethyl, 1-(n-Pentoxycarbonyl)ethyl, 1-(1-Methylbutoxycarbonyl)ethyl, 1-(n-Hexoxycarbonyl)ethyl, 2-(Methoxycarbonyl)-ethyl, 2-(Ethoxycarbonyl)ethyl, 2-(n-Propoxycarbonyl)ethyl, 2-(1-Methylethoxycarbonyl)ethyl, 2-(n-Butoxycarbonyl)ethyl, 2-(1-Methylpropoxycarbonyl)ethyl, 2-(2-Methylpropoxycarbonyl)ethyl, 2-(1,1-Dimethylethoxycarbonyl)ethyl;
- (C₁-C₆-Alkoxy)carbonyl-C₁-C₄-alkyl für: (C₁-C₆-Alkoxy)carbonyl-C₁-C₂-alkyl wie vorstehend genannt, sowie 2-(Methoxycarbonyl)propyl, 2-(Ethoxycarbonyl)propyl, 2-(n-Propoxycarbonyl)propyl, 2-(1-Methylethoxycarbonyl)propyl, 2-(n-Butoxycarbonyl)propyl, 2-(1-Methylpropoxycarbonyl)-propyl, 2-(2-Methylpropoxycarbonyl)propyl, 2-(1,1-Dimethylethoxycarbonyl)propyl, 3-(Methoxycarbonyl)propyl, 3-(Ethoxycarbonyl)propyl, 3-(n-Propoxycarbonyl)propyl, 3-(1-Methylethoxycarbonyl)propyl, 3-(n-Buthoxycarbonyl)propyl, 3-(1-Methylpropoxycarbonyl)propyl, 3-(2-Methylpropoxycarbonyl)propyl, 3-(1,1-Dimethylethoxycarbonyl)propyl, 2-(Methoxycarbonyl)butyl, 2-(Ethoxycarbonyl)butyl, 2-(n-Propoxycarbonyl)butyl, 2-(1-Methylethoxycarbonyl)butyl, 2-(n-Butoxycarbonyl)butyl, 2-(2-Methylpropoxycarbonyl)butyl, 2-(2-Methylpropoxycarbonyl)butyl, 2-(1,1-Dimethylethoxycarbonyl)butyl, 3-(Methoxycarbonyl)butyl, 3-(Ethoxycarbonyl)butyl, 3-(n-Propoxycarbonyl)butyl, 3-(1-Methylethoxycarbonyl)butyl, 3-(n-Butoxycarbonyl)butyl, 3-(1-Methylpropoxycarbonyl)butyl, 3-(2-Methypropoxycarbonyl)butyl, 3-(1,1-Dimethylethoxycarbonyl)butyl, 4-(Methoxycarbonyl)-butyl, 4-(Ethoxycarbonyl)butyl, 4-(n-Propoxycarbonyl)butyl, 4-(1-Methylethoxycarbonyl)butyl, 4-(n-Butoxycarbonyl)butyl, 4-(1-Methylpropoxycarbonyl)butyl, 4-(2-Methylpropoxycarbonyl)butyl oder 4-(1,1-Dimethylethoxycarbonyl)butyl, vorzugsweise CH₂-COOCH₃, CH₂-COOC₂H₅, 1-(Methoxycarbonyl)ethyl, 2-Methoxycarbonyl)ethyl oder 1-(Ethoxycarbony)-ethyl;
- (C₁-C₆-Alkoxy)carbonyl-C₁-C₆-alkyl für: durch (C₁-C₆-Alkoxy)-carbonyl wie vorstehend genannt substituiertes C₁-C₆-Alkyl, also z.B. für Methoxycarbonylmethyl, Ethoxycarbonylmethyl, 1-(Methoxycarbonyl)ethyl, 2-(Methoxycarbonyl)ethyl, 2-(Ethoxycarbonyl)ethyl, 3-(Methoxycarbonyl)propyl, 4-(Methoxycarbonyl)butyl, 5-(Methoxycarbonyl)pentyl oder 6-(Methoxycarbonyl)hexyl;
- (C₁-C₆-Alkoxy)carbonyl-C₁-C₄-alkoxy für: durch (C₁-C₆-Alkoxy)-carbonyl wie vorstehend genannt substituiertes C₁-C₆-Alkoxy, also z. B. für Methoxycarbonyl-methoxy, Ethoxycarbonylmethoxy, n-Propoxycarbonyl-methoxy, n-Butoxycarbonyl-methoxy, 1-(Methoxycarbonyl)ethoxy, 2-(Methoxycarbonyl)ethoxy, 2-(Ethoxycarbonyl)ethoxy, 2-(n-Propoxycarbonyl)ethoxy, 2-(n-Butoxycarbonyl)ethoxy, 3-(Methoxycarbonyl)propoxy, 3-(Ethoxycarbonyl)propoxy, 3-(n-Propoxycarbonyl)propoxy, 3-(n-Butoxycarbonyl)propoxy, 4-(Methoxycarbonyl)butoxy, 4-(Ethoxycarbonyl)butoxy, 4-(n-Propoxycarbonyl)butoxy, 4-(n-Butoxycarbonyl)butoxy, 1-(Ethoxycarbonyl)ethoxy, 1-(Propoxycarbonyl)ethoxy, 2-(Methoxycarbonyl)-2-propoxy, 2-(Ethoxycarbonyl)-2-propoxy, 2-Methyl-2-(methoxycarbonyl)-2-propoxy, insbesondere für Methoxycarbonylmethoxy, 2-Methoxycarbonyl-ethoxy oder 1-(Methoxycarbonyl)ethoxy;
- C₃-C₆-Alkenyloxy-C₁-C₆-alkyl für: durch C₃-C₆-Alkenyloxy wie vorstehend genannt, vorzugsweise Allyloxy, 2-Methylprop-2-en-1-yloxy, But-1-en-3-yloxy, But-1-en-4-yloxy oder But-2-en-1-yloxy substituiertes C₁-C₆-Alkyl, also beispielsweise für Allyloxymethyl, 2-Allyloxyethyl oder But-1-en-4-yloxymethyl;
- C₃-C₆-Alkinyloxy-C₁-C₆-alkyl für: durch C₃-C₆-Alkinyloxy wie vorstehend genannt, vorzugsweise Propargyloxy, But-1-in-3-yloxy, But-1-in-4-yloxy oder But-2-in-1-yloxy, substituiertes C₁-C₆-Alkyl, also beispielsweise für Propargyloxymethyl oder 2-Propargyloxyethyl;
- C₃-C₆-Cycloalkoxy-C₁-C₄-alkyl für: Cyclopropyloxymethyl, Cyclobutyloxymethyl, Cyclopentyloxymethyl, Cyclohexyloxymethyl, 1-(CyClopropyloxy)ethyl, 1-(Cyclobutyloxy)ethyl, 1-(Cyclopentyloxy)ethyl, 1-(Cyclohexyloxy)ethyl, 2-(Cyclopropyloxy)ethyl, 2-(Cyclobutyloxy)ethyl, 2-(Cyclopentyloxy)ethyl, 2-(Cyclohexyloxy)ethyl, 3-(Cyclopropyloxy)propyl, 3-(Cyclobutyloxy)propyl, 3-(Cyclopentyloxy)propyl, 3-(Cyclohexyloxy)propyl, 4-(Cyclopropyloxy)butyl, 4-(Cyclobutyloxy)butyl, 4-(Cyclopentyloxy)butyl oder 4-(Cyclohexyloxy)butyl, insbesondere für Cyclopentyloxymethyl, Cyclohexyloxymethyl oder 2-(Cyclopentyloxy)ethyl.

Die im erfindungsgemäßen Verfahren als Einsatzstoffe verwendeten N'-substituierten N-Aminoharnstoffderivate der Formel II wie auch Verfahren zu deren Herstellung sind an sich bekannt und in der Literatur, z.B. in der WO 94/10173 beschrieben, so daß hier wegen näherer Einzelheiten auf diese entsprechende Literatur verwiesen werden soll.

Im folgenden werden die Reaktionsbedingungen und die Durchführung des Verfahrens unter Bezugnahme auf die Herstellung der neuen anellierten Triazole I' beschrieben; die Angaben lassen sich aber auf die Herstellung aller Verbindungen der Formel I gemäß Anspruch 1 übertragen.

Bei der Anwendung des erfindungsgemäßen Verfahrens zur Herstellung der erfindungsgemäßen Verbindungen I' in guten Ausbeuten und hoher Reinheit ergibt sich folgendes Schema:

Im ersten Schritt werden substituierte N-Amino-N'-phenylharnstoffe der Formel II' 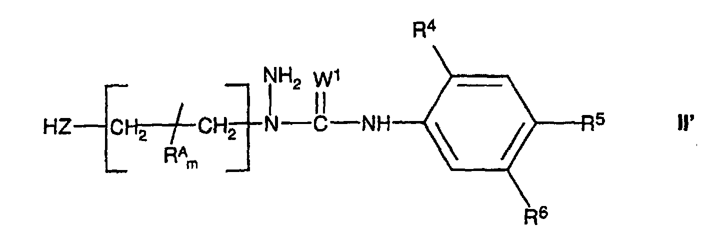 in der
- Z =: Sauerstoff oder Schwefel,
- R^{A},W¹,R⁴,R⁵ und R⁶: die vorgenannten Bedeutungen besitzen und
- m =: 0, 1 oder 2 bedeuten,
mit wässrigem Formaldehyd oder Paraformaldehyd säurefrei zu den neuen N-Methylenimino-N'-phenylharnstoffen der Formel III' 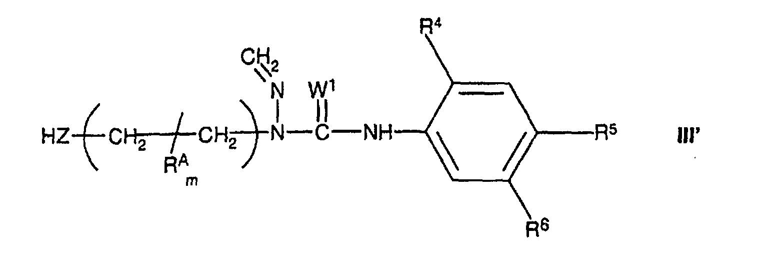 umgesetzt,
diese werden dann in Gegenwart von Säure oder eines neutralen oder sauren oberflächenaktiven Metalloxids zu den 4-(Phenylcarbamoyl)-tetrahydro-4H-1,3,4-ox(bzw. thia)diazinen der Formel IV' 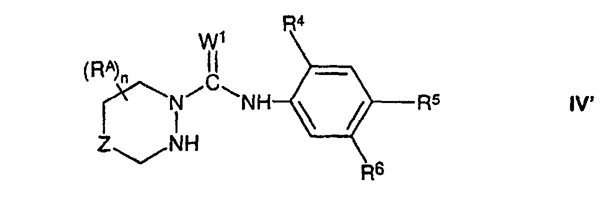 cyclisiert und letztere mit Phosgen oder einem Phosgenersatz zu den Verbindungen der Formel I' cyclisiert.

Im Falle der Verwendung eines N-Amino-N-2-hydroxyethyl-N'-phenylsubstituierten Harnstoffes und wässrigem Formaldehyd läßt sich das erfindungsgemäße Verfahren durch folgende Formel wiedergeben:

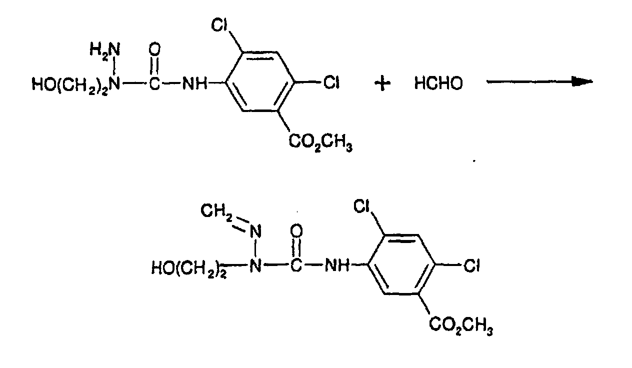

Die anschließende Cyclisierung zu dem substituierten 4-(Phenylcarbamoyl)-tetrahydro-4H-1,3,4-ox(bzw. thia)diazin IV' in Gegenwart von Essigsäure läßt sich nach dem erfindungsgemäßen Verfahren durch folgenden Formel wiedergeben:

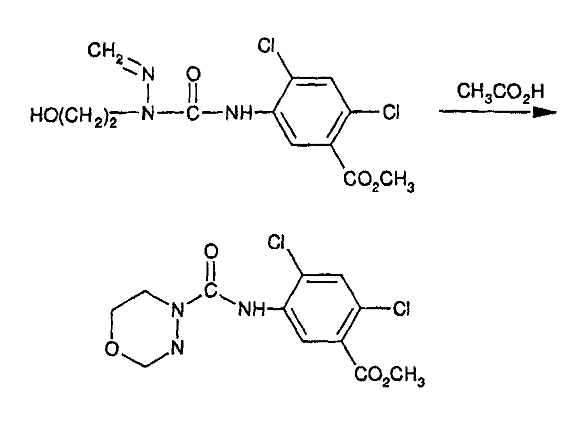

An Stelle einer Säure, beispielsweise Essigsäure kann als Katalysator auch ein oberflächenaktives Metalloxid für den ersten Cyclisierungsschritt verwendet werden.

Der letzte Cyclisierungsschritt zu den erfindungsgemäßen anellierten Triazolen kann bei Verwendung von Phosgen und einem weiteren substituierten 4-(Phenylcarbamoyl)-tetrahydro-4H-1,3,4-ox(bzw, thia)diazin durch folgende Formel wiedergegeben werden:

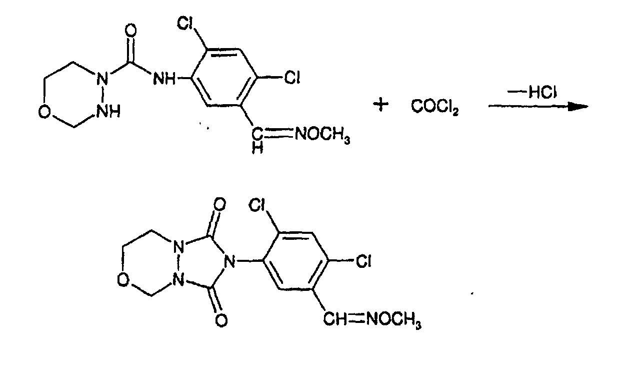

Die Umsetzung der N-Amino-N'-phenylharnstoffe II' mit Formaldehyd oder Paraformaldehyd wird vorteilhaft in Gegenwart eines Lösungsmittels bei Temperaturen im Bereich von 0 - 150°C, vorzugsweise 10 - 100°C, besonders bevorzugt 20 - 60°C durchgeführt.

Als Lösungsmittel wählt man für diese Umsetzungen - je nach Temperaturbereich - Kohlenwasserstoffe wie Pentan, Hexan, Heptan, Cyclohexan, Aromaten, z. B. Benzol, Toluol, Xylol, Heteroaromaten, z. B. Pyridin, α,β,γ-Picolin und Chinolin, chlorierte Kohlenwasserstoffe, z. B. Methylenchlorid, 1,1-Dichlorethan, 1,2-Dichlorethan, 1,1,2,2-Tetrachlorethan, 1,1-Dichlorethylen, Chlorbenzol, 1,2-, 1,3-, 1,4-Dichlorbenzol, 1-Chlornaphthalin und 1,2,4-Trichlorbenzol, Ether wie 1,4-Dioxan, Anisol, Glykolether wie Dimethylglykolether, Diethylglykolether, Diethylenglykoldimethylether, Ester wie Ethylacetat, Propylacetat, Methylisobutyrat, Isobutylacetat, Carbonsäureamide wie DMF, N-Methylpyrrolidon, Nitrokohlenwasserstoffe wie Nitromethan, Nitroethan, Nitropropan und Nitrobenzol, Harnstoffe wie Tetraethylharnstoff, Tetrabutylharnstoff, Dimethylethylenharnstoff, Dimethylpropylenharnstoff, Sulfoxide wie Dimethylsulfoxid, Sulfone wie Dimethylsulfon, Diethylsulfon, Tetramethylensulfon, Nitrile wie Acetonitril, Propionitril, Butyronitril oder Isobutyronitril; Wasser oder auch Gemische einzelner Lösungsmittel.

Die molaren Verhältnisse, in denen die Ausgangsverbindungen miteinander umgesetzt werden, betragen im allgemeinen 0,9 - 1,4, vorzugsweise 0,95 - 1,2, besonders bevorzugt 0,98 - 1,15 für das Verhältnis von Aldehyd zu N-Amino-N'-phenylharnstoff II'. Die Konzentration der Edukte im Lösungsmittel beträgt im allgemeinen 0,1 - 5 Mol/l, bevorzugt 0,2-2 Mol/l.

Vorteilhaft gibt man wässrigen Formaldehyd, bevorzugt als 37 %ige Lösung während 2 bis 20 min. zu einer Mischung des N-Amino-N'phenylharnstoffes II' in einem der vorgenannten Lösungsmittel bei 10 - 25°C und rührt dann zur Vervollständigung der Reaktion noch 0,5 bis 12 Stunden, vorzugsweise 1 bis 3 Stunden bei 20 - 60°C nach.

Man kann jedoch auch den N-Amino-N'-phenylharnstoff II' zu einer Mischung von Formaldehya in einem der vorgenannten Lösungsmittel geben und dann wie oben die Reaktion zu Ende führen.

An Stelle von wässrigem Formaldehyd kann man auch Paraformaldehyd einsetzen.

Eine Entfernung des Reaktionswassers ist im allgemeinen nicht erforderlich; man kann jedoch das Reaktionswasser auch über einen Wasserausscheider während der Reaktion abtrennen.

Die Reaktion wird unter Ausschluß saurer Katalysatoren, d.h. in neutralen bis leicht alkalischem Milieu durchgeführt. Gegebenenfalls neutralisiert man saure Verunreinigungen durch Zugabe basischer Verbindungen, z.B. Alkali- oder Erdalkalihydroxide bzw. -hydrogencarbonate oder -carbonate. Gegebenenfalls kann man auch organische Basen zusetzen oder arbeitet unter Verwendung eines basischen Lösungsmittelanteils wie Pyridin.

Die Reaktion kann drucklos oder unter Druck kontinuierlich oder diskontinuierlich durchgeführt werden.

Die Cyclisierung der N-Methylenimino-N'-phenyl-harnstoffe III' zu den 4-(Phenylcarbamoyl)-tetra-hydro-4H-1,3,4-ox(bzw. thia)-diazinen IV' wird unter Zugabe von im allgemeinen 1 bis 100 Gew.% einer Säure bez. auf III', vorteilhaft in Gegenwart eines der vorgenannten Lösungsmittel, bei Temperaturen im Bereich von 0 - 150°C, vorzugsweise 10 - 120°C, besonders bevorzugt 20 - 80°C durchgeführt.

Als Säure kann man aromatische Sulfonsäuren, z. B. Benzolsulfonsäure, p-Chlor- oder p-Toluolsulfonsäure, aliphatische Sulfonsäuren wie Methansulfonsäure, Trifluormethansulfonsäure, Ethansulfonsäure und n-Propylsulfonsäure, Sulfaminsäuren wie Methylsulfaminsäure, Ethylsulfaminsäure oder Isopropylsulfaminsäure, aliphatische Carbonsäuren wie Essigsäure, Trifluoressigsäure, Propionsäure, Buttersäure oder Isobuttersäure sowie anorganische Säuren wie Chlorwasserstoffsäure, Schwefelsäure, Salpetersäure oder Borsäure verwenden. Zweckmäßig kann man eine Säure wie Essigsäure oder Propionsäure direkt auch als Reaktionsmedium verwenden.

Vorteilhaft gibt man den N-Methylenimino-N'-phenylharnstoff III' während 2 bis 20 min bei 10 - 25°C zu der organischen Säure, bevorzugt Essigsäure als Reaktionsmedium und rührt noch 0,5 bis 12 Stunden, vorzugsweise 1 bis 3 Stunden bei 20 - 80°C nach. Man kann jedoch auch die Säure direkt zu der Reaktionslösung der aus N-Amino-N'-phenylharnstoff II' und Formaldehyd gebildeten N-Methylenimino-N'-phenylharnstoffstufe III' geben und diese ohne Isolierung, ggf. nach Abdestillation eines Lösungsmittelteils, zu dem 4-(Phenylcarbamoyl)-tetrahydro-4H-1,3,4-ox(bzw. thia)diazin IV' cyclisieren.

Die Konzentration der Edukte im Lösungsmittel beträgt im allgemeinen 0,1 - 5 Mol/l, bevorzugt 0,2 - 2 Mol/l.

An Stelle einer Säure kann man als Katalysator auch ein neutrales oder saures oberflächenaktives Metalloxid, beispielsweise Aluminiumoxid, Eisenoxid, Boroxid, Siliciumdioxid, Titandioxid, Arsenoxid, Antimonoxid, Chromoxid oder Manganoxid verwenden.

Die Reaktion kann drucklos oder unter Druck, kontinuierlich oder diskontinuierlich betrieben werden.

Die Cyclisierung der 4-(Phenylcarbamoyl)-tetrahydro-4H-1,3,4-ox(bzw. thia)diazine IV' zu den Verbindungen der Formel I wird mittels Phosgen oder einem Phosgenersatz, z.B. Diphosgen (ClC(=O)OCCl₃), vorteilhaft in Gegenwart eines der vorgenannten wasserfreien Lösungsmittel bei Temperaturen im Bereich von (-10) - 120°C, vorzugsweise 0 - 80°C, besonders bevorzugt 10 - 60°C durchgeführt.

Vorteilhaft leitet man das Phosgen bei 10 - 60°C unter Rühren in eine Mischung eines 4-(Phenylcarbamoyl)-tetrahydro-4H-1,3,4-ox(bzw. thia)diazins IV' und einer Menge von 0,5 - 5 Gew. %, bez. auf IV', Aktivkohle als Katalysator in einem der vorgenannten wasserfreien Lösungsmittel während 0,5 -20 Stunden, bevorzugt 1 -12 Stunden.

Die Reaktion kann zusätzlich durch einen basischen Amid-Katalysator beschleunigt werden, z. B. Dimethylformamid, das üblicherweise in einer Menge von 0,3 bis 10 Gew.% bez. auf IV' eingesetzt werden kann. Als basischen Katalysator kann man auch organische Basen wie Triethylamin, Tri-n-propylamin, N,N-Dimethylanilin oder N,N-Dimethylcyclohexylamin verwenden. Vorzugsweise kann man auch Pyridin, ggf. direkt als Lösungsmittel einsetzen.

An Stelle von Phosgen kann man auch Diphosgen verwenden. Vorteilhaft gibt man das Diphosgen während 2 bis 20 min. unter Rühren bei 0 bis (-5)°C zu der Mischung des Ausgangsstoffes IV' und einem der vorgenannten Lösungsmittel, ggf. unter Zusatz von Aktivkohle, Dimethylformamid oder der organischen Base, läßt innerhalb 1 Stunde auf 10°C erwärmen und rührt dann noch 1 - 12 Stunden bei 10 bis 60°C.
Die molare Menge an Phosgen bzw. Diphosgen beträgt 0,98 bis 5, vorzugsweise 1 bis 3, besonders bevorzugt 1 bis 1,3 pro mol Ausgangsstoff IV'.

Die Konzentration der Edukte im Lösungsmittel beträgt im allgemeinen 0,1-5 Mol/l, bevorzugt 0,2-2 mol/l.

Die Reaktion kann drucklos oder unter Druck, kontinuierlich oder diskontinuierlich durchgeführt werden.

Vorteilhaft kann man die mehrstufige Reaktion auch als Eintopfverfahren durchführen, wobei man im ersten Synthesschritt bei der Umsetzung der N-Amino-N'-phenylharnstoffe II' mit Formaldehyd das Reaktionswasser entfernt, die gebildeten N-Methylenimino-N'phenylharnstoffe III' unter Zugabe eines neutralen oder sauren Katalysators zu den 4-(phenylcarbamoyl)-tetrahydro-4H-1,3,4-ox(bzw. thia)diazinen IV' cyclisiert und diese dann mit Phosgen oder Diphosgen ggf. unter Zusatz von Aktivkohle oder einem Amid-Katalysator oder in Gegenwart einer Base zu den Endstoffen I' cyclisiert. Ggf. trennt man vor der Phosgencyclisierung durch Phasentrennung oder Destillation vorhandene saure Katalysatoren ab und führt dann den Ringschluß zu den Endstoffen I' durch.

Zur Aufarbeitung nimmt man die Zwischenprodukte III' - IV' in einem mit Wasser nicht mischbaren Lösungsmittel auf, extrahiert saure Verunreinigungen bzw. Oxidationsmittel mit verdünntem Alkali- bzw. Wasser, trocknet und entfernt das Lösungsmittel unter reduziertem Druck.

Grundsätzlich sind die anellierten Triazole I nach dem vorstehend genannten erfindungsgemäßen Syntheseverfahren herstellbar. Aus wirtschaftlichen oder verfahrenstechnischen Gründen kann es jedoch zweckmäßiger sein, einige Verbindungen I aus ähnlichen anellierten Triazolen, die sich jedoch in der Bedeutung eines Restes unterscheiden, herzustellen.

Die Aufarbeitung der Reaktionsgemische erfolgt in der Regel nach an sich bekannten Methoden, beispielsweise durch Verdünnen der Reaktionslösung mit Wasser und anschließender Isolierung des Produktes mittels Filtration, Kristallisation oder Lösungsmittelextraktion, oder durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die anellierten Triazole der Formel I können ein oder mehrere Chiralitätszentren enthalten und fallen dann üblicherweise als Enantiomeren- oder Diastereomerengemische an. Die Mischungen können gewünschtenfalls nach den hierfür üblichen Methoden wie Kristallisation oder Chromatographie, auch an einem optisch aktiven Adsorbat, in die weitgehend reinen Isomeren getrennt werden. Reine optische aktive Isomere lassen sich beispielsweise auch aus entsprechenden optisch aktiven Ausgangsmaterialien herstellen.

Diejenigen anellierten Triazole mit R²⁰ = OH, R²² und R²⁵ = Wasserstoff lassen sich auf an sich bekannte Weise in ihre Salze, vorzugsweise in ihre Alkalimetallsalze, überführen.

Salze von I, deren Metallion kein Alkalimetallion ist, können durch Umsalzen des entsprechenden Alkalimetallsalzes in üblicher Weise hergestellt werden, ebenso Ammonium-, Phosphonium-, Sulfonium- oder Sulfoxoniumhydroxiden.

Besonders vorteilhaft läßt sich das erfindungsgemäße Verfahren zur Herstellung von anellierten Triazolen der Formel I einsetzen, in denen Q für Q-1 und R⁴ für ein Halogenatom, insbesondere Fluor steht. Derartige Triazole sind nach dem in der WO 94/10173 beschriebenen Verfahren nur in geringer Ausbeute zugänglich. In der Regal erhält man ein nur schwer zu trennendes Gemisch verschiedener Produkte.

Eine weitere Gruppe anellierter Triazole, die nach dem erfindungsgemäßen Verfahren gut zugänglich sind, sind die neuen anellierten Triazole der Formel I', die einen weiteren Gegenstand der Erfindung darstellen: 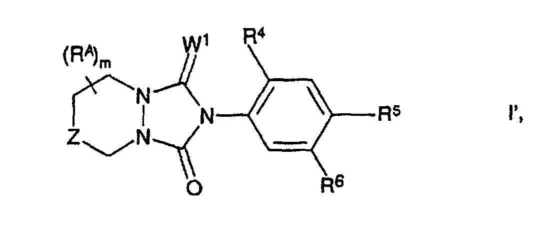 in der die Variablen folgende Bedeutungen haben:
- Z: O, S, S=O oder SO₂;
- R^{A}: Halogen oder C₁-C₃-Alkyl;
- W¹: Sauerstoff oder Schwefel;
- R⁴: Wasserstoff oder Halogen;
- R⁵: Halogen, Cyano oder Trifluormethyl;
- R⁶: eine Gruppe -C(R¹⁸)=C(R¹⁹)-CO-R²⁰, -CH(R¹⁸)-CH(R¹⁹)-CO-R²⁰, -C(R¹⁸)=C(R¹⁹)-CO-N(R²⁰,R²¹),-CH(R¹⁸)-CH(R¹⁹)-CO-N(R²⁰,R²¹), -C(R²¹)=N-OR²², -CO-OC(R²³)(R²⁴)-CO-OR²⁵, CO-N(R²⁶)-OR²² oder C(OR²⁷)=N-OR²²;
- R¹⁸, R²³, R²⁴: jeweils Wasserstoff oder C₁-C₃-Alkyl;
- R¹⁹: Halogen, Cyano oder Methyl;
- R²⁰: Hydroxy, C₁-C₆-Alkoxy, C₃-C₆-Cycloalkoxy, C₁-C₄-Alkoxy-C₁-C₄-alkoxy, C₁-C₄-Alkylthio-C₁-C₄-alkoxy, C₁-C₄-Alkylsulfinyl-C₁-C₄-alkoxy, C₁-C₄-Alkylsulfonyl-C₁-C₄-alkoxy, Cyano-C₁-C₆-alkoxy, C₁-C₄-Alkoxycarbonyl-C₁-C₄-alkoxy, C₃-C₆-Alkenyloxy, C₃-C₆-Alkinyloxy, partiell oder vollständig halogeniertes C₁-C₆-Alkoxy, partiell oder vollständig halogeniertes C₃-C₆-Alkenyloxy, partiell oder vollständig halogeniertes C₃-C₆-Alkinyloxy, C₁-C₆-Alkylthio,
ferner C₁-C₆-Alkoxy, das noch zwei C₁-C₆-Alkoxysubstituenten tragen kann;
- R²¹: Wasserstoff, C₁-C₆-Alkyl oder C₁-C₆-Halogenalkyl;
- R²²: Wasserstoff, C₁-C₆-Alkyl, C₁-C₄-Alkoxy-C₁-C₄-alkyl, C₁-C₆-Halogenalkyl, C₁-C₆-Cyanoalkyl, C₃-C₆-cycloalkyl, C₃-C₆-Alkenyl, C₃-C₆-Alkinyl, C₃-C₆-Halogenalkenyl, C₃-C₆-Halogenalkinyl, Carboxy-C₁-C₆-alkyl, C₁-C₆-Alkoxycarbonyl-C₁-C₄-alkyl, C₁-C₆-Alkylcarbonyl-C₁-C₆-alkyl oder C₁-C₆-Alkylcarbonyloxy-C₁-C₆-alkyl;
- R²⁵: Wasserstoff, C₁-C₆-Alkyl, C₃-C₆-Cycloalkyl, C₁-C₆-Alkoxy-C₁-C₄alkyl, Cyano-C₁-C₆-alkyl, Halogen-C₁-C₆-alkyl, C₃-C₆-Alkenyl oder C₃-C₆-Alkinyl;
- R²⁶, R²⁷: unabhängig voneinander C₁-C₆-Alkyl, C₁-C₄-Alkoxy-C₁-C₄-alkyl, C₃-C₆-Cycloalkyl, C₃-C₆-Alkenyl, C₃-C₆-Alkinyl, C₁-C₆-Halogenalkyl, C₁-C₆-Cyanoalkyl, C₃-C₆-Halogenalkenyl, C₃-C₆-Halogenalkinyl oder C₁-C₆-Alkoxycarbonyl-C₁-C₄-alkyl;
- R²⁶: darüberhinaus Wasserstoff und
- m: 0, 1, 2 oder 3,
sowie die landwirtschaftlich brauchbaren Salze der Verbindungen I.

Im Hinblick auf die Verwendung der erfindungsgemäßen anellierten Triazole als Herbizide und/oder als desikkant/defoliant wirksame verbindungen haben die Substituenten und Index m vorzugsweise folgende Bedeutungen, und zwar jeweils für sich allein oder in Kombination:
- Z: Sauerstoff;
- W¹: Sauerstoff;
- R⁵: Chlor oder Cyano, insbesondere Chlor;
- R⁶: eine Gruppe -C(R¹⁸)=C(R¹⁹)-CO-R²⁰, -CH(R¹⁸)-CH(R¹⁹)-CO-R²⁰ -C(R²¹)=N-OR²², CO-OC(R²³)(R²⁴)-CO-OR²⁵, CO-N(R²⁶)-OR²² oder C (O-R²⁷)=N-OR²²;
- R¹⁸, R²³, R²⁴: jeweils Wasserstoff oder Methyl;
- R¹⁹: Halogen oder Cyano;
- R²⁰: C₁-C₆-Alkoxy, C₃-C₅-Cycloalkoxy, C₁-C₄-Alkoxy-C₁-C₄-alkoxy, C₁-C₄-Alkoxycarbonyl-C₁-C₄-alkoxy, C₃-C₆-Alkenyloxy, C₃-C₆-Alkinyloxy oder C₁-C₄-Halogenalkoxy;
- R²¹: Wasserstoff oder C₁-C₄-Alkyl;
- R²²: C₁-C₆-Alkyl, C₃-C₆-Alkenyl, C₃-C₆-Alkinyl oder C₁-C₆-Alkoxycarbonyl-C₁-C₂-alkyl;
- R²⁵: C₁-C₆-Alkyl, C₁-C₆-Alkoxy-C₁-C₄-alkyl, C₃-C₆-Alkenyl oder C₃-C₆-Alkinyl;
- R²⁶: Wasserstoff, C₁-C₆-Alkyl, C₁-C₄-Alkoxy-C₁-C₄-alkyl, C₃-C₆-Alkenyl, C₃-C₆-Alkinyl oder C₁-C₆-Alkoxy-carbonyl-C₁-C₂-alkyl;
- R²⁷: C₁-C₆-Alkyl, C₁-C₄-Alkoxy-C₁-C₄-alkyl, C₃-C₆-Alkenyl, C₃-C₆-Alkinyl oder C₁-C₆-Alkoxy-carbonyl-C₁-C₂-alkyl;
- m: Null.

Bevorzugte Verbindungen der Formel I' (mit W¹ = Sauerstoff und R⁵ = Chlor) sind in Tabelle 1, annelierte Triazole I' mit W¹ = Schwefel und R⁵ = Chlor in Tabelle 2 aufgeführt:

**Tabelle 1**

| **Bsp.- Nr.** | **R**^{**4**} | **Z** | **R**^{**6**} | **Schmelzpunkt oder** ^{**1**}**H-NMR (CDCl**_{**3**}**, δ [ppm])** |
|---|---|---|---|---|
| 1.01 | H | O | -CH=N-OH | |
| 1.02 | F | O | -CH=N-OH | 189-190°C |
| 1.03 | Cl | O | -CH=N-OH | 204-207°C |
| 1.04 | H | O | -CH=N-OCH₃ | 166-168°C |
| 1.05 | F | O | -CH=N-OCH₃ | 173-175°C |
| 1.06 | Cl | O | -CH=N-OCH₃ | 184-185°C |
| 1.07 | H | O | -CH=N-OC₂H₅ | |
| 1.08 | F | O | -CH=N-OC₂H₅ | |
| 1.09 | Cl | O | -CH=N-OC₂H₅ | 95-103°C |
| 1.10 | H | O | -CH=N-OCH₂-C₂H₅ | |
| 1.11 | F | O | -CH=N-OCH₂-C₂H₅ | |
| 1.12 | Cl | O | -CH=N-OCH₂-C₂H₅ | |
| 1.13 | H | O | -CH=N-OCH(CH₃)₂ | |
| 1.14 | F | O | -CH=N-OCH(CH₃)₂ | |
| 1.15 | Cl | O | -CH=N-OCH(CH₃)₂ | |
| 1.16 | H | O | -CH=N-OCH₂-CH₂-OCH₃ | |
| 1.17 | F | O | -CH=N-OCH₂-CH₂-OCH₃ | |
| 1.18 | Cl | O | -CH=N-OCH₂-CH₂-OCH₃ | |
| 1.19 | H | O | -CH=N-OCH₂-CH₂-OC₂H₅ | |
| 1.20 | F | O | -CH=N-OCH₂-CH₂-OC₂H₅ | |
| 1.21 | Cl | O | -CH=N-OCH₂-CH₂-OC₂H₅ | |
| 1.22 | H | O | -CH=N-OCH₂-CO-OCH₃ | |
| 1.23 | F | O | -CH=N-OCH₂-CO-OCH₃ | 68- 72°C |
| 1.24 | Cl | O | -CH=N-OCH₂-CO-OCH₃ | 178-182°C |
| 1.25 | H | O | -CH=N-OCH₂-CO-OC₂H₅ | |
| 1.26 | F | O | -CH=N-OCH₂-CO-OC₂H₅ | |
| 1.27 | Cl | O | -CH=N-OCH₂-CO-OC₂H₅ | |
| 1.28 | H | O | -CH=N-OCH(CH₃)-CO-OCH₃ | |
| 1.29 | F | O | -CH=N-OCH(CH₃)-CO-OCH₃ | 85-88°C |
| 1.30 | Cl | O | -CH=N-OCH(CH₃)-CO-OCH₃ | 129-130°C |
| 1.31 | H | O | -CH=N-OCH (CH₃)-CO-OC₂H₅ | |
| 1.32 | F | O | -CH=N-OCH (CH₃)-CO-OC₂H₅ | |
| 1.33 | Cl | O | -CH=N-OCH(CH₃)-CO-OC₂H₅ | |
| 1.34 | H | S | -CH=N-OH | |
| 1.35 | F | S | -CH=N-OH | |
| 1.36 | Cl | S | -CH=N-OH | |
| 1.37 | H | S | -CH=N-OCH₃ | |
| 1.38 | F | S | -CH=N-OCH₃ | |
| 1.39 | Cl | S | -CH=N-OCH₃ | |
| 1.40 | Cl | S | -CH=N-OC₂H₅ | |
| 1.41 | Cl | S | -CH=N-O-n-C₃H₇ | |
| 1.42 | Cl | S | -CH=N-O-i-C₃H₇ | |
| 1.43 | Cl | O | -CH=N-O-CH₂-CO₂H | |
| 1.44 | Cl | S | -CH=N-O-CH₂-CO₂H | |
| 1.45 | H | S | -CH=N-O-CH₂-CO₂CH₃ | |
| 1.46 | Cl | S | -CH=N-O-CH₂-CO₂CH₃ | |
| 1.47 | H | S | -CH=N-O-CH₂-CO₂C₂H₅ | |
| 1.48 | Cl | S | -CH=N-O-CH₂-CO₂C₂H₅ | |
| 1.49 | Cl | O | -CH=N-O-CH₂-CO₂-i-C₃H₇ | |
| 1.50 | Cl | S | -CH=N-O-CH₂-CO₂-i-C₃H₇ | |
| 1.51 | H | O | -CH=N-O-C(CH₃)₂-CO₂CH₃ | |
| 1.52 | F | O | -CH=N-O-C(CH₃)₂-CO₂CH₃ | 70-74°C |
| 1.53 | F | O | -CH=N-O-CH₂-CH₂-Cl | |
| 1.54 | F | O | -CH=N-O-CH₂-CH₂-CH₂-Cl | |
| 1.55 | F | O | -CH=N-O-CHF₂ | |
| 1.56 | F | O | -CH=N-O-CH₂CF₃ | |
| 1.57 | F | O | -CH=N-O-CHF-CHF₂ | |
| 1.58 | F | O | -CH=N-O-CClF-CHF₂ | |
| 1.59 | F | O | -CH=N-O-CH₂-CCl=CH₂ | |
| 1.60 | F | O | -CH=N-O-CH₂-CH=CHCl | |
| 1.61 | F | O | -CH=N-O-CH₂-CCl=CHCl | |
| 1.62 | F | O | -CH=N-O-CH₂-CCl=CCl₂ | |
| 1.63 | F | O | -CH=N-O-CH₂-CH=CCl₂ | |
| 1.64 | F | S | -CH=N-O-CHF₂ | |
| 1.65 | F | S | -CH=N-O-CH₂-CH=CHCl | |
| 1.66 | F | O | -CH=N-O-CH₂-C≡C-Cl | |
| 1.67 | F | O | -CH=N-O-C≡C-CF₃ | |
| 1.68 | F | O | -CH=N-O-CH₂-C≡C-CH₂Cl | |
| 1.69 | F | O | -CH=N-O-C₃H₅ | |
| 1.70 | F | O | -CH=N-O-C₅H₉ | |
| 1.71 | F | O | -CH=N-O-C₆H₁₁ | |
| 1.72 | F | O | -CH=N-O-CH₂CN | |
| 1.73 | F | O | -CH=N-O-CH₂CH₂CN | |
| 1.74 | F | S | -CH=N-O-CH₂CN | |
| 1.75 | F | O | -CH=N-O-CH=CH₂ | |
| 1.76 | F | O | -CH=N-O-CH₂-CH=CH₂ | |
| 1.77 | F | O | -CH=N-O-CH(CH₃)-CH=CH₂ | |
| 1.78 | F | O | -CH=N-O-C(CH₃)₂-CH=CH₂ | |
| 1.79 | F | O | -CH=N-O-CH₂-C≡CH | 158-160°C |
| 1.80 | F | O | -CH=N-O-CH (CH₃)-C≡CH | |
| 1.81 | F | O | -CH=N-O-C(CH₃)₂-C≡CH | |
| 1.82 | F | S | -CH=N-O-CH₂-CH=CH₂ | |
| 1.83 | F | S | -CH=N-O-CH(CH₃)-CH=CH₂ | |
| 1.84 | F | S | -CH=N-O-CH₂-C≡CH | |
| 1.85 | F | S | -CH=N-O-CH-(CH₃)-C≡CH | |
| 1.86 | F | O | -CH=N-O-CH₂-C(=O)CH₃ | |
| 1.87 | F | O | -CH=N-O-CH₂-C(=O)C₂H₅ | |
| 1.88 | F | S | -CH=N-O-CH₂-C(=O)CH₃ | |
| 1.89 | F | O | -CH=N-O-CH₂-O-C(=O)CH₃ | |
| 1.90 | F | O | -CH=N-O-CH₂-O-C(=O)C₂H₅ | |
| 1.91 | F | O | -CH=N-O-CH₂CH₂-O-C(=O)CH₃ | |
| 1.92 | Cl | S | -CH=C(Cl)-CO₂CH₃ | |
| 1.93 | Cl | S | -CH=C(Cl)-CO₂C₂H₅ | |
| 1.94 | H | O | -CH=C(Cl)-CO₂CH₃ | |
| 1.95 | F | O | -CH=C(Cl)-CO₂CH₃ | 158-160°C |
| 1.96 | Cl | O | -CH=C(Cl)-CO₂CH₃ | 174-175°C |
| 1.97 | H | O | -CH=C(Cl)-CO₂C₂H₅ | |
| 1.98 | F | O | -CH=C(Cl)-CO₂C₂H₅ | 153-155°C |
| 1.99 | Cl | O | -CH=C(Cl)-CO₂C₂H₅ | |
| 1.100 | H | O | -CH=C(Cl)-CO-OCH₂CH₂-OCH₃ | |
| 1.101 | F | O | -CH=C(Cl)-CO-OCH₂CH₂-OCH₃ | |
| 1.102 | Cl | O | -CH=C(Cl)-CO-OCH₂CH₂-OCH₃ | |
| 1.103 | H | O | -CH=C(Cl)-CO-OCH₂CH₂-OC₂H₅ | |
| 1.104 | F | O | -CH=C(Cl)-CO-OCH₂CH₂-OC₂H₅ | |
| 1.105 | Cl | O | -CH=C (Cl)-CO-OCH₂CH₂-OC₂H₅ | |
| 1.106 | H | O | -CH=C(CH₃)-CO-OCH₃ | |
| 1.107 | F | O | -CH=C(CH₃)-CO-OCH₃ | |
| 1.108 | Cl | O | -CH=C(CH₃)-CO-OCH₃ | |
| 1.109 | H | O | -CH=C(CH₃)-CO-OC₂H₅ | |
| 1.110 | F | O | -CH=C(CH₃)-CO-OC₂H₅ | |
| 1.111 | Cl | O | -CH=C(CH₃)-CO-OC₂H₅ | |
| 1.112 | H | O | -CH=C (CH₃)-CO-OCH₂CH₂-OCH₃ | |
| 1.113 | F | O | -CH=C (CH₃)-CO-OCH₂CH₂-OCH₃ | |
| 1.114 | Cl | O | -CH=C (CH₃)-CO-OCH₂CH₂-OCH₃ | |
| 1.115 | H | O | -CH=C (CH₃)-CO-OCH₂CH₂-OC₂H₅ | |
| 1.116 | F | O | -CH=C (CH₃)-CO-OCH₂CH₂-OC₂H₅ | |
| 1.117 | Cl | O | -CH=C (CH₃)-CO-OCH₂CH₂-OC₂H₅ | |
| 1.118 | H | O | -CH=C(Br)-CO-OCH₃ | |
| 1.119 | F | O | -CH=C(Br)-CO-OCH₃ | |
| 1.120 | Cl | O | -CH=C(Br)-CO-OCH₃ | |
| 1.121 | H | O | -CH=C(Br)-CO-OCH₂CH₂-OC₂H₅ | |
| 1.122 | F | O | -CH=C(Br)-CO-OCH₂CH₂-OC₂H₅ | |
| 1.123 | Cl | O | -CH=C(Br) -CO-OCH₂CH₂-OC₂H₅ | |
| 1.124 | H | O | -CH=C(Br)-CO-OCH₂CH₂-OCH₃ | |
| 1.125 | F | O | -CH=C(Br)-CO-OCH₂CH₂-OCH₃ | |
| 1.126 | Cl | O | -CH=C(Br)-CO-OCH₂CH₂-OCH₃ | |
| 1.127 | H | O | -CH=C(Br)-CO-OCH₂CH₂-OC₂H₅ | |
| 1.128 | F | O | -CH=C(Br)-CO-OCH₂CH₂-OC₂H₅ | |
| 1.129 | Cl | O | -CH=C(Br)-CO-OCH₂CH₂-OC₂H₅ | |
| 1.130 | H | O | -CH=C(Br)-CO₂CH₃ | |
| 1.131 | H | O | -CH=C(Cl)-CO-SCH₃ | |
| 1.132 | H | O | -CH=C(Cl)-CO-SC₂H₅ | |
| 1.133 | H | O | -CH=C(Br)-CO-SC₂H₅ | |
| 1.134 | Cl | S | -CH=C(Cl)-CO-O-CH (CH₃)-CO₂CH₃ | |
| 1.135 | Cl | O | -CH=C(Cl)-CO-O-CH=CH₂ | |
| 1.136 | Cl | S | -CH=C(Cl)-CO-O-CH=CH₂ | |
| 1.137 | F | O | -CH=C(Cl)-CO-O-CH₂-CH=CH₂ | |
| 1.13 8 | F | O | -CH=C(Br)-CO-O-CH₂-CH=CH₂ | |
| 1.139 | Cl | O | -CH=C(Cl)-CO-O-CH(CH₃)-CH=CH₂ | |
| 1.140 | Cl | O | -CH=C(Br)-CO-O-CH(CH₃)-CH=CH₂ | |
| 1.141 | H | S | -CH=C(Cl)-CO-O-CH₂-CH=CH₂ | |
| 1.142 | F | O | -CH=C(Cl)-CO-O-CH₂C≡CH | |
| 1.143 | F | O | -CH=C(Cl)-CO-OC₃H₅ | |
| 1.144 | F | O | -CH=C(Cl)-CO-OC₄H₇ | |
| 1.145 | F | O | -CH=C(Cl)-CO-OC₅H₉ | |
| 1.146 | F | O | -CH=C(Cl)-CO-OC₆H₁₁ | |
| 1.147 | F | O | -CH=C(Cl)-CO-O-CH₂-SCH₃ | |
| 1.148 | F | O | -CH=C(Cl)-CO-O-CH₂SC₂H₅ | |
| 1.149 | F | O | -CH=C(Cl)-CO-O-CH₂CH₂-SCH₃ | |
| 1.150 | F | O | -CH=C(Br)-CO-O-CH₂CH₂-SCH₃ | |
| 1.151 | F | O | -CH=C(Cl)-CO-O-CH₂CH₂-S(O) CH₃ | |
| 1.152 | F | O | -CH=C(Cl)-CO-O-CH₂CH₂-SO₂CH₃ | |
| 1.153 | F | O | -CH=C(Br)-CO-O-CH₂CH₂-S(O)CH₃ | |
| 1.154 | F | O | -CH=C (Br)-CO-O-CH₂CH₂-SO₂CH₃ | |
| 1.155 | F | O | -CH=C(Cl)-CO-O-CH₂CN | |
| 1.156 | F | O | -CH=C(Cl)-CO-O-CH₂CH₂CN | |
| 1.157 | F | O | -CH=C(CH₃)-CO-O-CH₂CH₂CN | |
| 1.158 | F | O | -CH=C(Cl)-CO-O-CH(CH₃)CN | |
| 1.159 | F | O | -CH=C(Br)-CO-O-CH(CH₃)CH₂CN | |
| 1.160 | F | O | -CH=C(Cl)-CO-O-CH(CH₃)CH₂CN | |
| 1.161 | F | O | -CH=C(Br)-CO-O-CH(CH₃)₂CN | |
| 1.162 | F | O | -CH=C(Br)-CO-O-C(CH₃)CH₂CN | |
| 1.163 | F | O | -CH=C(Cl)-CO-O-C(CH₃)₂CH₂CN | |
| 1.164 | F | O | -CH=C(Br)-CO-O-CH₂-COOCH₃ | |
| 1.165 | F | O | -CH=C(Cl)-CO-O-CH₂-COOCH₃ | 118-120°C |
| 1.166 | F | S | -CH=C(CH₃)-CO-O-CH₂-COOCH₃ | |
| 1.167 | F | O | -CH=C (Br)-CO-O-CH(CH₃)COOCH₃ | |
| 1.168 | F | O | -CH=C(Cl)-CO-O-CH(CH₃)-COOCH₃ | 108-110°C |
| 1.169 | F | O | -CH=C(Cl)-CO-O-C(CH₃)₂-COOCH₃ | |
| 1.170 | F | O | -CH-C(Br)-CO-O-CH₂-C≡CH | |
| 1.171 | F | O | -CH=C(Cl)-CO-O-CH₂CH₂Cl | |
| 1.172 | F | O | -CH=C(Cl)-CO-O-CH₂CF₃ | |
| 1.173 | F | O | -CH=C(Cl)-CO-O-CH(CH₃)CH₂Cl | |
| 1.174 | F | S | -CH=C(Cl)-CO-O-CH₂CH₂Cl | |
| 1.175 | F | O | -CH=C(Br)-CO-O-CH₂CH₂Cl | |
| 1.176 | F | O | -CH=C(CH₃)-CO-O-CH₂CF₃ | |
| 1.177 | F | O | -CH=C(Cl)-CO-O-CH(CH₃)CH₂Cl | |
| 1.178 | F | O | -CH=C(Cl)-CO-O-CF₂CF₃ | |
| 1.179 | F | O | -CH=C(Cl)-CO-O-CFCl-CHF₂ | |
| 1.180 | F | O | -CH=C(Cl)-CO-O-CHF-CHF₂ | |
| 1.181 | F | O | -CH=C (Cl) -CO-O-CH₂-CH=CHCl | |
| 1.182 | F | O | -CH=C(Cl)-CO-O-CH₂-C(Cl)=CH₂ | |
| 1.183 | F | O | -CH=C(Cl)-CO-O-CH₂-CH=CCl₂ | |
| 1.184 | F | O | -CH=C(Cl)-COO-CH₂-C(Cl)=CHCl | |
| 1.185 | F | O | -CH=C(Cl)-CO-O-CH₂-C≡C-Cl | |
| 1.186 | F | O | -CH=C(Cl)-CO-O-C≡C-CF₃ | |
| 1.187 | F | O | -CH=C(Cl)-CO-O-CH₂-C≡C-CH₂Cl | |
| 1.188 | F | O | -CH=C(Cl)-CO-O-CH₂-C≡C-CF₃ | |
| 1.189 | F | O | -CH=C(Cl)-COOCHCl-C≡C-CH₂Cl | |
| 1.190 | F | O | -CH=C(Br)-CO-O-CH₂-C(Cl)=CH₂ | |
| 1.191 | F | O | -CH=C(Hr)-COO-CH₂-C(Cl)=CHCl | |
| 1.192 | F | O | -CH=C (Cl)-CO-O-CH₂-CH(OCH₃)₂ | |
| 1.193 | F | O | -CH=C (Br)-CO-O-CH₂-CH (OCH₃)₂ | |
| 1.194 | F | O | -CH=C (CH₃)-CO-O-CH₂-CH(OCH₃)₂ | |
| 1.195 | F | O | -CH=C(Cl)COOCH(CH₃)-CH(OCH₃)₂ | |
| 1.196 | F | O | -CH=C(Cl)-COO-CH₂CH₂CH(OCH₃)₂ | |
| 1.197 | F | O | -CH₂-CH₂-CO-OCH₃ | |
| 1.198 | F | O | -CH₂-CH₂-CO-O-C₂H₅ | |
| 1.199 | F | O | -CH₂-CH₂-CO-O-n-C₃H₇ | |
| 1.200 | F | O | -CH₂-CH₂-CO-O-n-C₄H₉ | |
| 1.201 | F | O | -CH₂-CH₂-CO-O-n-C₅H₁₁ | |
| 1.202 | F | O | -CH₂-CH(CH₃)-CO-OCH₃ | |
| 1.203 | F | O | -CH₂-CH(CH₃)-CO-OC₂H₅ | |
| 1.204 | F | O | -CH₂-CH(Cl)-CO-OC₂H₅ | |
| 1.205 | F | O | -CH₂-CH(CN)-CO-OC₂H₅ | |
| 1.206 | F | O | -CH₂-CH(Cl)-CO-O-n-C₃H₇ | |
| 1.207 | F | O | -C(CH₃)=N-OH | |
| 1.208 | F | O | -C(CH₃)=N-OCH₃ | |
| 1.209 | F | O | -C(CH₃)=N-OC₂H₅ | |
| 1.210 | F | O | -C(CH₃)=N-O-n-C₃H₇ | |
| 1.211 | F | O | -C(CH₃)=N-O-CH₂COOCH₃ | |
| 1.212 | F | O | -C(CH₃)=N-O-CH₂COOC₂H₅ | |
| 1.213 | F | O | -C(CH₃)=N-O-CH(CH₃)COOCH₃ | |
| 1.214 | F | O | -C(CH₃)=N-O-CH(CH₃)COOC₂H₅ | |
| 1.215 | F | O | -C(C₂H₅)=N-OCH₃ | |
| 1.216 | F | O | -C(C₂H₅)=N-OC₂H₅ | |
| 1.217 | F | O | -C(CH₃)=N-O-C(CH₃)₂COCH₃ | |
| 1.218 | F | O | -CO-O-CH₂-CO₂H | |
| 1.219 | F | O | -CO-O-CH₂-CO₂CH₃ | |
| 1.220 | F | O | -CO-O-CH₂-CO₂-C₂H₅ | |
| 1.221 | F | O | -CO-O-CH₂-CO₂-n-C₃H₇ | |
| 1.222 | F | O | -CO-O-CH₂-CO₂-n-C₄H₉ | |
| 1.223 | F | O | -CO-O-CH(CH₃)CO₂CH₃ | |
| 1.224 | F | O | -CO-O-CH(CH₃)CO₂C₂H₅ | |
| 1.225 | H | O | -CO-O-CH₂CO₂CH₃ | |
| 1.226 | H | O | -CO-O-CH₂CO₂C₂H₅ | |
| 1.227 | H | O | -CO-O-CH(CH₃)CO₂CH₃ | |
| 1.22 8 | H | S | -CO-O-CH₂CO₂CH₃ | |
| 1.229 | H | O | -CO-O-CH₂CO₂CH₂-CH=CH₂ | |
| 1.230 | F | O | -CO-O-CH₂CO₂CH₂-CH=CH₂ | |
| 1.231 | F | O | -CO-O-CH₂CO₂CH₂-C≡CH | |
| 1.232 | F | O | -CO-O-CH₂CO₂CH₂CH₂-OCH₃ | |
| 1.233 | F | O | -CO-O-CH₂CO₂CH₂CH₂-OC₂H₅ | |
| 1.234 | F | O | -CO-O-CH₂CO₂CH₂CH₂Cl | |
| 1.235 | F | O | -CO-O-CH₂CO₂CH₂CH₂CN | |
| 1.236 | F | O | -CO-O-CH(CH₃)CO₂CH₂-CH=CH₂ | |
| 1.237 | F | O | -CO-O-CH(CH₃)CO₂CH₂-CH≡CH | |
| 1.238 | F | O | -CO-O-CH(CH₃)CO₂CH₂CH₂-OCH₃ | |
| 1.239 | F | O | -CO-O-C(CH₃)₂CO₂CH₃ | |
| 1.240 | F | S | -CO-OCH₂CO₂CH₂-CH=CH₂ | |
| 1.241 | F | O | -CO-NH-OCH₃ | 172-174°C |
| 1.242 | F | O | -CO-NH-OC₂H₅ | 163-165°C |
| 1.243 | F | O | -CO-NH-O-n-C₃H₇ | |
| 1.244 | F | O | -CO-NH-O-n-C₄H₉ | |
| 1.245 | F | O | -CO-N-(CH₃)-OCH₃ | 151-153°C |
| 1.246 | F | O | -CO-N(CH₃)-OC₂H₅ | 161-163°C |
| 1.247 | F | O | -CO-N(CH₃)-O-n-C₃H₇ | |
| 1.248 | F | O | -CO-N(C₂H₅)-OCH₃ | |
| 1.249 | F | O | -CO-N(C₂H₅)-OC₂H₅ | |
| 1.250 | F | O | -CO-N(CH₃)-O-CH₂CO₂CH₃ | |
| 1.251 | F | O | -CO-N(CH₃)-O-CH(CH₃)CO₂CH₃ | |
| 1.252 | F | O | -CO-N(CH₃)-O-CH₂CO₂C₂H₅ | |
| 1.253 | F | O | -CO-N(CH₃)-O-CH₂-CH=CH₂ | |
| 1.254 | F | O | -CO-N(CH₃)-O-CH₂-C≡CH | |
| 1.255 | F | O | -CO-N(CH₃)-O-CH₂-CH₂Cl | |
| 1.256 | F | O | -CO-N(CH₃)-O-CH₂-C(Cl)=CH₂ | |
| 1.257 | F | O | -CO-N(CH₃)-O-CH₂-CH=CHCl | |
| 1.258 | F | O | -CO-N(CH₃)-O-CH₂-C≡C-CH₂Cl | |
| 1.259 | F | O | -CO-N(CH₃)-O-CH₂CN | |
| 1.260 | F | O | -CO-N (CH₃)-O-CH₂CH₂CN | |
| 1.261 | F | O | -CO-N(CH₃)-O-CH₂-CO₂H | |
| 1.262 | F | O | -CO-N(CH₃)-O-CH(CH₃)-CO₂H | |
| 1.263 | F | O | -CO-N(CH₃)-O-CH₂-C(O)-CH₃ | |
| 1.264 | F | O | -CO-N(CH₃)-O-CH₂-C(O)C₂H₅ | |
| 1.265 | F | O | -CO-N(CH₃)-O-CH₂CH₂OC(O)CH₃ | |
| 1.266 | F | O | -CO-N(CH₃)-O-CH₂CH₂-OCH₃ | |
| 1.267 | F | O | -CO-N(C₂H₅)-O-CH₂CH₂-OCH₃ | |
| 1.268 | F | O | -CO-N(C₂H₅)-O-CH₂C(O)CH₃ | |
| 1.269 | F | O | -CO-N(C₂H₅)-O-CH₂CO₂C₂H₅ | |
| 1.270 | F | O | -CO-N(C₂H₅)-O-CH₂-CH=CHCl | |
| 1.271 | F | O | -CO-N(C₂H₅)-O-CH₂CH₂Cl | |
| 1.272 | F | O | -CO-N(C₂H₅)-O-CH₂CH₂CN | |
| 1.273 | F | O | -CO-N(CH₂-CH=CH₂)-OCH₃ | |
| 1.274 | F | O | -CO-N(CH₂-CH=CH₂)-OC₂H₅ | |
| 1.275 | F | O | -CO-N(CH₂-C≡CH)-OCH₃ | |
| 1.276 | F | O | -CO-N(CH₂CH₂Cl)-OCH₃ | |
| 1.277 | F | O | -CO-N(CH₂C(O)OCH₃)OCH₃ | |
| 1.278 | F | O | -CO-N(CH₂C(O)OC₂H₅)OCH₃ | |
| 1.279 | F | O | -CO-N(CH₂CH₂OCH₃)OCH₃ | |
| 1.280 | F | O | -CO-N(C₃H₅)OCH₃ | |
| 1.281 | F | O | -CO-N(CH₂CH₂CN)-O-CH₃ | |
| 1.282 | F | O | -CO-N(CH₂-CH=CHCl)-OCH₃ | |
| 1.283 | F | O | -CO-N(CH₂-C≡C-CH₂Cl)-OCH₃ | |
| 1.284 | F | O | -CO-N(CH₂-CH=CH₂)-OCH₂CO₂CH₃ | |
| 1.285 | F | O | -CO-N(CH₂-C≡CH)-OCH₂CO₂CH₃ | |
| 1.286 | F | O | -C(OCH₃)=N-OCH₃ | 100-105°C |
| 1.287 | F | O | -C(OCH₃)=N-OC₂H₅ | 180-181°C |
| 1.288 | F | O | -C(OCH₃)=N-O-n-C₃H₇ | |
| 1.289 | F | O | -C(OCH₃)=N-O-CH₂CO₂CH₃ | |
| 1.290 | F | O | -C(OCH₃)=N-O-CH(CH₃)CO₂CH₃ | |
| 1.291 | F | O | -C(OCH₃)=N-O-CH₂CO₂C₂H₅ | |
| 1.292 | F | O | -C(OCH₃)=N-O-CH₂-CH=CH₂ | |
| 1.293 | F | O | -C(OCH₃)=N-O-CH₂-C≡CH | |
| 1.294 | F | O | -C(OCH₃)=N-O-CH₂CH₂Cl | |
| 1.295 | F | O | -C(OCH₃)=N-O-CH₂-C(Cl)=CH₂ | |
| 1.296 | F | O | -C(OCH₃)=N-O-CH₂-CH=CHCl | |
| 1.297 | F | O | -C(OCH₃)=N-O-CH₂-C≡C-CH₂Cl | |
| 1.298 | F | O | -C(OCH₃)=N-O-CH₂CN | |
| 1.299 | F | O | -C(OCH₃)=N-OCH₂CH₂CN | |
| 1.300 | H | O | -C(OCH₃)=N-O-CH₂CO₂CH₃ | |
| 1.301 | F | S | -C(OCH₃)=N-O-CH(CH₃)-CO₂CH₃ | |
| 1.302 | F | O | -C(OCH₃)=N-O-CH₂-C(O)CH₃ | |
| 1.303 | F | O | -C(OCH₃)=N-O-CH₂-C(O)C₂H₅ | |
| 1.304 | F | O | -C(OCH₃)=N-O-CH₂CH₂OC(O)CH₃ | |
| 1.305 | F | O | -C(OCH₃)=N-O-CH₂CH₂-OCH₃ | |
| 1.306 | F | O | -C(OC₂H₅)=N-O-CH₂CH₂-OCH₃ | |
| 1.307 | F | O | -C(OC₂H₅)=N-O-CH₂C(O)CH₃ | |
| 1.308 | F | O | -C(OC₂H₅)=N-O-CH₂CO₂C₂H₅ | |
| 1.309 | F | O | -C(OC₂H₅)=N-O-CH₂CH=CHCl | |
| 1.310 | F | O | -C(OC₂H₅)=N-O-CH₂CH₂Cl | |
| 1.311 | F | O | -C(OC₂H₅)=N-OCH₂CH₂CN | |
| 1.312 | F | O | -C(OCH₂-CH=CH₂)=N-OCH₃ | |
| 1.313 | F | O | -C(OCH₂-CH=CH₂)=N-OC₂H₅ | |
| 1.314 | F | O | -C(OCH₂-C≡CH)=N-OCH₃ | |
| 1.315 | F | O | -C(OCH₂CH₂Cl)=N-OCH₃ | |
| 1.316 | F | O | -C(OCH₂C(O)OCH₃)=N-OCH₃ | 65-70°C |
| 1.317 | F | O | -C(OCH₂C(O)OC₂H₅)=N-OCH₃ | |
| 1.318 | F | O | -C(OCH₂CH₂OCH₃)=N-OCH₃ | |
| 1.319 | F | O | -C(OC₃H₅)=NOCH₃ | |
| 1.320 | Cl | O | -CH=N-O-CH₂CH₂Cl | |
| 1.321 | Cl | O | -CH=N-O-CH₂-CH=CHCl | |
| 1.322 | Cl | O | -CH=N-O-CH₂CCl=CHCl | |
| 1.323 | Cl | O | -CH=N-O-CH₂CCl=CCl₂ | |
| 1.324 | Cl | O | -CH=N-O-CH₂-C≡C-CH₂Cl | |
| 1.325 | Cl | O | -CH=N-O-C₃H₅ | |
| 1.326 | Cl | O | -CH=N-O-C₅H₉ | |
| 1.327 | Cl | O | -CH=N-O-CH₂CH₂CN | |
| 1.328 | Cl | O | -CH=N-O-CH₂CH=CH₂ | |
| 1.329 | Cl | O | -CH=N-O-CH₂-C≡CH | |
| 1.330 | Cl | S | -CH=N-O-CH₂-C≡CH | |
| 1.331 | Cl | O | -CH=N-O-CH₂-C(O)CH₃ | |
| 1.332 | Cl | O | -CH=C(Br)-CO-O-CH₂-CH=CH₂ | |
| 1.333 | Cl | O | -CH=C(Cl)-CO-O-CH₂-C≡CH | |
| 1.334 | Cl | O | -CH=C(Cl)-CO-O-C₃H₅ | |
| 1.335 | Cl | O | -CH=C(Cl) -CO-O-C₅H₉ | |
| 1.336 | Cl | O | -CH=C(Br)-CO-O-CH₂CH₂SCH₃ | |
| 1.337 | Cl | O | -CH=C(Cl)-O-CH₂CN | |
| 1.338 | Cl | O | -CH=C(Cl)-O-CH₂CH₂CN | |
| 1.339 | Cl | O | -CH=C(Br)-O-CH₂CO₂CH₃ | |
| 1.340 | Cl | O | -CH=C(Cl)-CO-O-CH(CH₃)-CO₂CH₃ | Ph=8,15(s/1H), 8,03(s/1H); CH=7, 7 (s/1H); CH=5,25-5,3 (q/1H) |
| 1.341 | Cl | O | -CH=C(Cl)-CO-O-CH₂CH₂Cl | |
| 1.342 | Cl | O | -CH=C(Cl)-CO-O-CH₂-CH=CHCl | |
| 1.343 | Cl | O | -CH=C(Cl)-CO-O-CH₂CH(OCH₃)₂ | |
| 1.344 | Cl | O | -CH₂-CH₂-CO₂CH₃ | |
| 1.345 | Cl | O | -CH₂-CH₂-CO₂C₂H₅ | |
| 1.346 | Cl | O | -C(CH₃)=N-OCH₃ | |
| 1.347 | Cl | O | -C(CH₃)=N-O-CH₂CO₂CH₃ | |
| 1.348 | Cl | O | -CO-O-CH₂CO₂CH₃ | |
| 1.349 | Cl | O | -CO-O-CH₂CO₂C₂H₅ | |
| 1.350 | H | O | -CO-O-CH(CH₃)-CO₂C₂H₅ | |
| 1.351 | Cl | O | -CO-O-CH(CH₃)-CO₂CH₃ | |
| 1.352 | Cl | O | -CO-O-CH₂CO₂CH₂CH=CH₂ | |
| 1.353 | Cl | O | -CO-O-CH₂CO₂CH₂CH₂-OC₂H₅ | |
| 1.354 | Cl | O | -CO-O-C(CH₃)₂CO₂CH₃ | |
| 1.355 | F | O | -CO-O-C(CH₃)₂CO₂CH₃ | |
| 1.356 | Cl | O | -CO-O-C(CH₃)₂CO₂CH₂CH=CH₂ | |
| 1.357 | Cl | O | -CO-NH-OCH₃ | |
| 1.358 | Cl | O | -CO-NH-OC₂H₅ | |
| 1.359 | Cl | O | -CO-N(CH₃)-OCH₃ | |
| 1.360 | Cl | O | -CO-N(CH₃)-OC₂H₅ | |
| 1.361 | Cl | O | -CO-N(C₂H₅)-OCH₃ | |
| 1.362 | Cl | O | -CO-N(CH₃)-OCH₂-CH=CH₂ | |
| 1.363 | Cl | O | -CO-N(CH₃)-O-CH₂-CCH | |
| 1.364 | Cl | O | -CO-N(CH₃)-OCH₂CH₂CN | |
| 1.365 | Cl | O | -CO-N(CH₃)-O-CH₂CO₂CH₃ | |
| 1.366 | Cl | O | -CO-N(CH₃)-O-CH₂C(O)CH₃ | |
| 1.367 | Cl | O | -CO-N(CH₃)-O-CH₂CH₂-OCH₃ | |
| 1.368 | Cl | O | -CO-N(C₂H₅)-O-CH₂CH₂-OCH₃ | |
| 1.369 | Cl | O | -CO-N(C₂H₅)-O-CH₂CO₂CH₃ | |
| 1.370 | Cl | O | -CO-N(CH₂-CH=CH₂)-OCH₃ | |
| 1.371 | Cl | O | -CO-N(CH₂CH₂CN)-OCH₃ | |
| 1.372 | Cl | O | -C(OCH₃)=N-OCH₃ | |
| 1.373 | Cl | O | -C(OCH₃)=N-OC₂H₅ | |
| 1.374 | Cl | O | -C(OCH₃)=N-O-CH₂CO₂CH₃ | |
| 1.375 | Cl | O | -C(OCH₃)=N-O-CH₂CH=CH₂ | |
| 1.376 | Cl | O | -C(OCH₃)=N-O-CH₂CH₂Cl | |
| 1.377 | Cl | O | -C(OCH₃)=N-O-CH₂CH₂CN | |
| 1.378 | Cl | O | -C(OCH₃)=N-O-CH₂CO₂CH₃ | |
| 1.379 | Cl | O | -C(OCH₃)=N-O-C(CH₃)₂CO₂CH₃ | |
| 1.380 | Cl | O | -C(OCH₃)=N-O-CH₂CH₂OCH₃ | |
| 1.381 | Cl | O | -C(OC₂H₅)=N-O-CH₂CH₂Cl | |
| 1.382 | Cl | O | -C(OCH₂CH=CH₂)=N-OCH₃ | |
| 1.383 | Cl | O | -C(OCH₂-C≡CH)=N-OCH₃ | |
| 1.384 | F | O | -CH=C(Cl)-CO-O-tert.C₄H₉ | 138-139°C |
| 1.385 | F | O | -CH=C(Cl)-CO-OH | 252-253°C |
| 1.386 | Cl | O | -CH=C(Cl)-CO-O-tert.C₄H₉ | 83-86°C |
| 1.387 | Cl | O | -CH=C(Cl)-CO-OH | 215-220°C |
| 1.388 | Cl | O | -CH=N-O-C(CH₃)₂CO-OCH₃ | |
| 1.389 | F | O | -CH=N-O-CH(CH₃)-CO-OCH₃ | 85-88°C |
| 1.390 | F | O | -CH=C(Cl)-CO-NH-OCH₃ | 130°C Zers. |
| 1.391 | F | O | -CH=C(Cl)-CO-NH-OC₂H₅ | 105-108°C |
| 1.392 | F | O | -CH=CH-CO-OCH₃ | 198-200°C |
| 1.393 | H | O | CO-NH-OCH₃ | 169-170°C |
| 1.394 | H | O | CO-NH-OC₂H₅ | 163-167°C |
| 1.395 | H | O | CO-N(CH₃)-OC₂H₅ | 71-76°C |
| 1.396 | H | O | C(OCH₃)=N-OC₂H₅ | 90-95°C |

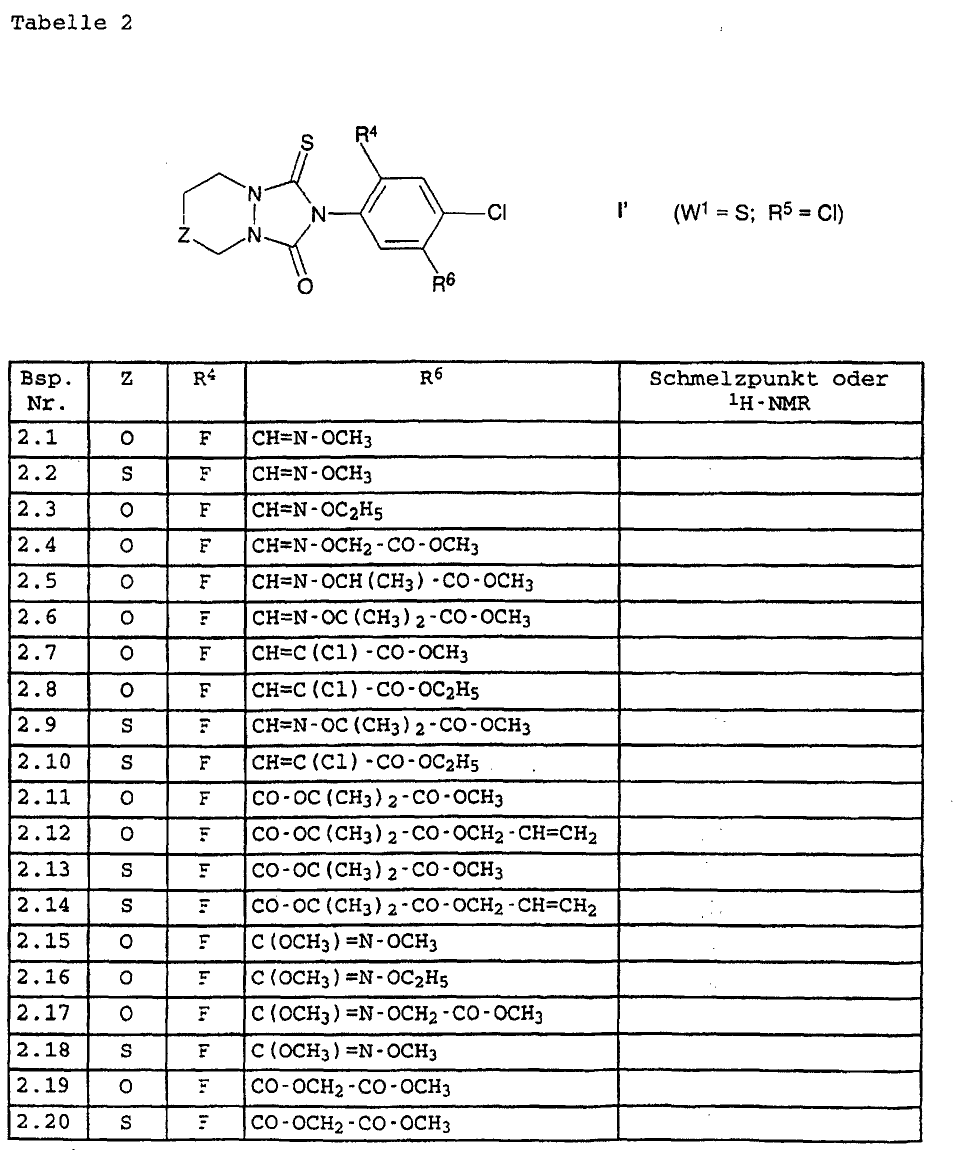

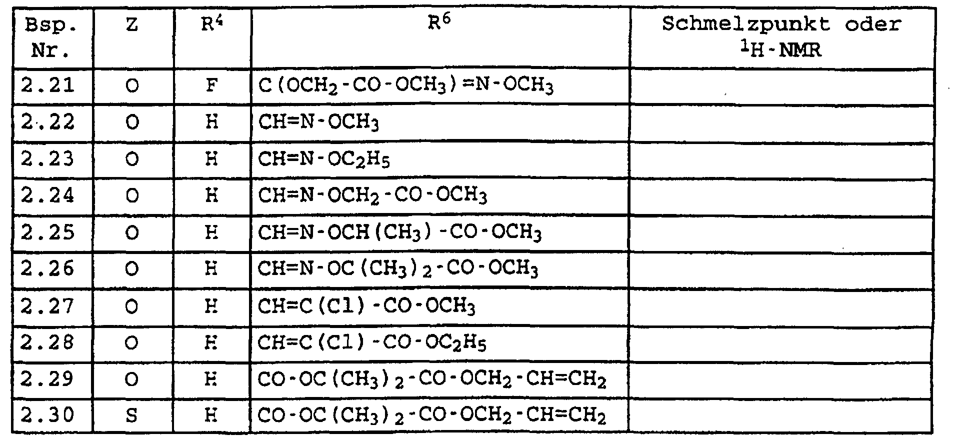

Schließlich sind auch die anellierten Triazole der Formel I mit X-X = C-O oder C-S, V = C=W², W¹ und W² = Sauerstoff, m = Null, Q = Q⁵ mit Y = Sauerstoff, R⁷,R⁸ = Wasserstoff und R⁹ = C₁-C₆-Alkoxy (= Wirkstoffe der Formel I'' mit Z = Sauerstoff oder Schwefel) bevorzugt, also z.B. die Verbindungen der Tabelle 3:

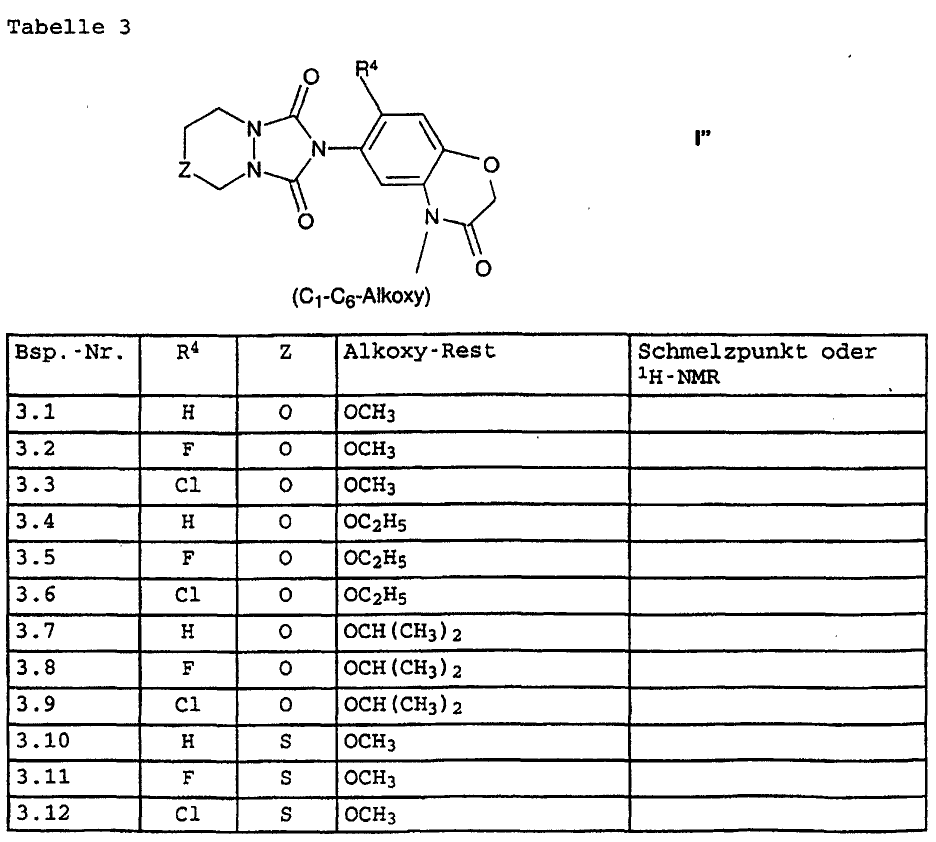

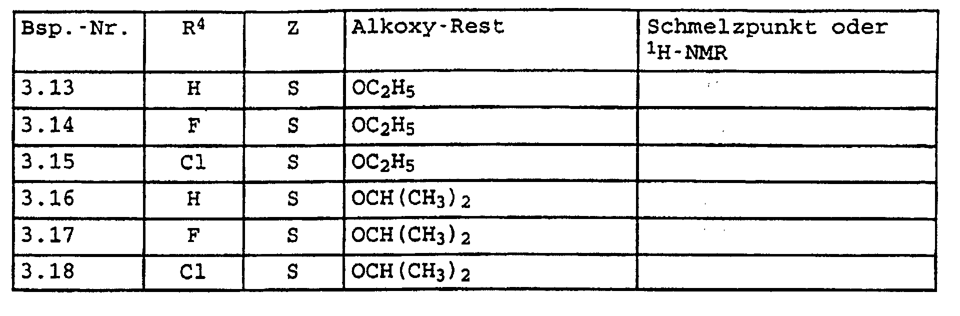

Die Verbindungen I' und deren landwirtschaftlich brauchbaren Salze eignen sich - sowohl als Isomerengemische als auch in Form der reinen Isomeren - als Herbizide. Die I' enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I' bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I' auch in Kulturen, die durch Züchtung einschließlich gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I' bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen im wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z.B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die anellierten Triazole als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe. Die Konzentrationen der Wirkstoffe I' in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die erfindungsgemäßen Verbindungen I' können beispielsweise wie folgt formuliert werden:
I 20 Gewichtsteile der Verbindung Nr. 1.05 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
II 20 Gewichtsteile der Verbindung Nr. 1.23 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
III 20 Gewichtsteile des Wirkstoffs Nr. 1.79 warden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. -% des Wirkstoffs enthält.
IV 20 Gewichtsteile des Wirkstoffs Nr. 1.23 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20 000 Gewichtsteilen Wasser enthält man eine Spritzbrühe, die 0,1 Gew. % des Wirkstoffs enthält.
V 3 Gewichtsteile des Wirkstoffs Nr. 1.05 werden mit 97 Gewichtsteilen feinteiligem Kaolin vermischt. Man erhält auf diese Weise ein Stäubemittel, das 3 Gew. % des Wirkstoffs enthält.
VI 20 Gewichtsteile des Wirkstotts Nr. 1.95 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII 1 Gewichtsteil der Verbindung 1.98 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII 1 Gewichtsteil der Verbindung 1.02 wird in einer Mischung gelöst, die aus 80 Gewichtsteilen Cyclohexanon und 20 Gewichtsteilen Wettol® EM 31 (nicht ionischer Emulgator auf der Basis von ethoxyliertem Ricinusöl). Man erhält ein stabiles Emulsionskonzentrat.

Zur Verbreiterung des Wirkungsspektrums und zur Erzielung synergistischer Effekte können die anellierten Triazole mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF3-phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I' allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.)

### Beispiel 1

### a) N-Amino-N-2-hydroxyethyl-N'-(4-chlor-2-fluor-5-methoxyiminomethyl-phenyl)harnstoff

63,8 g (0,63 mol) Triethylamin wurden bei 0 - 5°C unter Rühren zu einer Lösung von 47,9 g (0,63 mol) Hydrazinoethanol in 300 ml 1,2-Dichlorethan gegeben und 30 min. nachgerührt. Nun wurden bei gleicher Temperatur 0,63 mol (ber. 100 %) 75 % reines 4-Chlor-2-fluor-5-methoxyimino-methyl-phenyl-isocyanat suspendiert in 1,2-Dichlorethan innerhalb 1 Std. zugegeben und 12 Std. nachgerührt. Der entstandene Niederschlag wurde abgesaugt und getrocknet, wobei man als Nebenprodukt 20,6 g (14,7 % d. Th.) des entsprechenden subst. Diphenylharnstoffes mit Fp. > 260°C erhielt.

In dem Filtrat verblieben nach dem Einengen 211,6 g (88,2 % d. Th.) der rohen Titelverbindung als gelbliches Harz.
H-NMR (in (CD₃)₂SO); δ[ppm] = 3,95 (s/3) CH₃O; 4,9 (s/2) NH₂; 7,5 (d/1) Ph; 8,3 (s/1) CHO; 8,7 (d/1) Ph; 9,1 (s/1) NH.

### b) N-Methylenimino-N-2-hydroxyethyl-N'-(4-chlor-2-fluor-5-methoxyiminomethyl-phenyl)harnstoff

27,6 g (0,34 mol) 37 %ige Formaldehydlösung wurden innerhalb 15 min. unter Rühren bei 22 - 25°C zu einer Lösung von 90,2 g (0,296 mol) der in Schritt a) erhaltenen Verbindung in 700 ml Methylenchlorid gegeben und 14 Std. bei 23°C gerührt. Die wässrige Phase wurde abgetrennt und die organische Phase über Aktivkohle und Magnesiumsulfat zum Trocknen gerührt. Nach dem Absaugen über Kieselgel und Einengen erhielt man 83,9 g (89,6 % d. Th.) der Titelverbindung vom Fp. 138-139°C.

### c) Tetrahydro-N-(4-chlor-2-fluor-5-methoxyimino-methyl-phenyl) 4H-1,3,4-oxdiazin-4-carboxamid

22,5 g (0,072 mol) der in Stufe b) erhaltenen Titelverbindung wurden in 350 ml Eisessig 5 Std. bei 70°C gerührt. Die erhaltene klare Reaktionslösung wurde eingeengt, der Rückstand in Methylenchlorid gelöst, mit Natriumhydrogencarbonatlösung gewaschen und getrocknet. Nach dem Absaugen über Kieselgel, Einengen und Verrühren mit Diisopropylether erhielt man nach dem Absaugen und Trocknen 9,7 g (42,6 % d. Th.) der Titelverbindung vom Fp. 169-171°C.

### d) 8-(4'-Chlor-2'-fluor-5'-methoxyiminomethyl-phenyl)4-oxa-7,9-dioxo-1,2,8-triaza[4.3.0.]nonan (Verbindung 1.05 aus Tabelle 1)

15 g (0,0475 mol) der in Stufe c) hergestellten Verbindung wurden analog Beispiel 4c mit 9,4 g (0,0474 mol) Diphosgen in 200 ml Pyridin in die Titelverbindung überführt. Man erhielt hiervon 14,1 g (86,9 % d. Th.) mit dem Fp. 173-175°C.

### Beispiel 2 (Vergleich)

0,54 g (0,018 mol) 37 %ige Formaldehydlösung wurde unter Rühren bei 22°C zu einer Lösung von 5 g (0,0164 mol) der in Beispiel la) erhaltenen Verbindung und 0,5 g p-Toluolsulfonsäure in 250 ml Dichlormethan gegeben und unter Rückfluß 10 Std. gerührt. Das Reaktionsgemisch wurden eingeengt, der Rückstand in Methylenchlorid gelöst, mit Aktivkohle gereinigt und über Kieselgel gesaugt. Man erhielt 2 Fraktionen - 0,3 g als Harz, das nach dem NMR-Spektrum überwiegend aus aliphatischen Verunreinigungen bestand, und 3,1 g als gelbliche Kristalle vom Fp. 105-110°C - die nach der dünnschichtchromatographischen Untersuchung aus 11 Komponenten bestanden. Nach der HPLC-Untersuchung waren höchstens 0,673 g der Verbindung Ic (≙ einer Ausbeute von 12,9 %) entstanden. (Das entsprechende NMR-Spektrum war so uneinheitlich, daß es nicht ausgewertet werden konnte.) Dies zeigt, daß bei der Mitverwendung von Säure in der ersten Stufe die gewünschten Zwischenprodukte nicht in der erforderlichen Reinheit und Ausbeute erhalten werden.

### Beispiel 3

### a) N-Amino-N-2-hydroxyethyl-N'-(4-chlor-2-fluor-5-methoxyphenyl)harnstoff wurde ausgehend von 121 g (0,569 mol) 4-Chlor-2-fluor-5-methoxy-phenylisocyanat analog Beispiel 1a erhalten. Man isolierte 94,5 g (59,7 % d. Th.) der Titelverbindung vom Fp. 125-128°C.

### b) N-Methylenimino-N-2-hydroxyethyl-N'-(4-chlor-2-fluor-5-methoxy-phenyl)harnstoff

27,8 g (0,1 mol) der in Schritt a) erhaltenen Verbindung und 9 g (0,11 mol) 37 %ige wässrige Formaldehydlösung wurden in 500 ml Methylenchlorid vorgelegt und 2 Std. bei 42°C gerührt. Die Reaktionsmischung wurde über Magnesiumsulfat getrocknet und über Kieselgel abgesaugt. Nach dem Einengen erhielt man 25,6 g (84 % d. Th.) der Titelverbindung von Fp. 141-142°C.

### c1) Tetrahydro-N-(4-chlor-2-fluor-5-methoxy-phenyl)-4H-1,3,4-oxdiazin-4-carboxamid

19,2 g (0,0663 mol) des in Stufe b) hergestellten Produkts wurden in 250 ml Essigsäure 2 Std. bei 60°C gerührt. Das Reaktionsgemisch wurde im Vakuum eingeengt und der Rückstand in Diisopropylether/Pentan (2 : 1) verrührt. Nach dem Absaugen und Trocknen erhielt man 11,5 g (59,9 % d. Th.) der Titelverbindung vom Fp. 147-150°C. Aus der Mutterlauge wurden durch Einengen und Behandeln wie oben weitere 1,8 g (9,4 % d. Th.) vom Fp. 145-147°C erhalten.

### c2) Tetrahydro-N-(2,4-dichlor-5-methoxy-phenyl)-4H-1,3,4-oxdiazin-4-carboxamid (Alternative zu c1)

Eine Mischung von 29,4 g (0,1 mol) N-Amino-N-2-hydroxyethyl) -N'-(2,4-dichlor-5-methoxy-phenyl)harnstoff und 9 g (0,11 mol) 37 %ige Formaldehydlösung in 500 ml Methylenchlorid wurde 2 Std. bei 42°C gerührt. Nach dem Abkühlen wurde das Reaktionsgemisch über eine Nutsche mit ca. 300 ml Kieselgel gesaugt und mit Methylenchlorid gewaschen. Hierbei ergab sich in der dünnschichtchromatographischen Untersuchung ein höherer Laufwertgegenüber der nicht mit Kieselgel behandelten, auf der Methyleniminostufe verbliebenen Reaktionsmischung. Nach dem Einengen im Vakuum erhielt man 27,8 g (81,8 % d. Th.) der Titelverbindung von Fp. 128-130°C.

### Beispiel 4

### a) N-Amino-N-2-hydroxyethyl-N'-(2,4-dichlor-5-methoxyiminomethyl-phenyl)harnstoff

wurde ausgehend von 166 g (0,6776 mol) 2,4-Dichlor-5-methoxyiminomethyl-phenylisocyanat analog Beispiel 1a erhalten. Man isolierte nach dem Einengen und Behandeln mit Methylenchlorid als leichter lösliche Fraktion 74,6 g (34 % d. Th.) der Titelverbindung vom Fp. 152-156°C. Als schwerer lösliche Fraktion blieben 75 g vom Fp. 168-200°C zurück, die nach dem NMR-Spektrum weitere 45 g (20 % d. Th.) der Titelverbindung enthielten. Die schwerer lösliche Fraktion enthielt nach dem NMR-Spektrum zusätzlich das N'-2-Hydroxyethyl-N-(2,4-dichlor-5-methoxyiminomethyl-phenylcarbamoyl)hydrazid als Nebenkomponente.

### b) Tetrahydro-N-(2,4-dichlor-5-methoxyimino-methyl-phenyl) 4H-1,3,4-oxdiazin-4-carboxamid

Eine Mischung von 53,5 g (0,1666 mol) der in Stufe a) erhaltenen Verbindung and 14,9 g 37 %ige Formaldehydlösung in 1 1 Methylenchlorid wurde 3 Std. bei 42°C gerührt. Eine Probe ergab beim Ausfällen mit Pentan, Absaugen, Waschen und Trocknen die entsprechende Methylenimino-Zwischenstufe vom Fp. 156-157°C.
¹H-NMR (in (CD₃)₂SO): δ[ppm] = 9,3 (1/s) NH; 8,4 (1/s) =C-H; 7,8 (1/s) und 8,74 (1/s) Ar; 6,6 (1/d) und 7,05 (1/d) N=CH₂; 4,8 (1/t) OH, 3,98 (3/s) CH₃O; 3,5 (m/2) und 3,95 (m/2) CH₂-CH₂.

Das Reaktionsgemisch wurde nun mit 200 ml Essigsäure versetzt und 40 Std. bei 43°C gerührt. Nach dem Abkühlen wurde der ausgefallene Niederschlag abgesaugt, mit wenig Methylenchlorid gewaschen und getrocknet. Man erhielt 23,3 g der Titelverbindung vom Fp. 222-223°C. Aus dem Filtrat wurden weitere 13 g vom Fp. 205-208°C isoliert, so daß sich die Gesamtausbeute auf 65 % d. Th. belief.

### c) 8-(2',4'-dichlor-5'-methoxyimino-methyl-phenyl)-4-oxa-7,9-dioxo-1,2,8-triaza[4.3.0.]nonan (Verbindung 1.06 aus Tabelle 1

9,8 g (0,0495 mol) Diphosgen wurden innerhalb 10 Min. unter Rühren bei 0 bis (-5)°C zu einer Lösung von 15 g (0,045 mol) der in Stufe b) erhaltenen Verbindung in 200 ml Pyridin gegeben. Nach 1 Std. Rühren bei 0-10°C and 12 Std. unter Erwärmen bis auf 22°C wurde das Reaktionsgemisch eingeengt, in Methylenchlorid aufgenommen und je zweimal mit Wasser und 1n Salzsäure gewaschen. Nach dem Trocknen über Magnesiumsulfat, Rühren über Aktivkohle und Absaugen über Kieselgel wurde die Reaktionslösung eingeengt. Man erhielt 11,3 g (68 % d. Th.) der Titelverbindung vom Fp. 184-185°C.

### Beispiel 5 (Verfahren)

### a) N-Methylenimino-N-2-hydroxyethyl-N'-(4-chlor-2-fluor-5-propargyloxy-phenyl)harnstoff

5 g (16,57 mmol) N-Amino-N-2-hydroxyethyl-N'-(4-chlor-2-fluor-5-propargyloxy-phenyl)harnstoff (gelbes Harz, hergestellt analog Beispiel 1) und 1,5 g (18,23 mmol) 37 %iges Formalin wurden in 250 ml Methylenchlorid vorgegeben und 3 Std. bei 42°C gerührt. Nach dem Abkühlen versetzte man das Reaktionsgemisch mit 2 Eßlöffeln Magnesiumsulfat. Dann wurde über 200 ml Kieselgel chromatographiert. Ausbeute: 3,77 g (72,4 % d.Th.) der Wertprodukte als gelbes Harz.
¹H-NMR (in (CD₃)₂SO) : δ [ppm] = 7,55 (d/1H), 7,95 (d/1H) Ph, 6,6 (d/1H), 7,0 (d/1H) N=CH₂.

### b) Tetrahydro-N-(4-chlor-2-fluor-5-propargyloxy-phenyl)-4H-1,3,4-oxdiazin-4-carboxamid

Eine Lösung von 4,9 g (15,62 mmol) der Verbindung 5a in 100 ml Essigsäure wurde 24 Std. bei 60°C gerührt, wonach man das Reaktionsgemisch abkühlte und im Vakuum einengte. Der Rückstand wurde mit Methylenchlorid:Diethylether (2:1) über Kieselgel chromatographiert. Es wurde mit Methylenchlorid, zum Schluß mit Essigester chromatographiert. Ausbeute: 1,4 g (29 % d.Th.) des Wertproduktes als gelbes Harz.
¹H-NMR (in (CD₃)₂SO) : δ [ppm] = 8,6 (s/1H) NH; 8,1 (d/1H), 7,48 (d/1H) Ph, 5,92 (t/1H)OH; 4,85 (s/2) OCH₂; 4,6-4,25 (m/2H), 4,9-3,6 (m/4H); 3,6 (s/1H) -C≡CH.

### Beispiel 6 (Vergleich, Verfahren)

### Tetrahydro-N-(4-chlor-2-fluor-5-propargyloxy-phenyl)4H-1,3,4-oxdiazin-4-carboxamid

5,0 g (16,57 mmol) des gleichen wie in Beispiel 5a verwendeten Ausgangsmaterials 1,5 g (18,23 mmol) 37 %iges Formalin und 0,5 g p-Toluolsulfonsäure wurden in Methylenchlorid 17 Std. bei 42°C am Wasserauskreiser gerührt. Nach dem Abkühlen engte man das Reaktionsgemisch im Vakuum teilweise ein. Die Reinigung erfolgte mittels Chromatographie über 200 ml Kieselgel (Laufmittel, zunächst Methylenchlorid, dann Essigsäureethylester). Ausbeute: 3,6 g eines Substanzgemisches, das nach DC, HPLC und NMR aus 6 - 7 Substanzen bestand. Nach der HPLC-Untersuchung war darin die Titelverbindung mit 0,74 g (14,2 % d.Th.) enthalten.
Beim Nachspülen der Säule mit Methanol konnte nur p-Toluolsulfonsäure isoliert werden.

### Anwendungsbeispiele

Die herbizide Wirkung der anellierten Triazole der Formel I' ließ sich durch Gewächshausversuche mit der Verbindung aus Beispiel 1 (1.05 aus Tabelle 1) zeigen:

Als Kulturgefäße dienten Plastiktöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0.0313 bzw. 0.0156 kg/ha a.S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Versuchen verwendeten Pflanzen setzten sich aus folgenden Arten zusammen:

| Kurzbezeichnung | Latein. Name | Deutscher Name | Englischer Name |
|---|---|---|---|
| ABUTH | Abutilon theoprasti | Chinesischer Hanf | velvet leaf |
| CHEAL | Chenopodium album | Weißer Gänsefuß | lambsquarters (goosefoot) |
| GALAP | Galium aparine | Klettenlabkraut | catchweed bedstraw |
| POLPE | Polygonum persicaria | Flohknöterich | ladysthumb |
| SOLNI | Solanum nigrum | Schwarzer Nachtschatten | black nightshade |
| VERSS | Veronica spp. | Ehrenpreisarten | speedwell |

Bei der Prüfung der Verbindung aus Bsp. ld wurden folgende Ergebnisse erhalten:

| Bsp-Nr. | 7 | 8 |
|---|---|---|
| Aufwandmenge (kg/ha a. S.) | 0.0313 | 0.0156 |
| Testpflanzen | | |
| ABUTH | 100 | 100 |
| CHEAL | 98 | 98 |
| GALAP | 98 | 98 |
| POLPE | 98 | 98 |
| SOLNI | 100 | 100 |
| VERSS | 100 | 100 |

Diese Ergebnisse belegen die gute herbizide Wirksamkeit der neuen anellierten Triazole I'.

## Patentansprüche

1. verfahren zur Herstellung von annellierten Triazolen der Formel I, 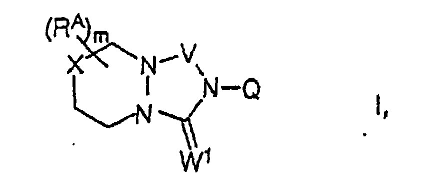 wobei die Substituenten folgende Bedeutungen haben:
X O, S, SO, SO₂ oder N(R¹);
V C=W²;
W¹ Sauerstoff oder Schwefel;
W² Sauerstoff;
R^{A} Wasserstoff, Hydroxy, COOH, COOR², Halogen, Cyano, C(O)NR¹¹R¹², OR³, C₁-C₆-Alkyl, C₁-C₆-Haloalkyl, C₃-C₆-Alkenyl, C₃-C₆-Alkinyl, COR³, S(O)ₙR³ oder C(O)SR²;
R¹ Wasserstoff, Hydroxy, Halogen, CN, C₁-C₆-Alkyl, C₁-C₆-Haloalkyl, C₂-C₆-Alkenyl, C₂-C₆-Alkinyl, COR³, CHO, OR³, COOR², C(O)SR², C(O)NR¹¹R¹²;
R² Wasserstoff, C₁-C₆-Alkyl, C₁-C₆-Haloalkyl, C₂-C₆-Alkenyl oder C₂-C₆-Alkinyl;
R³ C₁-C₆-Alkyl, C₃-C₆-Cycloalkyl, C₂-C₆-Alkenyl, C₂-C₆-Alkinyl, C₁-C₆-Haloalkyl, C₁-C₆-Alkoxy-C₁-C₆-alkyl, Carboxyl-C₁-C₆-alkyl, C₁-C₆-Alkoxycarbonyl-C₁-C₆-alkyl, C₁-C₆-Alkylthio-C₁-C₆-alkyl, C₁-C₆-Alkylsulfinyl-C₁-C₆-alkyl, C₁-C₆-Alkylsulfonyl-C₁-C₆-alkyl, C₃-C₆-Cycloalkyl-C₁-C₆-alkyl, C₃-C₆-Alkenyloxycarbonyl-C₁-C₆-alkyl, C₃-C₆-Alkinyloxycarbonyl-C₁-C₆-alkyl, C₃-C₆-Cycloalkoxy-C₁-C₆-alkyl, C₃-C₆-Alkenyloxy-C₁-C₆-alkyl, C₃-C₆-Alkinyloxy-C₁-C₆-alkyl, C₁-C₆-Haloalkoxy-C₁-C₆-alkyl, C₃-C₆-Haloalkenyloxy-C₁-C₆-alkyl, C₃-C₆-Haloalkinyloxy-C₁-C₆-alkyl, C₃-C₆-Cycloalkyl-C₁-C₆-thioalkyl, C₃-C₆-Alkenylthio-C₁-C₆-alkyl, C₃-C₆-Alkinylthio-C₁-C₆-alkyl, Cyano-C₁-C₆-alkyl, C₃-C₆-Halocyclo-C₁-C₆-alkyl, Halogen-C₃-C₆-alkenyl, C₁-C₆-Alkoxy-C₃-C₆-alkenyl, C₁-C₆-Haloalkoxy-C₃-C₆-alkenyl, C₁-C₆-Alkylthio-C₃-C₆-alkenyl, C₃-C₆-Haloalkinyl, C₁-C₆-Alkoxy-C₃-C₆-alkinyl, C₃-C₆-Haloalkoxyalkinyl, C₁-C₆-Alkylthioalkinyl, C₁-C₆-Alkylcarbonyl, CHR¹⁶COR¹⁷, CHR¹⁶P(O)(OR¹⁷)₂, P(O)(OR¹⁷)₂, CHR¹⁶P(S)(OR¹⁷)₂, CHR¹⁶C(O)NR¹¹R¹², CHR¹⁶C(O)NH₂, C₁-C₆-Alkyl, welches mit Phenoxy oder Benzyloxy substituiert ist, wobei die Ringe ihrerseits mit Halogen, C₁-C₄-Alkyl oder C₁-C₄-Haloalkyl substituiert sein können, Benzyl welches mit Halogen, C₁-C₄-Alkyl oder C₁-C₄-Haloalkyl substituiert sein kann oder Phenyl und pyridyl, welche mit Halogen, C₁-C₄-Alkyl, C₁-C₄-Haloalkyl oder C₁-C₄-Alkoxy substituiert sein können;
m den Wert 0, 1, 2 oder 3;
n den Wert 0, 1 oder 2;
Q einen der Reste Q-1 bis Q-7 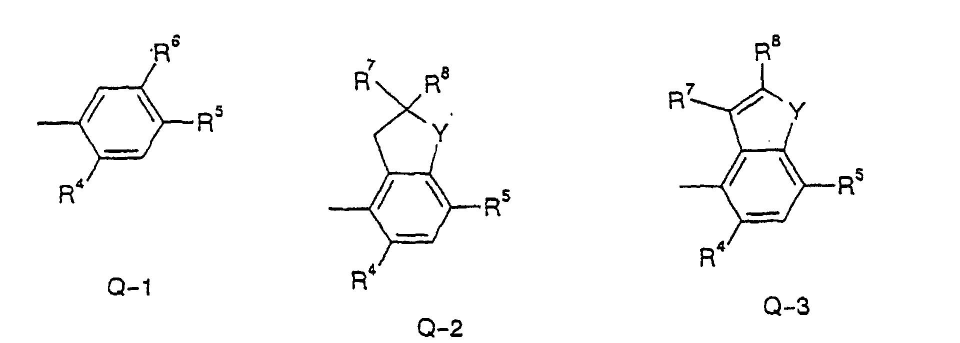 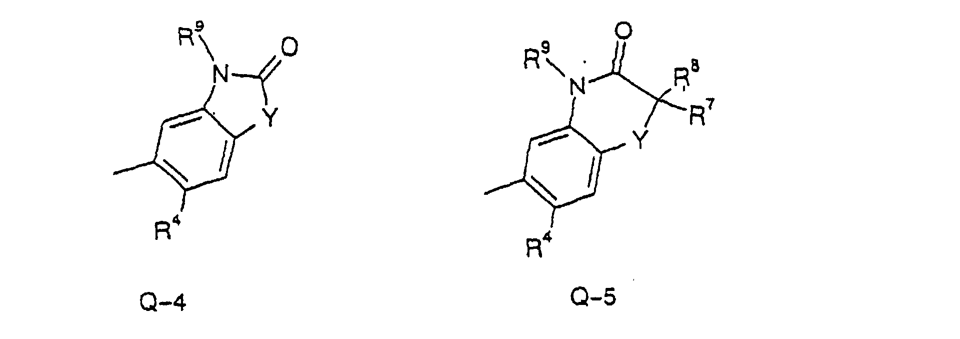 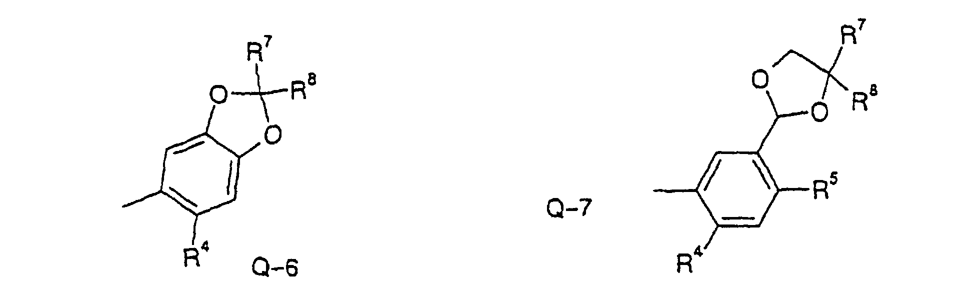
mit
Y Sauerstoff oder Schwefel;
R⁴ Wasserstoff oder Halogen;
R⁵ C₁-C₆-Alkyl, C₁-C₆-Haloalkyl, OCH₃, SCH₃, OCHF₂, Halogen, CN oder NO₂;
R⁶ Wasserstoff, C₁-C₆-Alkyl, C₁-C₆-Haloalkyl, Halogen, OR¹⁰, S(O)ₙR¹⁰, COR¹⁰, COOR¹⁰, C(O)SR¹⁰, SCH₂C≡CH, C(O)NR¹¹R¹², CHO, CH=CHCO-OR¹⁰, CO-ON=CR¹³R¹⁴, NO₂, CN, NHSO₂R¹⁵, NHSO₂NHR¹⁵, -C(R¹⁸)=C(R¹⁹)-CO-R²⁰, -CH(R¹⁸)-CH(R¹⁹)-CO-R²⁰, -C(R¹⁸)=C(R¹⁹)-CO-N(R²⁰,R²¹), -CH(R¹⁸)-CH(R¹⁹)-CO-N(R²⁰,R²¹), -C(R²¹)=N-OR²², -COOC(R²³)(R²⁴)-COOR²⁵, -CO-N(R²⁶)-OR²² oder -C(OR²⁷)=N-OR²²;
R⁷, R⁸ unabhängig voneinander Wasserstoff, C₁-C₆-Alkyl, C₁-C₆-Haloalkyl oder Halogen; wenn Q Q-2, Q-5 oder Q-6 ist, können R⁷ und R⁸ zusammen mit dem C-Atom, an das sie gebunden sind, eine Gruppe C=O bilden;
R⁹ C₁-C₆-Alkyl, C₁-C₆-Haloalkyl, C₁-C₆-Alkoxy, C₁-C₆-Alkoxy-C₁-C₆-alkyl, C₂-C₆-Alkenyl oder C₂-C₆-Alkinyl;
R¹⁰ einen der unter R³ angegebenen Reste;
R¹¹, R¹³ unabhängig voneinander Wasserstoff oder C₁-C₆-Alkyl;
R¹², R¹⁴ unabhängig voneinander C₁-C₆-Alkyl oder Phenyl, welches mit Halogen, C₁-C₄-Alkyl, C₁-C₄-Haloalkyl oder C₁-C₄-Alkoxy substituiert sein kann;
R¹¹ und R¹² können zusammen eine Gruppierung -(CH₂)₅-, -(CH₂)₄- oder -CH₂CH₂OCH₂CH₂-sein, wobei jeder Ring mit C₁-C₃-Alkyl substituiert sein kann, oder Phenyl oder Benzyl;
R¹³ und R¹⁴ können auch zusammen mit dem C-Atom, an das sie gebunden sind, eine C₃-C₈-Cyclyoalkylgruppe bilden;
R¹⁵ C₁-C₆-Alkyl oder C₁-C₆-Haloalkyl;
R¹⁶ Wasserstoff oder C₁-C₆-Alkyl;
R¹⁷ C₁-C₆-Alkyl, C₂-C₆-Alkenyl oder C₂-C₆-Alkinyl;
R¹⁸, R²³, R²⁴ jeweils wasserstoff oder C₁-C₃-Alkyl;
R¹⁹ Halogen, Cyano oder Methyl;
R²⁰ Hydroxy, C₁-C₆-Alkoxy, C₃-C₆-Cycloalkoxy, C₁-C₄-Alkoxy-C₁-C₄-alkoxy, C₁-C₄-Alkylthio-C₁-C₄-alkoxy, C₁-C₄-Alkylsulfinyl-C₁-C₄-alkoxy, C₁-C₄-Alkylsulfonyl-C₁-C₄-alkoxy, Cyano-C₁-C₆-alkoxy, C₁-C₄-Alkoxycarbonyl-C₁-C₄-alkoxy, C₃-C₆-Alkenyloxy, C₃-C₆-Alkinyloxy, partiell oder vollständig halogeniertes C₁-C₆-Alkoxy, partiell oder vollständig halogeniertes C₃-C₆-Alkenyloxy, partiell oder vollständig halogeniertes C₃-C₆-Alkinyloxy, C₁-C₆-Alkylthio ferner C₁-C₆-Alkoxy das noch zwei C₁-C₆-Alkoxysubstituenten tragen kann,
R²¹ Wasserstoff, C₁-C₆-Alkyl oder C₁-C₆-Aalogenalkyl;
R²² Wasserstoff, C₁-C₆-Alkyl, C₁-C₄-Alkoxy-C₁-C₄-alkyl, C₁-C₆-Halogenalkyl, C₁-C₆-Cyanoalkyl, C₃-C₆-Cycloalkyl, C₃-C₆-Alkenyl, C₃-C₆-Alkinyl, C₃-C₆-Halogenalkenyl, C₃-C₆-Halogenalkinyl, Carboxy-C₁-C₆-alkyl, C₁-C₆-Alkoxycarbonyl-C₁-C₄-alkyl, C₁-C₆-Alkylcarbonyl-C₁-C₆-alkyl oder C₁-C₆-Alkylcarbonyloxy-C₁-C₆-alkyl;
R²⁵ Wasserstoff, C₁-C₆-Alkyl, C₃-C₆-Cycloalkyl, C₁-C₆-Alkoxy-C₁-C₄-alkyl, Cyano-C₁-C₆-alkyl, Halogen-C₁-C₆-alkyl, C₃-C₆-Alkenyl oder C₃-C₆-Alkinyl;
R²⁶,R²⁷ unabhängig voneinander C₁-C₆-Alkyl, C₁-C₄-Alkoxy-C₁-C₄-alkyl, C₃-C₆-Cycloalkyl, C₃-C₆-Alkenyl, C₃-C₆-Alkinyl, C₁-C₆-Halogenalkyl, C₁-C₆-Cyanoalkyl, C₃-C₆-Halogenalkenyl, C₃-C₆-Halogenalkinyl oder C₁-C₆-Alkoxycabonyl-C₁-C₄-alkyl;
R²⁶ darüberhinaus Wasserstoff,
**dadurch gekennzeichnet, daß** man in einem ersten Schritt Verbindungen der Formel II 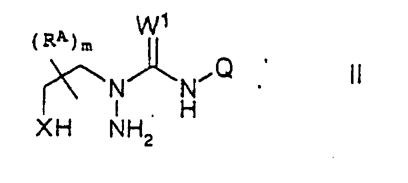 mit wässrigem Formaldehyd oder Paraformaldehyd in Abwesenheit von Säuren in neutralem oder schwach alkalischem Medium zu N-Methylenimino-N'-substituierten Harnstoffen der Formel III 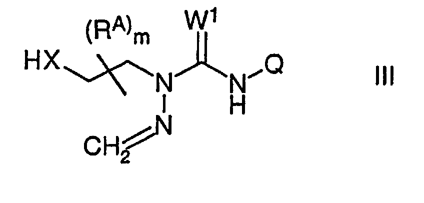 umsetzt, diese dann in Gegenwart katalytischer Mengen Säure oder eines neutralen oder sauren oberflächenaktiven Metalloxids zu substituierten Tetrahydro-4H-1,3,4-ox(bzw. thia)diazinen der Formel IV 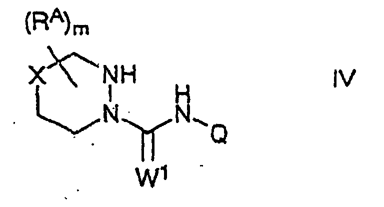 cyclisiert und letztere mit Phosgen oder einem Phosgenersatz zu den Verbindungen der Formel I cyclisiert.

2. Verfahren nach Anspruch 1, **dadurch gekennzeichnet, daß** man die Cyclisierung der Verbindungen der Formel III zu den substituierten Tetrahydro-4H-1,3,4-ox(bzw, thia)diazinen der Formel IV in Gegenwart eines oberflächenaktiven Metalloxids durchführt.

3. Verfahren nach Anspruch 2, **dadurch gekennzeichnet, daß** man als oberflächenaktives Metalloxid Kieselgel verwendet.

4. Anellierte Triazole der Formel I' 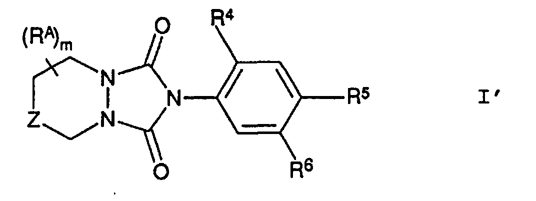 in der die Variablen die Bedeutung haben:
Z O, S, S=O oder SO₂;
R^{A} Halogen oder C₁-C₃-Alkyl;
R⁴ Wasserstoff oder Halogen;
R⁵ Halogen, Cyano oder Trifluormethyl und
R⁶ eine Gruppe -C(R¹⁸)=C(R¹⁹)-CO-R²⁰, -CH(R¹⁸)-CH(R¹⁹)-CO-R²⁰, -C(R¹⁸)=C(R¹⁹)-CO-N(R²⁰,R²¹), -CH(R¹⁸)-CH(R¹⁹)-C=O-N(R²⁰,R²¹), -C(R²¹)=N-O-R²², -CO-OC(R²³)(R²⁴)-CO-O-R²⁵, CO-N(R²⁶)-OR²² oder C(O-R²⁷)=N-OR²²;
R¹⁸, R²³, R²⁴ jeweils Wasserstoff oder C₁-C₃-Alkyl;
R¹⁹ Halogen, Cyano oder Methyl;
R²⁰ Hydroxy, C₁-C₆-Alkoxy, C₃-C₆-Cycloalkoxy, C₁-C₄-Alkoxy-C₁-C₄-alkoxy, C₁-C₄-Alkylthio-C₁-C₄-alkoxy, C₁-C₄-Alkylsulfinyl-C₁-C₄-alkoxy, C₁-C₄-Alkylsulfonyl-C₁-C₄-alkoxy, Cyano-C₁-C₆-alkoxy, C₁-C₄-Alkoxycarbonyl-C₁-C₄-alkoxy, C₃-C₆-Alkenyloxy, C₃-C₆-Alkinyloxy, partiell oder vollständig halogeniertes C₁-C₆-Alkoxy, partiell oder vollständig halogeniertes C₃-C₆-Alkenyloxy, partiell oder vollständig halogeniertes C₃-C₆-Alkinyloxy, C₁-C₆-Alkylthio,
ferner C₁-C₆-Alkoxy, das noch zwei C₁-C₆-Alkoxysubstituenten tragen kann,
R²¹ Wasserstoff, C₁-C₆-Alkyl oder C₁-C₆-Halogenalkyl;
R²² Wasserstoff, C₁-C₆-Alkyl, C₁-C₄-Alkoxy-C₁-C₄-alkyl, C₁-C₆-Halogenalkyl, C₁-C₆-Cyanoalkyl, C₃-C₆-Cycloalkyl, C₃-C₆-Alkenyl, C₃-C₆-Alkinyl, C₃-C₆-Halogenalkenyl, C₃-C₆-Halogenalkinyl, Carboxy-C₁-C₆-alkyl, C₁-C₆-Alkoxycarbonyl-C₁-C₄-alkyl, C₁-C₆-Alkylcarbonyl-C₁-C₆-alkyl oder C₁-C₆-Alkylcarbonyloxy-C₁-C₆-alkyl;
R²⁵ Wasserstoff, C₁-C₆-Alkyl, C₃-C₆-Cycloalkyl, C₁-C₆-Alkoxy-C₁-C₄-alkyl, Cyano-C₁-C₆-alkyl, Halogen-C₁-C₆-alkyl, C₃-C₆-Alkenyl oder C₃-C₆-Alkinyl;
R²⁶, R²⁷ unabhängig voneinander C₁-C₆-Alkyl, C₁-C₄-Alkoxy-C₁-C₄-alkyl, C₃-C₆-Cycloalkyl, C₃-C₆-Alkenyl, C₃-C₆-Alkinyl, C₁-C₆-Halogenalkyl, C₁-C₆-Cyanoalkyl, C₃-C₆-Halogenalkenyl, C₃-C₆-Halogenalkinyl oder C₁-C₆-Alkoxycarbonyl-C₁-C₄alkyl;
R²⁶ darüberhinaus Wasserstoff und
m 0, 1, 2 oder 3,
sowie die landwirtschaftlich brauchbaren Salze der Verbindungen I.

5. Annellierte Triazole der Formel I' nach Anspruch 4, wobei die Substituenten und der Index m folgende Bedeutungen haben:
Z Sauerstoff;
R⁵ Chlor oder Cyano;
R¹⁸, R²³, R²⁴ jeweils Wasserstoff oder Methyl;
R¹⁹ Halogen oder Cyano;
R²⁰ C₁-C₆-Alkoxy, C₃-C₅-Cycloalkoxy, C₁-C₄-Alkoxy-C₁-C₄-alkoxy, C₁-C₄-Alkoxycarbonyl-C₁-C₄-alkoxy, C₃-C₆-Alkenyloxy, C₃-C₆-Alkinyloxy oder C₁-C₄-Halogenalkoxy;
R²¹ Wasserstoff oder C₁-C₄-Alkyl;
R²² C₁-C₆-Alkyl, C₃-C₆-Alkenyl, C₃-C₆-Alkinyl oder C₁-C₆-Alkoxycarbonyl-C₁-C₂-alkyl;
R²⁵ C₁-C₆-Alkyl, C₁-C₆-Alkoxy-C₁-C₄-alkyl, C₃-C₆-Alkenyl oder C₃-C₆ Alkinyl;
R²⁶ Wasserstoff, C₁-C₆-Alkyl, C₁-C₄-Alkoxy-C₁-C₄-alkyl, C₃-C₆-Alkenyl, C₃-C₆-Alkinyl oder C₁-C₆-Alkoxy-carbonyl-C₁-C₂alkyl;
R²⁷ C₁-C₆-Alkyl, C₁-C₄-Alkoxy-C₁-C₄-alkyl, C₃-C₆-Alkenyl, C₃-C₆-Alkinyl oder C₁-C₆-Alkoxy-carbonyl-C₁-C₂-alkyl;
m 0,
sowie deren landwirtschaftlich brauchbaren Salze.

6. Substituierte N-Methylenimino-N'-phenylharnstoffe der Formel III' 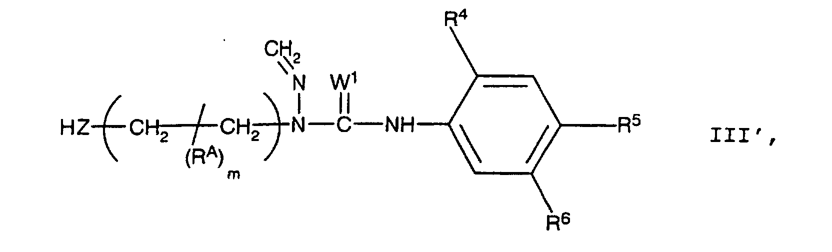 in der die Substituenten folgende Bedeutungen haben:
Z O, S, S=O oder SO₂;
R^{A} Halogen oder C₁-C₃-Alkyl;
m 0, 1, 2 oder 3;
W¹ Sauerstoff oder Schwefel;
R⁴ Wasserstoff oder Halogen;
R⁵ Halogen, Cyano oder Trifluormethyl;
R⁶ eine Gruppe -C(R¹⁸)=C(R¹⁹)-CO-R²⁰, -CH(R¹⁸)-CH(R¹⁹)-CO-R²⁰, -C(R¹⁸)=C(R¹⁹)-CO-N(R²⁰;R²¹), -CH(R¹⁸)-CH(R¹⁹)-CO-N(R²⁰, R²¹), -C(R²¹)=N-OR²², -CO-OC(R²³)(R²⁴)-CO-OR²⁵, CO-N(R²⁶)-OR²² oder C(O-R²⁷)=N-OR²²;
R¹⁸, R²³, R²⁴ jeweils Wasserstoff oder C₁-C₃-Alkyl;
R¹⁹ Halogen, Cyano oder Methyl;
R²⁰ Hydroxy, C₁-C₆-Alkoxy, C₃-C₆-Cycloalkoxy, C₁-C₄-Alkoxy-C₁-C₄-alkoxy, C₁-C₄-Alkylthio-C₁-C₄-alkoxy, C₁-C₄-Alkylsulfinyl-C₁-C₄-alkoxy, C₁-C₄-Alkylsulfonyl-C₁-C₄-alkoxy, Cyano-C₁-C₆-alkoxy, C₁-C₄-Alkoxycarbonyl-C₁-C₄-alkoxy, C₃-C₆-Alkenyloxy, C₃-C₆-Alkinyloxy, partiell oder vollständig halogeniertes C₁-C₆-Alkoxy, partiell oder vollständig halogeniertes C₃-C₆-Alkenyloxy, partiell oder vollständig halogeniertes C₃-C₆-Alkinyloxy, C₁-C₆-Alkylthio,
ferner C₁-C₆-Alkoxy, das noch zwei C₁-C₆-Alkoxysubstituenten tragen kann;
R²¹ Wasserstoff, C₁-C₆-Alkyl oder C₁-C₆-Halogenalkyl;
R²² Wasserstoff C₁-C₆-Alkyl, C₁-C₄-Alkoxy-C₁-C₄-alkyl, C₁-C₆-Halogenalkyl, C₁-C₆-Cyanoalkyl, C₃-C₆-Cycloalkyl, C₃-C₆-Alkenyl, C₃-C₆-Alkinyl, C₃-C₆-Halogenalkenyl, C₃-C₆-Halogenalkinyl, Carboxy-C₁-C₆-alkyl, C₁-C₆-Alkoxycarbonyl-C₁-C₄-alkyl, C₁-C₆-Alkylcarbonyl-C₁-C₆-alkyl oder C₁-C₆-Alkylcarbonyloxy-C₁-C₆-alkyl;
R²⁵ Wasserstoff, C₁-C₆-Alkyl, C₃-C₆-Cycloalkyl, C₁-C₆-Alkoxy-C₁-C₄-alkyl, Cyano-C₁-C₆-alkyl, Halogen-C₁-C₆-alkyl, C₃-C₆-Alkenyl oder C₃-C₆-Alkinyl;
R²⁶, R²⁷ unabhängig voneinander C₁-C₆-Alkyl, C₁-C₄-Alkoxy-C₁-C₄-alkyl, C₃-C₆-Cycloalkyl, C₃-C₆-Alkenyl, C₃-C₆-Alkinyl, C₁-C₆-Halogenalkyl, C₁-C₆-Cyanoalkyl, C₃-C₆-Halogenalkenyl, C₃-C₆-galogenalkinyl oder C₁-C₆-Alkoxycarbonyl-C₁-C₄alkyl, und
R²⁶ darüberhinaus Wasserstoff.

7. Substituierte N-Methylenimino-N'-phenylharnstoffe der Formel III' nach Anspruch 6, wobei die Substituenten die folgenden Bedeutungen haben:
Z Sauerstoff;
R⁵ Chlor oder Cyano;
R¹⁸, R²³, R²⁴ jeweils Wasserstoff oder Methyl;
R¹⁹ Halogen oder Cyano;
R²⁰ C₁-C₆-Alkoxy, C₃-C₅-Cycloalkoxy, C₁-C₄-Alkoxy-C₁-C₄-alkoxy, C₁-C₄-Alkoxycarbonyl-C₁-C₄-alkoxy, C₃-C₆-Alkenyloxy, C₃-C₆-Alkinyloxy oder C₁-C₄-Halogenalkoxy;
R²¹ Wasserstoff oder C₁-C₄-Alkyl;
R²² C₁-C₆-Alkyl, C₃-C₆-Alkenyl, C₃-C₆-Alkinyl oder C₁-C₆-Alkoxycarbonyl-C₁-C₂-alkyl;
R²⁵ C₁-C₆-Alkyl, C₁-C₆-Alkoxy-C₁-C₄-alkyl, C₃-C₆-Alkenyl oder C₃-C₆ Alkinyl;
R²⁶ Wasserstoff, C₁-C₆-Alkyl, C₁-C₄-Alkoxy-C₁-C₄-alkyl, C₃-C₆-Alkenyl, C₃-C₆-Alkinyl oder C₁-C₆-Alkoxy-carbonyl-C₁-C₂alkyl;
R²⁷ C₁-C₆-Alkyl, C₁-C₄-Alkoxy-C₁-C₄-alkyl, C₃-C₆-Alkenyl, C₃-C₆-Alkinyl oder C₁-C₆-Alkoxy-carbonyl-C₁-C₂-alkyl;
m Null.

8. Verwendung der anellierten Triazole der Formel I' und der landwirtschaftlichen brauchbaren Salze von I' gemäß Anspruch 4, als Herbizide oder zur Desikkation und/oder Defoliation von Pflanzen.

9. Berbizides Mittel, enthaltend eine herbizid wirksame Menge mindestens eines anellierten Triazols der Formel I' oder eines landwirtschaftlich brauchbaren Salzes von I' gemäß Anspruch 4, und mindestens einen inerten flüssigen und/oder festen Trägerstoff sowie gewünschtenfalls mindestens ein Adjuvans.

10. Mittel zur Desikkation und/oder Defoliation von Pflanzen, enthaltend eine desikkant und/oder eine defoliant wirksame Menge mindestens eines anellierten Triazols der Formel I' oder eines landwirtschaftlich brauchbaren Salzes von I' gemäß Anspruch 4, und mindestens einen inerten flüssigen und/oder festen Trägerstoff sowie gewünschtenfalls mindestens ein Adjuvans.

11. Verfahren zur Bekämpfung von unerwünschtem Pflanzenwuchs, **dadurch gekennzeichnet, daß** man eine herbizid wirksame Menge mindestens eines anellierten Triazols der Formel I' oder eines landwirtschaftlich brauchbaren Salzes von I' gemäß Anspruch 4 auf Pflanzen, deren Lebensraum oder auf Saatgut einwirken läßt.

12. Verfahren zur Desikkation und/oder Defoliation von Pflanzen, **dadurch gekennzeichnet, daß** man eine desikkant und/oder eine defoliant wirksame Menge mindestens eines anellierten Triazols der Formel I' oder eines landwirtschaftlich brauchbaren Salzes von I' geinäß Anspruch 4 auf Pflanzen einwirken läßt.

13. Anellierte Triazole der Formel I'' 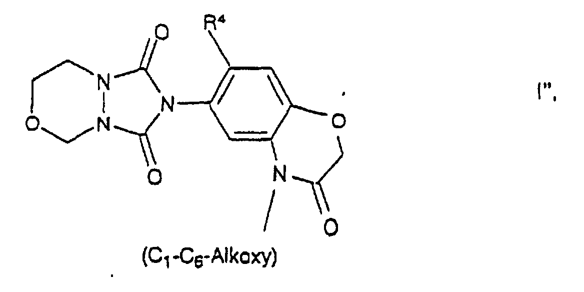 wobei R⁴ für Wasserstoff oder Halogen steht.

## Claims

1. A process for preparing fused triazoles of the formula I, where: 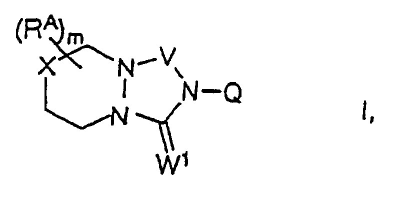
X is O, S, SO, SO₂ or N(R¹);
V is C=W²;
W¹ is oxygen or sulfur;
W₂ is oxygen;
R^{A} is hydrogen, hydroxyl, COOH, COOR², halogen, cyano, C(O)NR¹¹R¹², OR³, C₁-C₆-alkyl, C₁-C₆-haloalkyl, C₃-C₆-alkenyl, C₃-C₆-alkynyl, COR³, S(O)ₙR³ or C(O)_{S}R²;
R¹ is hydrogen, hydroxyl, halogen, CN, C₁-C₆-alkyl, C₁-C₆-haloalkyl, C₂-C₆-alkenyl, C₂-C₆-alkynyl, COR³, CHO, OR³, COOR², C(O)SR², C(O)NR¹¹R¹²;
R² is hydrogen, C₁-C₆-alkyl, C₁-C₆-haloalkyl, C₂-C₆-alkenyl or C₂-C₆-alkynyl;
R³ is C₁-C₆-alkyl, C₃-C₆-cycloalkyl, C₂-C₆-alkenyl, C₂-C₆-alkynyl, C₁-C₆-haloalkyl, C₁-C₆-alkoxy-C₁-C₆-alkyl, carboxyl-C₁-C₆-alkyl, C₁-C₆-alkoxycarbonyl-C₁-C₆-alkyl, C₁-C₆-alkylthio-C₁-C₆-alkyl, C₁-C₆-alkylsulfinyl-C₁-C₆-alkyl, C₁-C₆-alkylsulfonyl-C₁-C₆-alkyl, C₃-C₆-cycloalkyl-C₁-C₆-alkyl, C₃-C₆-alkenyloxycarbonyl-C₁-C₆-alkyl, C₃-C₆-alkynyloxycarbonyl-C₁-C₆-alkyl, C₃-C₆-cycloalkoxy- C₁-C₆-alkyl, C₃-C₆-alkenyloxy-C₁-C₆-alkyl, C₃-C₆-alkynyloxy-C₁-C₆-alkyl, C₁-C₆-haloalkoxy-C₁-C₆-alkyl, C₃-C₆-haloalkenyloxy-C₁-C₆-alkyl, C₃-C₆-haloalkynyloxy-C₁-C₆-alkyl, C₃-C₆-cycloalkyl-C₁-C₆-thioalkyl, C₃-C₆-alkenylthio-C₁-C₆-alkyl, C₃-C₆-alkynylthio-C₁-C₆-alkyl, cyano-C₁-C₆-alkyl, C₃-C₆-halocyclo-C₁-C₆-alkyl, halo-C₃-C₆-alkenyl, C₁-C₆-alkoxy-C₃-C₆-alkenyl, C₁-C₆-haloalkoxy-C₃-C₆-alkenyl, C₁-C₆-alkylthio-C₃-C₆-alkenyl, C₃-C₆-haloalkynyl, C₁-C₆-alkoxy-C₃-C₆-alkynyl, C₃-C₆-haloalkoxyalkynyl, C₁-C₆-alkylthioalkynyl, C₁-C₆-alkylcarbonyl, CHR¹⁶COR¹⁷, CHR¹⁶P(O)(OR¹⁷)₂, P(O)(OR¹⁷)₂, CHR¹⁶P(S)(OR¹⁷)₂, CHR¹⁶C(O)NR¹¹R¹², CHR¹⁶C(O)NH₂, C₁-C₆-alkyl, which is substituted by phenoxy or benzyloxy, where the rings for their part may be substituted by halogen, C₁-C₄-alkyl or C₁-C₄-haloalkyl, benzyl which may be substituted by halogen, C₁-C₄-alkyl or C₁-C₄-haloalkyl, or is phenyl or pyridyl which may be substituted by halogen, C₁-C₄-alkyl, C₁-C₄-haloalkyl or C₁-C₄-alkoxy;
m has the value 0, 1, 2 or 3;
n has the value 0, 1 or 2;
Q is one of the radicals Q-1 to Q-7 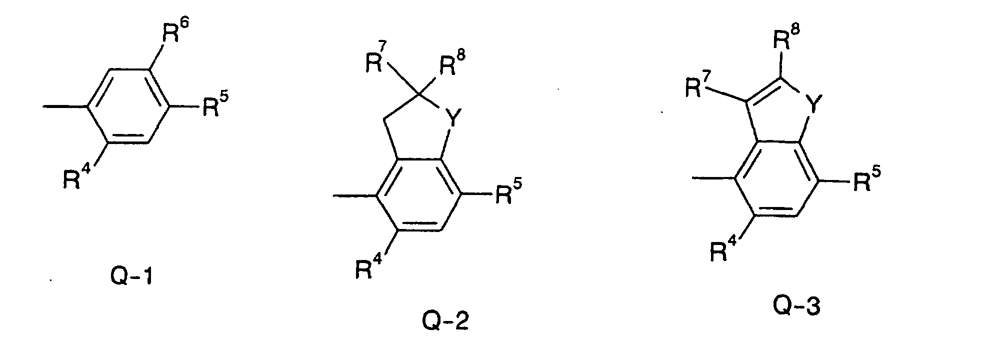 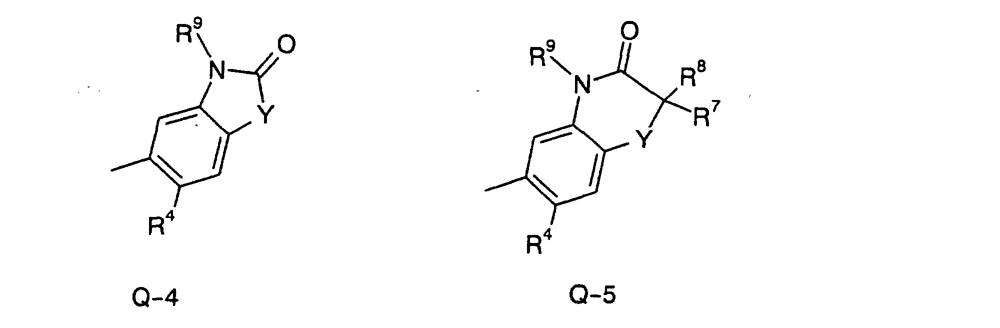 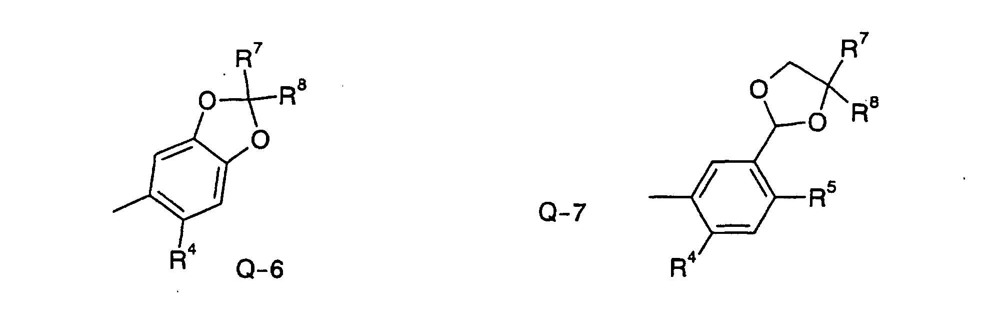
where
Y is oxygen or sulfur;
R⁴ is hydrogen or halogen;
R⁵ is C₁-C₆-alkyl, C₁-C₆-haloalkyl, OCH₃, SCH₃, OCHF₂, halogen, CN or NO₂;
R⁶ is hydrogen, C₁-C₆-alkyl, C₁-C₆-haloalkyl, halogen, OR¹⁰, S(O)ₙR¹⁰, COR¹⁰, COOR¹⁰, C(O)SR¹⁰, SCH₂C≡CH, C(O)NR¹¹R¹², CHO, CH=CHCO-OR¹⁰, CO-ON=CR¹³R¹⁴, NO₂, CN, NHSO₂R¹⁵, NHSO₂NHR¹⁵, -C(R¹⁸)=C(R¹⁹)-CO-R²⁰, -CH(R¹⁸)-CH(R¹⁹)-CO-R²⁰, -C(R¹⁸)=C(R¹⁹)-CO-N(R²⁰,R²¹), -CH(R¹⁸)-CH(R¹⁹)-CO-N(R²⁰,R²¹), -C(R²¹)=N-OR²², -COOC(R²³)(R²⁴)-COOR²⁵, -CO-N(R²⁶)-OR²² or -C(OR²⁷)=N-OR²²;
R⁷, R⁸ independently of one another are hydrogen, C₁-C₆-alkyl, C₁-C₆-haloalkyl or halogen; if Q is Q-2, Q-5 or Q-6, R⁷ and R⁸ may, together with the linking carbon atom, form a group C=O;
R⁹ is C₁-C₆-alkyl, C₁-C₆-haloalkyl, C₁-C₆-alkoxy, C₁-C₆-alkoxy-C₁-C₆-alkyl, C₂-C₆-alkenyl or C₂-C₆-alkynyl;
R¹⁰ is one of the radicals indicated under R³;
R¹¹, R¹³ independently of one another are hydrogen or C₁-C₆-alkyl;
R¹², R¹⁴ independently of one another are C₁-C₆-alkyl or phenyl which may be substituted by halogen, C₁-C₄-alkyl, C₁-C₄-haloalkyl or C₁-C₄-alkoxy;
R¹¹ and R¹² together may be a group -(CH₂)₅-, -(CH₂)₄- or -CH₂CH₂OCH₂CH₂- where each ring may be substituted by C₁-C₃-alkyl, or may be phenyl or benzyl;
R¹³ and R¹⁴ together with the linking carbon atom may also form a C₃-C₈-cycloalkyl group;
R¹⁵ is C₁-C₆-alkyl or C₁-C₆-haloalkyl;
R¹⁶ is hydrogen or C₁-C₆-alkyl;
R¹⁷ is C₁-C₆-alkyl, C₂-C₆-alkenyl or C₂-C₆-alkynyl;
R¹⁸, R²³, R²⁴ are each hydrogen or C₁-C₃-alkyl;
R¹⁹ is halogen, cyano or methyl;
R²⁰ is hydroxyl, C₁-C₆-alkoxy, C₃-C₆-cycloalkoxy, C₁-C₄-alkoxy-C₁-C₄-alkoxy, C₁-C₄-alkylthio-C₁-C₄-alkoxy, C₁-C₄-alkylsulfinyl-C₁-C₄-alkoxy, C₁-C₄-alkylsulfonyl-C₁-C₄-alkoxy, cyano-C₁-C₆-alkoxy, C₁-C₄-alkoxycarbonyl-C₁-C₄-alkoxy, C₃-C₆-alkenyloxy, C₃-C₆-alkynyloxy, partially or fully halogenated C₁-C₆-alkoxy, partially or fully halogenated C₃-C₆-alkenyloxy, partially or fully halogenated C₃-C₆-alkynyloxy, C₁-C₆-alkylthio, furthermore C₁-C₆-alkoxy which may carry two additional C₁-C₆-alkoxy substituents,
R²¹ is hydrogen, C₁-C₆-alkyl or C₁-C₆-haloalkyl;
R²² is hydrogen, C₁-C₆-alkyl, C₁-C₄-alkoxy-C₁-C₄-alkyl, C₁-C₆-haloalkyl, C₁-C₆-cyanoalkyl, C₃-C₆-cycloalkyl, C₃-C₆-alkenyl, C₃-C₆-alkynyl, C₃-C₆-haloalkenyl, C₃-C₆-haloalkynyl, carboxyl-C₁-C₆-alkyl, C₁-C₆-alkoxycarbonyl-C₁-C₄-alkyl, C₁-C₆-alkylcarbonyl-C₁-C₆-alkyl or C₁-C₆-alkylcarbonyloxy-C₁-C₆-alkyl;
R²⁵ is hydrogen, C₁-C₆-alkyl, C₃-C₆-cycloalkyl, C₁-C₆-alkoxy-C₁-C₄-alkyl, cyano-C₁-C₆-alkyl, halo-C₁-C₆-alkyl, C₃-C₆-alkenyl or C₃-C₆-alkynyl;
R²⁶,R²⁷ independently of one another are C₁-C₆-alkyl, C₁-C₄-alkoxy-C₁-C₄-alkyl, C₃-C₆-cycloalkyl, C₃-C₆-alkenyl, C₃-C₆-alkynyl, C₁-C₆-haloalkyl, C₁-C₆-cyanoalkyl, C₃-C₆-haloalkenyl, C₃-C₆-haloalkynyl or C₁-C₆-alkoxycarbonyl-C₁-C₄-alkyl;
R²⁶ is additionally hydrogen;
which comprises reacting, in a first step, compounds of the formula II 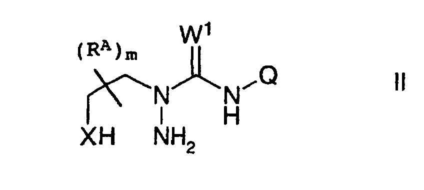 with aqueous formaldehyde or paraformaldehyde in the absence of acids in neutral or weakly alkaline medium to give N-methyleneimino-N'-substituted ureas of the formula III 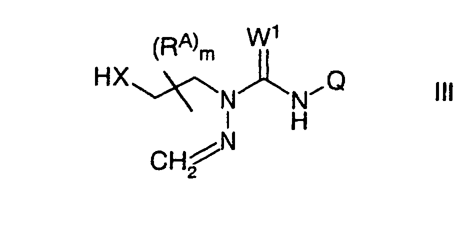 cyclizing these subsequently in the presence of catalytical amounts of acid or a neutral or acidic surface-active metal oxide to substituted tetrahydro-4H-1,3,4-oxa(or thia)diazines of the formula IV 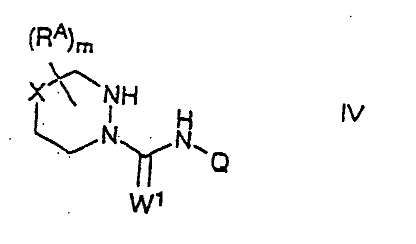 and cyclizing the latter with phosgene or a phosgene substitute to give the compounds of the formula I.

2. A process as claimed in claim 1, wherein the cyclization of the compounds of the formula III to the substituted tetrahydro-4H-1,3,4-oxa(or thia)diazines of the formula IV is carried out in the presence of a surface-active metal oxide.

3. A process as claimed in claim 2, wherein the surface-active metal oxide used is silica gel.

4. A fused triazole of the formula I' 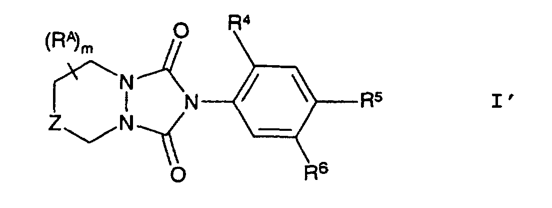 where:
Z is O, S, S=O or SO₂;
R^{A} is halogen or C₁-C₃-alkyl;
R⁴ is hydrogen or halogen;
R⁵ is halogen, cyano or trifluoromethyl and
R⁶ is a group -C(R¹⁸)=C(R¹⁹)-CO-R²⁰, -CH(R¹⁸)-CH(R¹⁹)-CO-R²⁰, -C(R¹⁸)=C(R¹⁹)-CO-N(R²⁰,R²¹), -CH(R¹⁸)-CH(R¹⁹)-C=O-N(R²⁰,R²¹), -C(R²¹)=N-O-R²², -CO-OC(R²³)(R²⁴)-CO-O-R²⁵, CO-N(R²⁶)-OR²² or C(O-R²⁷)=N-OR²²;
R¹⁸, R²³, R²⁴ are each hydrogen or C₁-C₃-alkyl;
R¹⁹ is halogen, cyano or methyl;
R²⁰ is hydroxyl, C₁-C₆-alkoxy, C₃-C₆-cycloalkoxy, C₁-C₄-alkoxy-C₁-C₄-alkoxy, C₁-C₄-alkylthio-C₁-C₄-alkoxy, C₁-C₄-alkylsulfinyl-C₁-C₄-alkoxy, C₁-C₄-alkylsulfonyl-C₁-C₄-alkoxy, cyano-C₁-C₆-alkoxy, C₁-C₄-alkoxycarbonyl-C₁-C₄-alkoxy, C₃-C₆-alkenyloxy, C₃-C₆-alkynyloxy, partially or fully halogenated C₁-C₆-alkoxy, partially or fully halogenated C₃-C₆-alkenyloxy, partially or fully halogenated C₃-C₆-alkynyloxy, C₁-C₆-alkylthio,
furthermore C₁-C₆-alkoxy which may carry two additional C₁-C₆-alkoxy substituents,
R²¹ is hydrogen, C₁-C₆-alkyl or C₁-C₆-haloalkyl;
R²² is hydrogen, C₁-C₆-alkyl, C₁-C₄-alkoxy-C₁-C₄-alkyl, C₁-C₆-haloalkyl, C₁-C₆-cyanoalkyl, C₃-C₆-cycloalkyl, C₃-C₆-alkenyl, C₃-C₆-alkynyl, C₃-C₆-haloalkenyl, C₃-C₆-haloalkynyl, carboxyl-C₁-C₆-alkyl, C₁-C₆-alkoxycarbonyl-C₁-C₄-alkyl, C₁-C₆-alkylcarbonyl-C₁-C₆-alkyl or C₁-C₆-alkylcarbonyloxy-C₁-C₆-alkyl;
R²⁵ is hydrogen, C₁-C₆-alkyl, C₃-C₆-cycloalkyl, C₁-C₆-alkoxy-C₁-C₄-alkyl, cyano-C₁-C₆-alkyl, halo-C₁-C₆-alkyl, C₃-C₆-alkenyl or C₃-C₆-alkynyl;
R²⁶, R²⁷ independently of one another are C₁-C₆-alkyl, C₁-C₄-alkoxy-C₁-C₄-alkyl, C₃-C₆-cycloalkyl, C₃-C₆-alkenyl, C₃-C₆-alkynyl, C₁-C₆-haloalkyl, C₁-C₆-cyanoalkyl, C₃-C₆-haloalkenyl, C₃-C₆-haloalkynyl or C₁-C₆-alkoxycarbonyl-C₁-C₄-alkyl;
R²⁶ is additionally hydrogen and
m is 0, 1, 2 or 3,
and the agriculturally useful salts of the compound I.

5. A fused triazole of the formula I' as claimed in claim 4, where the substituents and the index m have the following meanings:
Z is oxygen;
R⁵ is chlorine or cyano;
R¹⁸, R²³, R²⁴ are each hydrogen or methyl;
R¹⁹ is halogen or cyano;
R²⁰ is C₁-C₆-alkoxy, C₃-C₅-cycloalkoxy, C₁-C₄-alkoxy-C₁-C₄-alkoxy, C₁-C₄-alkoxycarbonyl-C₁-C₄-alkoxy, C₃-C₆-alkenyloxy, C₃-C₆-alkynyloxy or C₁-C₄-haloalkoxy;
R²¹ is hydrogen or C₁-C₄-alkyl;
R²² is C₁-C₆-alkyl, C₃-C₆-alkenyl, C₃-C₆-alkynyl or C₁-C₆-alkoxycarbonyl-C₁-C₂-alkyl;
R²⁵ is C₁-C₆-alkyl, C₁-C₆-alkoxy-C₁-C₄-alkyl, C₃-C₆-alkenyl or C₃-C₆-alkynyl;
R²⁶ is hydrogen, C₁-C₆-alkyl, C₁-C₄-alkoxy-C₁-C₄-alkyl, C₃-C₆-alkenyl, C₃-C₆-alkynyl or C₁-C₆-alkoxycarbonyl-C₁-C₂-alkyl;
R²⁷ is C₁-C₆-alkyl, C₁-C₄-alkoxy-C₁-C₄-alkyl, C₃-C₆-alkenyl, C₃-C₆-alkynyl or C₁-C₆-alkoxycarbonyl-C₁-C₂-alkyl;
m is 0,
and agriculturally useful salts thereof.

6. A substituted N-methyleneimino-N'-phenylurea of the formula III' 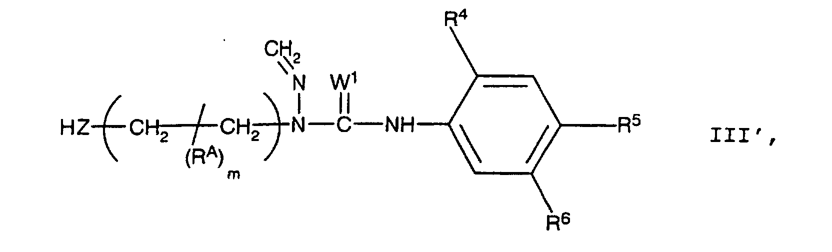 where:
Z is O, S, S=O or SO₂;
R^{A} is halogen or C₁-C₃-alkyl;
m is 0, 1, 2 or 3
W¹ is oxygen or sulfur;
R⁴ is hydrogen or halogen;
R⁵ is halogen, cyano or trifluoromethyl;
R⁶ is a group -C(R¹⁸)=C(R¹⁹)-CO-R²⁰, -CH(R¹⁸)-CH(R¹⁹)-CO-R²⁰, -C(R¹⁸)=C(R¹⁹)-CO-N(R²⁰,R²¹), -CH(R¹⁸)-CH(R¹⁹)-CO-N(R²⁰, R²¹), -C(R²¹)=N-OR²², -CO-OC(R²³)(R²⁴)-CO-OR²⁵, CO-N(R²⁶)-OR²² or C(O-R²⁷)=N-OR²²;
R¹⁸, R²³, R²⁴ are each hydrogen or C₁-C₃-alkyl;
R¹⁹ is halogen, cyano or methyl;
R²⁰ is hydroxyl, C₁-C₆-alkoxy, C₃-C₆-cycloalkoxy, C₁-C₄-alkoxy-C₁-C₄-alkoxyl C₁-C₄-alkylthio-C₁-C₄-alkoxy, C₁-C₄-alkylsulfinyl-C₁-C₄-alkoxy, C₁-C₄-alkylsulfonyl-C₁-C₄-alkoxy, cyano-C₁-C₆-alkoxy, C₁-C₄-alkoxycarbonyl-C₁-C₄-alkoxy, C₃-C₆-alkenyloxy, C₃-C₆-alkynyloxy, partially or fully halogenated C₁-C₆-alkoxy, partially or fully halogenated C₃-C₆-alkenyloxy, partially or fully halogenated C₃-C₆-alkynyloxy, C₁-C₆-alkylthio, furthermore C₁-C₆-alkoxy which may carry two additional C₁-C₆-alkoxy substituents;
R²¹ is hydrogen, C₁-C₆-alkyl or C₁-C₆-haloalkyl;
R²² is hydrogen, C₁-C₆-alkyl, C₁-C₄-alkoxy-C₁-C₄-alkyl, C₁-C₆-haloalkyl, C₁-C₆-cyanoalkyl, C₃-C₆-cycloalkyl, C₃-C₆-alkenyl, C₃-C₆-alkynyl, C₃-C₆-haloalkenyl, C₃-C₆-haloalkynyl, carboxyl-C₁-C₆-alkyl, C₁-C₆-alkoxycarbonyl-C₁-C₄-alkyl, C₁-C₆-alkylcarbonyl-C₁-C₆-alkyl or C₁-C₆-alkylcarbonyloxy-C₁-C₆-alkyl;
R²⁵ is hydrogen, C₁-C₆-alkyl, C₃-C₆-cycloalkyl, C₁-C₆-alkoxy-C₁-C₄-alkyl, cyano-C₁-C₆-alkyl, halo-C₁-C₆-alkyl, C₃-C₆-alkenyl or C₃-C₆-alkynyl;
R²⁶,R²⁷ independently of one another are C₁-C₆-alkyl, C₁-C₄-alkoxy-C₁-C₄-alkyl, C₃-C₆-cycloalkyl, C₃-C₆-alkenyl, C₃-C₆-alkynyl, C₁-C₆-haloalkyl, C₁-C₆-cyanoalkyl, C₃-C₆-haloalkenyl, C₃-C₆-haloalkynyl or C₁-C₆-alkoxycarbonyl-C₁-C₄-alkyl, and
R²⁶ is additionally hydrogen.

7. A substituted N-methyleneimino-N'-phenylurea of the formula III' as claimed in claim 6, where:
Z is oxygen;
R⁵ is chlorine or cyano;
R¹⁸, R²³, R²⁴ are each hydrogen or methyl;
R¹⁹ is halogen or cyano;
R²⁰ is C₁-C₆-alkoxy, C₃-C₅-cycloalkoxy, C₁-C₄-alkoxy-C₁-C₄-alkoxy, C₁-C₄-alkoxycarbonyl-C₁-C₄-alkoxy, C₃-C₆-alkenyloxy, C₃-C₆-alkynyloxy or C₁-C₄-haloalkoxy;
R²¹ is hydrogen or C₁-C₄-alkyl;
R²² is C₁-C₆-alkyl, C₃-C₆-alkenyl, C₃-C₆-alkynyl or C₁-C₆-alkoxycarbonyl-C₁-C₂-alkyl;
R²⁵ is C₁-C₆-alkyl, C₁-C₆-alkoxy-C₁-C₄-alkyl, C₃-C₆-alkenyl or C₃-C₆-alkynyl;
R²⁶ is hydrogen, C₁-C₆-alkyl, C₁-C₄-alkoxy-C₁-C₄-alkyl, C₃-C₆-alkenyl, C₃-C₆-alkynyl or C₁-C₆-alkoxycarbonyl-C₁-C₂-alkyl;
R²⁷ is C₁-C₆-alkyl, C₁-C₄-alkoxy-C₁-C₄-alkyl, C₃-C₆-alkenyl, C₃-C₆-alkynyl or C₁-C₆-alkoxycarbonyl-C₁-C₂-alkyl;
m is zero.

8. The use of the fused triazoles of the formula I' and the agriculturally useful salts of I' as claimed in claim 4, as herbicides or for the dessication and/or defoliation of plants.

9. A herbicidal composition, comprising a herbicidally effective amount of at least one fused triazole of the formula I' or an agriculturally useful salt of I' as claimed in claim 4 and at least one inert liquid and/or solid carrier and also, if desired, at least one adjuvant.

10. A composition for the dessication and/or defoliation of plants, comprising such an amount of at least one fused triazole of formula I' or an agriculturally useful salt of I' as claimed in claim 4, that acts as a dessicant and/or defoliant, and at least one inert liquid and/or solid carrier and, if desired, at least one adjuvant.

11. A method for controling undesirable vegetation, which comprises allowing a herbicidally effective amount of at least one fused trizole of the formula I' or an agriculturally useful salt of I' as claimed in claim 4 to act on plants, their habitat or on seeds.

12. A method for the dessication and/or defoliation of plants, which comprises allowing such an amount of at least one fused triazole of the formula I' or an agriculturally useful salt of I' as claimed in claim 4 to act on plants that has a dessicant and/or a defoliant action.

13. A fused triazole of the formula I'' 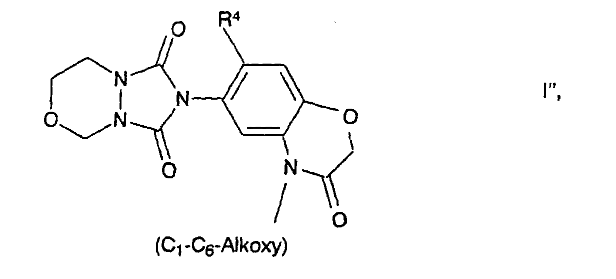 where R⁴ is hydrogen or halogen.

## Revendications

1. Procédé pour la préparation de triazoles condensés de formule I, 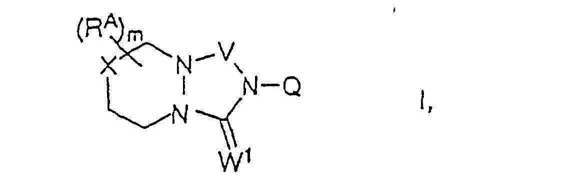 où les substituants ont les significations suivantes:
X O, S, SO, SO₂ ou N(R¹);
V C=W²;
W¹ oxygène ou soufre;
W² oxygène;
R^{A} hydrogène, hydroxy, COOH, COOR², halogène, cyano, C(O)NR¹¹R¹², OR³, alkyle en C₁-C₆, halogénoalkyle en C₁-C₆, alcényle en C₃-C₆, alcynyle en C₃-C₆, COR³, S(O)ₙR³ ou C(O)SR²;
R¹ hydrogène, hydroxy, halogène, CN, alkyle en C₁-C₆, halogénoalkyle en C₁-C₆, alcényle en C₂-C₆, alcynyle en C₂-C₆, COR³, CHO, OR³, COOR², C(O)SR², C(O)NR¹¹R¹²;
R² hydrogène, alkyle en C₁-C₆, halogénoalkyle en C₁-C₆, alcényle en C₂-C₆ ou alcynyle en C₂-C₆;
R³ alkyle en C₁-C₆, cycloalkyle en C₃-C₆, alcényle en C₂-C₆, alcynyle en C₂-C₆, halogénoalkyle en C₁-C₆, alcoxy(C₁-C₆)alkyle(C₁-C₆), carboxyl-alkyle-(C₁-C₆), alcoxy(C₁-C₆)carbonyl-alkyle-(C₁-C₆), alkylthio(C₁-C₆)alkyle(C₁-C₆), alkylsulfinyl(C₁-C₆)-alkyle(C₁-C₆), alkylsulfonyl(C₁-C₆)-alkyle(C₁-C₆), cycloalkyl(C₃-C₆)-alkyle-(C₁-C₆), alcényloxy(C₃-C₆)carbonyl-alkyle-(C₁-C₆), alcynyloxy(C₃-C₆)carbonyl-alkyle-(C₁-C₆), cycloalcoxy(C₃-C₆)-alkyle(C₁-C₆), alcényloxy(C₃-C₆)alkyle(C₁-C₆), alcynyloxy-(C₃-C₆)alkyle(C₁-C₆), halogénoalcoxy(C₂-C₆)-alkyle(C₁-C₆), halogénoalcényloxy(C₃-C₆)-alkyle(C₁-C₆), halogénoalcynyloxy(C₃-C₆)-alkyle(C₁-C₆), cycloalkyl(C₃-C₆)-thioalkyle(C₁-C₆), alcénylthio(C₃-C₆)-alkyle(C₁-C₆), alcynylthio(C₃-C₆)-alkyle(C₁-C₆), cyano-alkyle(C₁-C₆), halogénocycloalkyl(C₃-C₆)-alkyle(C₁-C₆), halogénoalcényle(C₃-C₆), alcoxy(C₁-C₆)-alcényle(C₃-C₆), halogénoalcoxy(C₁-C₆)-alcényle(C₃-C₆), alkylthio(C₁-C₆)-alcényle(C₃-C₆), halogénoalcynyle en C₃-C₆, alcoxy(C₁-C₆)-alcynyle(C₃-C₆), halogénoalcoxyalcynyle(C₃-C₆), alkylthio(C₁-C₆)-alcynyle, alkyl(C₁-C₆)carbonyle, CHR¹⁶COR¹⁷, CHR¹⁶P(O)-(OR¹⁷)₂, P(O)(OR¹⁷)₂, CHR¹⁶P(S)(OR¹⁷)₂, CHR¹⁶C(O)NR¹¹R¹², CHR¹⁶C(O)NH₂, alkyle en C₁-C₆ qui est substitué par un phénoxy ou un benzyloxy, tandis que les cycles peuvent à leur tour être substitués par des groupes halogéno, alkyle en C₁-C₄ ou halogénoalkyle en C₁-C₄, benzyle qui peut être substitué par des groupes halogéno, alkyle en C₁-C₄ ou halogénoalkyle en C₁-C₄, ou phényle et pyridyle qui peuvent être substitués par des groupes halogéno, alkyle en C₁-C₄, halogénoalkyle en C₁-C₄ ou alcoxy en C₁-C₄;
m la valeur 0, 1, 2 ou 3;
n la valeur 0, 1 ou 2;
Q l'un des restes Q-1 à Q-7 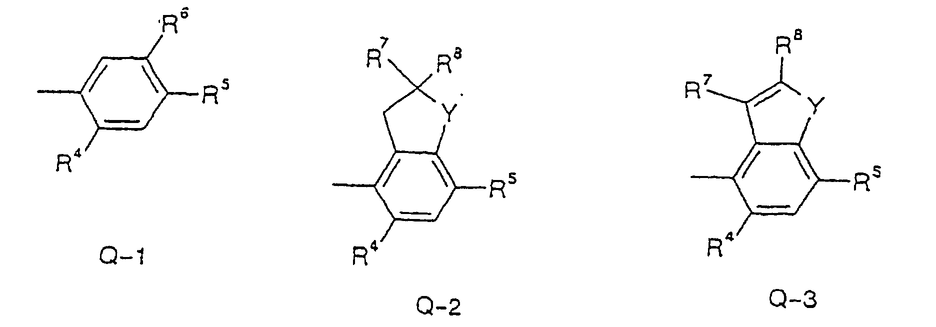 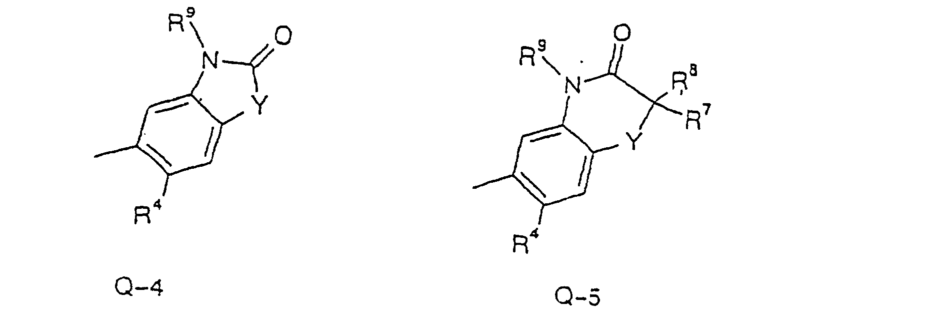 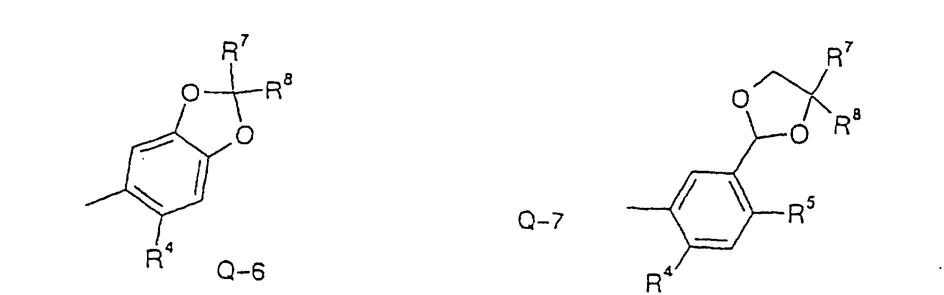
Y oxygène ou soufre;
R⁴ hydrogène ou halogéno;
R⁵ alkyle en C₁-C₆, halogénoalkyle en C₁-C₆, OCH₃, SCH₃, OCHF₂, halogéno, CN ou NO₂;
R⁶ hydrogène, alkyle en C₁-C₆, halogénoalkyle en C₁-C₆, halogéno, OR¹⁰, S(O)ₙR¹⁰, COR¹⁰, COOR¹⁰, C(O)SR¹⁰, SCH₂C≡CH, C(O)NR¹¹R¹², CHO, CH=CHCO-OR¹⁰, CO-ON=CR¹³R¹⁴, NO₂, CN, NHSO₂R¹⁵, NHSO₂NHR¹⁵, -C(R¹⁸)=C(R¹⁹)-CO-R²⁰, -CH(R¹⁸)-CH(R¹⁹)-CO-R²⁰, -C(R¹⁸)=C(R¹⁹)-CO-N(R²⁰,R²¹), -CH(R¹⁸)-CH(R¹⁹)-CO-N(R²⁰,R²¹), -C(R²¹)=N-OR²², -COOC(R²³)R²⁴)-COOR²⁵, -CO-N(R²⁶)-OR²² ou -C(OR²⁷)=N-OR²²;
R⁷, R⁸ indépendamment l'un de l'autre, hydrogène, alkyle en C₁-C₆, halogénoalkyle en C₁-C₆ ou halogéno; quand Q est Q-2, Q-5 ou Q-6, R⁷ et R⁸ peuvent former un groupe C=O conjointement avec l'atome de C auquel ils sont liés;
R⁹ alkyle en C₁-C₆, halogénoalkyle en C₁-C₆, alcoxy en C₁-C₆, alcoxy(C₁-C₆)-alkyle(C₁-C₆), alcényle en C₂-C₆ ou alcynyle en C₂-C₆;
R¹⁰ l'un des restes indiqués sous R³;
R¹¹, R¹³ indépendamment l'un de l'autre, hydrogène ou alkyle en C₁-C₆;
R¹², R¹⁴ indépendamment l'un de l'autre, alkyle en C₁-C₆ ou phényle, qui peut être substitué par des groupes halogéno, alkyle en C₁-C₄, halogénoalkyle en C₁-C₄ ou alcoxy en C₁-C₄;
R¹¹ et R¹² peuvent former ensemble un groupement -(CH₂)₅-, -(CH₂)₄- ou -CH₂CH₂OCH₂CH₂-, tandis que chaque cycle peut être substitué par des groupes alkyle en C₁-C₃, ou phényle ou benzyle;
R¹³ et R¹⁴ peuvent également former un groupe cycloalkyle en C₃-C₈ conjointement avec l'atome de C auquel ils sont liés;
R¹⁵ alkyle en C₁-C₆ ou halogénoalkyle en C₁-C₆;
R¹⁶ hydrogène ou alkyle en C₁-C₆;
R¹⁷ alkyle en C₁-C₆, alcényle en C₂-C₆ ou alcynyle en C₂-C₆;
R¹⁸, R²³, R²⁴ chacun hydrogène ou alkyle en C₁-C₃;
R¹⁹ halogéno, cyano ou méthyle;
R²⁰ hydroxy, alcoxy en C₁-C₆, cycloalcox en C₃-C₆, alcoxy(C₁-C₄)-alcoxy(C₁-C₄), alkylthio(C₁-C₄)-alcoxy(C₁-C₄), alkylsulfinyl(C₁-C₄)-alcoxy(C₁-C₄), alkylsulfonyl(C₁-C₄)-alcoxy(C₁-C₄), cyano-alcoxy(C₁-C₆), alcoxy(C₁-C₄)carbonyl-alcoxy(C₁-C₄), alcényloxy en C₃-C₆, alcynyloxy en C₃-C₆, alcoxy en C₁-C₆ partiellement ou totalement halogéné, alcényloxy en C₃-C₆ partiellement ou totalement halogéné, alcynyloxy en C₃-C₆ partiellement ou totalement halogéné, alkylthio en C₁-C₆, ainsi que alcoxy en C₁-C₆ qui peut encore porter deux substituants alcoxy en C₁-C₆;
R²¹ hydrogène, alkyle en C₁-C₆ ou halogénoalkyle en C₁-C₆;
R²² hydrogène, alkyle en C₁-C₆, alcoxy(C₁-C₄)-alkyle(C₁-C₄), halogénoalkyle en C₁-C₆, cyano-alkyle(C₁-C₆), cycloalkyle en C₃-C₆, alcényle en C₃-C₆, alcynyle en C₃-C₆, halogénoalcényle en C₃-C₆, halogénoalcynyle en C₃-C₆, carboxyalkyle(C₁-C₆), alcoxy(C₁-C₆)carbonyl-alkyle(C₁-C₄), alkyl(C₁-C₆)carbonyl-alkyle(C₁-C₆) ou alkyl(C₁-C₆)carbonyloxy-alkyle(C₁-C₆);
R²⁵ hydrogène, alkyle en C₁-C₆, cycloalkyle en C₃-C₆, alcoxy(C₁-C₆)-alkyle(C₁-C₄), cyano-alkyle(C₁-C₆), halogénoalkyle en C₁-C₆, alcényle en C₃-C₆ ou alcynyle en C₃-C₆;
R²⁶, R²⁷ indépendamment l'un de l'autre, alkyle en C₁-C₆, alcoxy(C₁-C₄)-alkyle(C₁-C₄), cycloalkyle en C₃-C₆, alcényle en C₃-C₆, alcynyle en C₃-C₆, halogénoalkyle en C₁-C₆, cyano-alkyle(C₁-C₆), halogénoalcényle en C₃-C₆, halogénoalcynyle en C₃-C₆ ou alcoxy(C₁-C₆)carbonyl-alkyle(C₁-C₄);
R²⁶ en outre hydrogène;
**caractérisé par le fait que**, dans une première étape, on fait réagir des composés de formule II 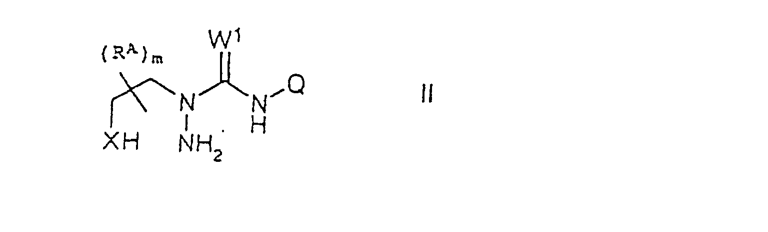 avec du formaldéhyde ou du paraformaldéhyde aqueux, en l'absence d'acide, en milieu neutre ou faiblement alcalin, pour former des urées N-méthylèneimino-N'-substituées de formule III 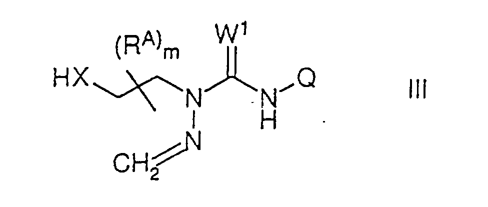 on ensuite cyclise celles-ci en présence de quantités catalytiques d'acide ou d'un oxyde métallique tensio-actif neutre ou acide pour former des tétrahydro-4H-1,3,4-oxa(ou thia)diazines substituées de formule IV 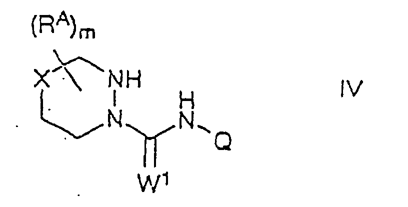 et on cyclise cette dernière avec du phosgène ou un substitut du phosgène pour former les composés de formule I.

2. Procédé selon la revendication 1, **caractérisé par le fait qu'**on effectue la cyclisation des composés de formule III en les tétrahydro-4H-1,3,4-oxa(ou thia)diazines substituées de formule IV en présence d'un oxyde métallique tenio-actif.

3. Procédé selon la revendication 2, **caractérisé par le fait qu'**on utilise du gel de silice comme oxyde métallique tensio-actif.

4. Triazoles condensés de formule I' 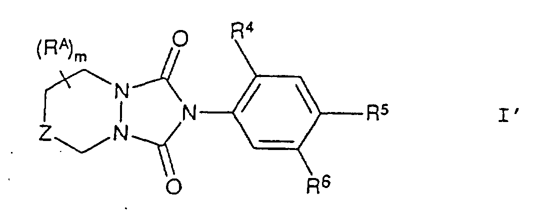 où les variables ont la signification:
Z O, S, S=O ou SO₂;
R^{A} halogéno ou alkyle en C₁-C₃;
R⁴ hydrogène ou halogéno;
R⁵ halogéno, cyano ou trifluorométhyle et
R⁶ un groupe -C(R¹⁸)=C(R¹⁹)-CO-R²⁰, -CH(R¹⁸)-CH(R¹⁹)-CO-R²⁰, -C(R¹⁸)=C(R¹⁹)-CO-N(R²⁰,R²¹), -CH(R¹⁸)-CH(R¹⁹)-C=O-N(R²⁰,R²¹), -C(R²¹)=N-O-R²², -CO-OC(R²³)(R²⁴)-CO-O-R²⁵, CO-N(²⁶)-OR²² ou C(O-R²⁷)=N-OR²²;
R¹⁸, R²³, R²⁴, chacun, hydrogène ou alkyle en C₁-C₃;
R¹⁹ halogéno, cyano ou méthyle;
R²⁰ hydroxy, alcoxy en C₁-C₆, cycloalcoxy en C₃-C₆, alcoxy(C₁-C₄)-alcoxy(C₁-C₄), alkylthio(C₁-C₄)-alcoxy(C₁-C₄), alkylsulfinyl(C₁-C₄)-alcoxy(C₁-C₄), alkylsulfonyl(C₁-C₄)-alcoxy(C₁-C₄), cyano-alcoxy(C₁-C₆), alcoxy(C₁-C₄)carbonyl-alcoxy(C₁-C₄), alcényloxy en C₃-C₆, alcynyloxy en C₃-C₆, alcoxy en C₁-C₆ partiellement ou totalement halogéné, alcényloxy en C₃-C₆ partiellement ou totalement halogéné, alcynyloxy en C₃-C₆ partiellement ou totalement halogéné, alkylthio en C₁-C₆, ainsi que alcoxy en C₁-C₆ qui peut encore porter deux substituants alcoxy en C₁-C₆;
R²¹ hydrogène, alkyle en C₁-C₆ ou halogénoalkyle en C₁-C₆;
R²² hydrogène, alkyle en C₁-C₆, alcoxy(C₁-C₄)-alkyle(C₁-C₄), halogénoalkyle en C₁-C₆, cyano-alkyle en C₁-C₆, cycloalkyle en C₃-C₆, alcényle en C₃-C₆, alcynyle en C₃-C₆, halogénoalcényle en C₃-C₆, halogénoalcynyle en C₃-C₆, carboxyalkyle(C₁-C₆), alcoxy(C₁-C₆)carbonyl-alkyle(C₁-C₄), alkyl(C₁-C₆)carbonyl-alkyle(C₁-C₆) ou alkyl(C₁-C₆)carbonyloxy-alkyle(C₁-C₆);
R²⁵ hydrogène, alkyle en C₁-C₆, cycloalkyle en C₃-C₆, alcoxy(C₁-C₆)-alkyle(C₁-C₄) cyano-alkyle(C₁-C₆), halogénoalkyle en C₁-C₆, alcényle en C₃-C₆ ou alcynyle en C₃-C₆;
R²⁶, R²⁷, indépendamment l'un de l'autre, alkyle en C₁-C₆, alcoxy(C₁-C₄)-alkyle(C₁-C₄), cycloalkyle en C₃-C₆, alcényle en C₃-C₆, alcynyle en C₃-C₆, halogénoalkyle en C₁-C₆, cyano-alkyle(C₁-C₆), halogénoalcényle en C₃-C₆, halogénoalcynyle en C₃-C₆ ou alcoxy(C₁-C₆)carbonyl-alkyle(C₁-C₄);
R²⁶ en outre hydrogène et
m 0, 1, 2 ou 3,
ainsi que les sels utilisables en agriculture de composés I.

5. Triazoles condensés de formule I' selon la revendication 4, dans lesquels les substituants et l'indice m ont les significations suivante:
Z oxygène;
R⁵ chloro ou cyano;
R¹⁸, R²³, R²⁴, chacun, hydrogène ou méthyle;
R¹⁹ halogéno ou cyano;
R²⁰ alcoxy en C₁-C₆, cycloalcoxy en C₃-C₆, alcoxy(C₁-C₄)-alcoxy(C₁-C₄), alcoxy(C₁-C₄)carbonyl-alcoxy(C₁-C₄), alcényloxy en C₃-C₆, alcynyloxy en C₃-C₆ ou halogénoalcoxy en C₁-C₄;
R²¹ hydrogène ou alkyle en C₁-C₄;
R²² alkyle en C₁-C₆, alcényle en C₃-C₆, alcynyle en C₃-C₆ ou alcoxy(C₁-C₆)carbonyl-alkyle(C₁-C₂);
R²⁵ alkyle en C₁-C₆, alcoxy(C₁-C₆)-alkyle(C₁-C₄), alcényle en C₃-C₆ ou alcynyle en C₃-C₆;
R²⁶ hydrogène, alkyle en C₁-C₆, alcoxy(C₁-C₄)-alkyle(C₁-C₄, alcényle en C₃-C₆, alcynyle en C₃-C₆ ou alcoxy(C₁-C₆)carbonyl-alkyle(C₁-C₂);
R²⁷ alkyle en C₁-C₆, alcoxy(C₁-C₄)-alkyle(C₁-C₄), alcényle en C₃-C₆, alcynyle en C₃-C₆ ou alcoxy(C₁-C₆)carbonyl-alkyle(C₁-C₂);
m 0,
ainsi que leurs sels utilisables en agriculture.

6. N-méthylèneimino-N'-phénylurées substituées de formule III' 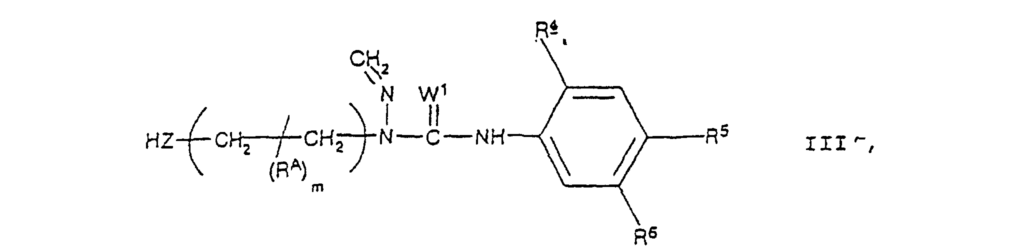 où les substituants ont les significations suivantes:
Z O, S, S=O ou SO₂;
R^{A} halogéno ou alkyle en C₁-C₃;
m 0, 1, 2 ou 3;
W¹ oxygène ou soufre;
R⁴ hydrogène ou halogéno;
R⁵ halogéno, cyano ou trifluorométhyle;
R⁶ un groupe -C(R¹⁸)=C(R¹⁹)-CO-R²⁰, -CH(R¹⁸)-CH(R¹⁹)-CO-R²⁰, -C(R¹⁸)=C(R¹⁹)-CO-N(R²⁰,R²¹), -CH(R¹⁸)-CH(R¹⁹)-C=O-N(R²⁰,R²¹), -C(R²¹)=N-O-R²², -CO-OC(R²³)(R²⁴)-CO-O-R²⁵, CO-N(²⁶)-OR²² ou C(O-R²⁷)=N-OR²²;
R¹⁸, R²³, R²⁴, chacun, hydrogène ou alkyle en C₁-C₃;
R¹⁹ halogéno, cyano ou méthyle;
R²⁰ hydroxy, alcoxy en C₁-C₆, cycloalcoxy en C₃-C₆, alcoxy(C₁-C₄)-alcoxy(C₁-C₄), alkylthio(C₁-C₄)-alcoxy(C₁-C₄), alkylsulfinyl(C₁-C₄)-alcoxy(C₁-C₄), alkylsulfonyl(C₁-C₄)-alcoxy(C₁-C₄), cyano-alcoxy(C₁-C₆), alcoxy(C₁-C₄)carbonyl-alcoxy(C₁-C₄), alcényloxy en C₃-C₆, alcynyloxy en C₃-C₆, alcoxy en C₁-C₆ partiellement ou totalement halogéné, alcényloxy en C₃-C₆ partiellement ou totalement halogéné, alcynyloxy en C₃-C₆ partiellement ou totalement halogéné, alkylthio en C₁-C₆, ainsi que alcoxy en C₁-C₆ qui peut encore porter deux substituants alcoxy en C₁-C₆;
R²¹ hydrogène, alkyle en C₁-C₆ ou halogénoalkyle en C₁-C₆;
R²² hydrogène, alkyle en C₁-C₆, alcoxy(C₁-C₄)-alkyle(C₁-C₄), halogénoalkyle en C₁-C₆, cyano-alkyle en C₁-C₆, cycloalkyle en C₃-C₆, alcényle en C₃-C₆, alcynyle en C₃-C₆, halogénoalcényle en C₃-C₆, halogénoalcynyle en C₃-C₆, carboxyalkyle(C₁-C₆), alcoxy(C₁-C₆)carbonyl-alkyle(C₁-C₄), alkyl(C₁-C₆)carbonyl-alkyle(C₁-C₆) ou alkyl(C₁-C₆)carbonyloxy-alkyle(C₁-C₆);
R²⁵ hydrogène, alkyle en C₁-C₆, cycloalkyle en C₃-C₆, alcoxy(C₁-C₆)-alkyle(C₁-C₄) cyano-alkyle(C₁-C₆), halogénoalkyle en C₁-C₆, alcényle en C₃-C₆ ou alcynyle en C₃-C₆;
R²⁶, R²⁷, indépendamment l'un de l'autre, alkyle en C₁-C₆, alcoxy(C₁-C₄)-alkyle(C₁-C₄), cycloalkyle en C₃-C₆, alcényle en C₃-C₆, alcynyle en C₃-C₆, halogénoalkyle en C₁-C₆, cyano-alkyle(C₁-C₆), halogénoalcényle en C₃-C₆, halogénoalcynyle en C₃-C₆ ou alcoxy(C₁-C₆)carbonyl-alkyle(C₁-C₄);
R²⁶ en outre hydrogène.

7. N-méthylèneimino-N'-phénylurées substituées de formule III' selon la revendication 6, dans lesquelles les substituants ont les significations suivantes:
Z oxygène;
R⁵ chloro ou cyano;
R¹⁸, R²³, R²⁴, chacun, hydrogène ou méthyle;
R¹⁹ halogéno ou cyano;
R²⁰ alcoxy en C₁-C₆, cycloalcoxy en C₃-C₆, alcoxy(C₁-C₄)-alcoxy(C₁-C₄), alcoxy(C₁-C₄)carbonyl-alcoxy(C₁-C₄), alcényloxy en C₃-C₆, alcynyloxy en C₃-C₆ ou halogénoalcoxy en C₁-C₄;
R²¹ hydrogène ou alkyle en C₁-C₄;
R²² alkyle en C₁-C₆, alcényle en C₃-C₆, alcynyle en C₃-C₆ ou alcoxy(C₁-C₆)carbonyl-alkyle(C₁-C₂) ;
R²⁵ alkyle en C₁-C₆, alcoxy(C₁-C₆)-alkyle(C₁-C₄), alcényle en C₃-C₆ ou alcynyle en C₃-C₆;
R²⁶ hydrogène, alkyle en C₁-C₆, alcoxy(C₁-C₄)-alkyle(C₁-C₄), alcényle en C₃-C₆, alcynyle en C₃-C₆ ou alcoxy(C₁-C₆)carbonyl-alkyle(C₁-C₂);
R²⁷ alkyle en C₁-C₆, alcoxy(C₁-C₄)-alkyle(C₁-C₄), alcényle en C₃-C₆, alcynyle en C₃-C₆ ou alcoxy(C₁-C₆)carbonyl-alkyle(C₁-C₂);
m zéro.

8. Utilisation des triazoles condensés de formule I' et des sels utilisables en agriculture de I' selon la revendication 4, comme herbicides ou pour la dessiccation et/ou la défoliation de plantes.

9. Produits herbicides, contenant une quantité efficace du point de vue herbicide d'au moins un triazole condensé de formule I' ou d'un sel utilisable en agriculture de I' selon la revendication 4, et au moins un support inerte liquide et/ou solide, ainsi qu'en option au moins un adjuvant.

10. Produit pour la dessiccation et/ou la défoliation de plantes, contenant une quantité efficace pour la dessiccation et/ou la défoliation d'au moins un triazole condensé de formule I' ou d'un sel utilisable en agriculture de I' selon la revendication 4, et au moins un support inerte liquide et/ou solide, ainsi qu'en option au moins un adjuvant.

11. Procédé pour la lutte contre la croissance de plantes parasites, **caractérisé par le fait qu'**on fait agir une quantité efficace du point de vue herbicide d'au moins un triazole condensé de formule I' ou d'un sel utilisable en agriculture de I' selon la revendication 4 sur des plantes, sur leur biotope ou sur des semences.

12. Procédé pour la dessiccation et/ou la défoliation de plantes, **caractérisé par le fait qu'**on fait agir une quantité efficace pour la dessiccation et/ou la défoliation d'au moins un triazole condensé de formule I' ou d'un sel utilisable en agriculture de I' selon la revendication 4 sur des plantes.

13. Triazoles condensés de formule I'' 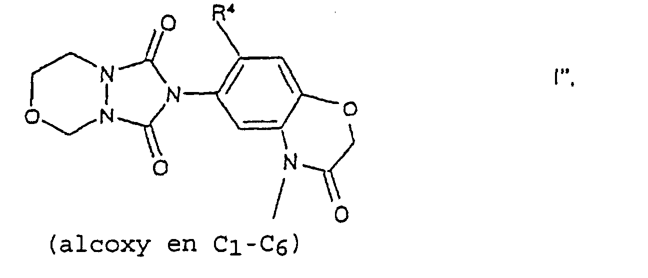 où R⁴ désigne l'hydrogène ou un halogène.
